# EUROPEAN PATENT SPECIFICATION

(11) **EP 1 979 355 B1**
(45) Date of publication and mention of the grant of the patent: **18.08.2010**
(21) Application number: 07717085.0
(22) Date of filing: 25.01.2007
(51) Int. Cl.: C07D 471/10, A61K 31/435, A61P 3/00, A61P 9/00, A61P 11/00

(54) **SPIRO IMIDAZOLE DERIVATIVES AS PPAR MODULATORS**
SPIROIMIDAZOL-DERIVATE ALS PPAR-MODULATOREN
DERIVES D'IMADAZOLE SPIRO UTILISES COMME MODULATEURS DU RECEPTEUR PPAR

(30) Priority: 30.01.2006 US 763557 P
(43) Date of publication of application: 15.10.2008
(73) Proprietor: IRM LLC, Hamilton HM 11 (BM)
(72) Inventor: EPPLE, Robert, San Diego, California 92122 (US); RUSSO, Ross, Encinitas, California 92024 (US); AZIMIOARA, Mihai, La Jolla, California 92037 (US); COW, Christopher, San Diego, California 92128 (US); MOLTENI, Valentina, San Diego, California 92102 (US); LI, Xiaolin, San Diego, California 92131 (US); CHIANELLI, Donatella, San Diego, California 92130 (US)
(74) Representative: Kiddle, Simon John
(86) International application number: PCT/US2007/002315
(87) International publication number: WO 2007/087448

(56) References cited:
- EP-A- 1 619 193
- WINTERS, GIORGIO ET AL: "Synthesis of spirohydantoins from basic heterocyclic ketones" FARMACO, EDIZIONE SCIENTIFICA , 25(9), 681-93 CODEN: FRPSAX; ISSN: 0430-0920, 1970, XP009083295

## Description

### BACKGROUND OF THE INVENTION

### Field of the Invention

The present invention provides compounds, pharmaceutical compositions comprising such compounds and the medical uses of the compounds and compositions to treat or prevent diseases or disorders associated with the activity of the Peroxisome Proliferator Activated Receptor (PPAR) families.

### Background

Peroxisome Proliferation Activated Receptors (PPARs) are members of the nuclear hormone receptor super family, which are ligand-activated transcription factors regulating gene expression. Certain PPARs are associated with a number of disease states including dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, IBDs (irritable bowel disease), ulcerative colitis and Crohn's disease. Accordingly, molecules that modulate the activity of PPARs are useful as therapeutic agents in the treatment of such diseases.

European Patent No: 1619193 A describes spiro-piperidine compounds represented by formula (I) and their uses in the prevention and/or treatment of inflammatory diseases, immune diseases, such as autoimmune diseases, allergic diseases and HIV infection. Winters et al (Farmaco Edizione Scientifica, 25(9): 681-693, 1970) discloses the synthesis of several spirohydantoins of N-substituted 4-piperidones, 3-piperidones and 3-pyrrolidinones and the preparation of spirothiohydantoins of 4-piperidones. The anti-inflammatory activity of the compounds is mentioned.

### SUMMARY OF THE INVENTION

In one aspect, the present invention provides compounds selected from Formula Ia, Ib and Ic: 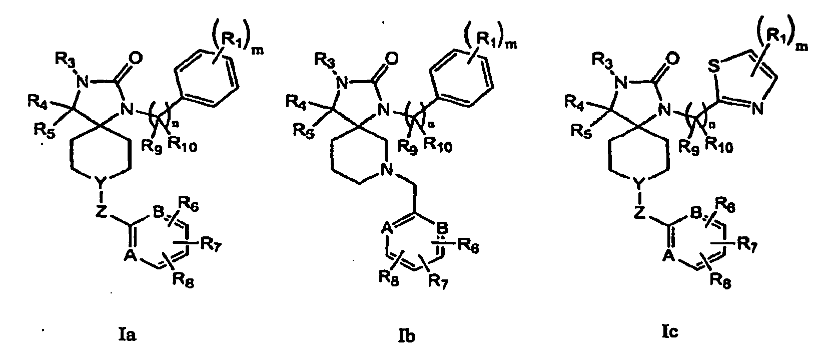 in which
n is selected from 1 or 2;
m is selected from 1, 2, 3, 4 and 5; each
R₁ is independently selected from hydrogen, halo, C₁₋₆alkyl, halo-substituted C₁₋₆alkyl, C₁₋₆alkoxy and halo-substituted-C₁₋₆alkoxy;
R₃ is selected from C₁₋₈alkyl, C₂₋₈alkenyl, halo-substituted-C₁₋₆alkyl, halo-substituted-C₂₋₆alkenyl, C₅₋₁₀heteroaryl-C₀₋₄alkyl and C₃₋₁₂cycloalkyl-C₀₋₄alkyl; wherein R₂ is selected from hydrogen and C₁₋₆alkyl;
R₄ is selected from hydrogen and C₁₋₆alkyl;
R₅ is selected from hydrogen and C₁₋₆alkyl; or R₄ and R₅ together with the carbon atom to which R₄ and R₅ are both attached form carbonyl;
Y is N and ;
Z is selected from a bond, -S(O)₀₋₂- and -CR₁₁R₁₂-, wherein R₁₁ and R₁₂ are independently selected from hydrogen and C₁₋₆alkyl;
A and B are independently selected from CH and N;
R₆ and R₇ are independently selected from hydrogen, halo, C₁₋₆alkyl, halo-substituted C₁₋₆alkyl, C₁₋₆alkoxy and halo-substituted-C₁₋₆alkoxy;
R₈ is selected from -X₂CO₂R₁₃, -X₂CR₁₄R₁₅X₃CO₂R₁₃,-X₂SCR₁₄R₁₅X₃CO₂R₁₃ and -X₂OCR₁₄R₁₅X₃CO₂R₁₃; wherein X₂ and X₃ are independently selected from a bond and C₁₋₄alkylene; and R₁₄ and R₁₅ are independently selected from hydrogen, C₁₋₄alkyl and C₁₋₄alkoxy; or R₁₄ and R₁₅ together with the carbon atom to which R₁₄ and R₁₅ are attached form C₃₋₁₂cycloalkyl; and R₁₃ is selected from hydrogen and C₁₋₆alkyl;
R₉ and R₁₀ are independently selected from hydrogen, C₁₋₆alkyl and-OR₁₆; wherein R₁₆ is selected from hydrogen and C₁₋₆alkyl; and individual isomers and mixture of isomers thereof; and the pharmaceutically acceptable salts and solvates (e.g. hydrates) of such compounds.

In a second aspect, the present invention provides a pharmaceutical composition that contains a compound of Formula I. as set out in the claims admixture with one or more suitable excipients.

In a third aspect, the present invention provides the use of a compound of Formula I in the manufacture of a medicament for treating a disease in an animal in which PPAR activity activity contributes to the pathology and/or symptomology of the disease, as set out in the claims.

### DETAILED DESCRIPTION OF THE INVENTION

### Definitions

"Alkyl" as a group and as a structural element of other groups, for example halo-substituted-alkyl and alkoxy, can be either straight-chained or branched. C₁₋₆alkoxy includes, methoxy, ethoxy, and the like. Halo-substituted alkyl includes trifluoromethyl, pentafluoroethyl, and the like.

"Aryl" means a monocyclic or fused bicyclic aromatic ring assembly containing six to ten ring carbon atoms. For example, aryl can be phenyl or naphthyl, preferably phenyl. "Arylene" means a divalent radical derived from an aryl group. "Heteroaryl" is as defined for aryl where one or more of the ring members are a heteroatom. For example heteroaryl includes pyridyl, indolyl, indazolyl, quinoxalinyl, quinolinyl, benzofuranyl, benzopyranyl, benzothiopyranyl, benzo[1,3]dioxole, imidazolyl, benzo-imidazolyl, pyrimidinyl, furanyl, oxazolyl, isoxazolyl, triazolyl, tetrazolyl, pyrazolyl, thienyl, etc. "C₆₋₁₀arylC₀₋₄alkyl" means an aryl as described above connected via a alkylene grouping. For example, C₆₋₁₀arylC₀₋₄alkyl includes phenethyl, benzyl, etc.

"Cycloalkyl" means a saturated or partially unsaturated, monocyclic, fused bicyclic or bridged polycyclic ring assembly containing the number of ring atoms indicated. For example, C₃₋₁₀cycloalkyl includes cyclopropyl, cyclobutyl, cyclopentyl, cyclohexyl, etc. "Heterocycloalkyl" means cycloalkyl, as defined in this application, provided that one or more of the ring carbons indicated, are replaced by a moiety selected from -O-, -N=, -NR-, -C(O) -, -S-, -S(O) - or -S(O)₂-, wherein R is hydrogen, C₁₋₄alkyl or a nitrogen protecting group. For example, C₃₋₈heterocycloalkyl as used in this application to describe compounds of the invention includes morpholino, pyrrolidinyl, piperazinyl, piperidinyl, piperidinylone, 1,4-dioxa-8-aza-spiro[4.5]dec-8-yl, etc.

"Halogen" (or halo) preferably represents chloro or fluoro, but can also be bromo or iodo.

"Treat", "treating" and "treatment" refer to a method of alleviating or abating a disease and/or its attendant symptoms.

### Description of the Preferred Embodiments

The present invention provides compounds, compositions and medical uses for the treatment of diseases in which modulation of one or more PPARs can prevent, inhibit or ameliorate the pathology and/or symptomology of the diseases, which employ compounds of Formula I, as set out in the claims.

In one embodiment, with reference to compounds of Formula Ia, Ib and Ic: n is selected from 1 or 2; m is selected from 1, 2 and 3; each R₁ is independently selected from hydrogen, halo, C₁₋₆alkyl, halo-substituted C₁₋₆alkyl, C₁₋₆alkoxy and halo-substituted-C₁₋₆alkoxy; R₃ is selected from C₁₋₈alkyl, C₂₋₈alkenyl, halo-substituted-C₁₋₆alkyl, halo-substituted-C₂₋₆alkenyl, -X₁C(O)R₂, C₅₋₁₀heteraryl-C₀₋₄alkyl and C₃₋₁₂cycloalkyl-C₀₋₄alkyl; wherein R₂ is selected from hydrogen and C₁₋₆alkyl; R₄ is selected from hydrogen and C₁₋₆alkyl; R₅ is selected from hydrogen and C₁₋₆alkyl; or R₄ and R₅ together with the carbon atom to which R₄ and R₅ are both attached form carbonyl; Y is N; Z is selected from a bond, -S(O)₀₋₂- and -CR₁₁R₁₂-; wherein R₁₁ and R₁₂ are independently selected from hydrogen and C₁₋₆alkyl; A and B are independently selected from CH and N; R₆ and R₇ are independently selected from hydrogen, halo, C₁₋₆alkyl, halo-substituted C₁₋₆alkyl and C₁₋₆alkoxy; R₈ is selected from - X₂CO₂R₁₃, -X₂CR₁₄R₁₅X₃CO₂R₁₃ and -X₂OCR₁₄R₁₅X₃CO₂R₁₃; wherein X₁ and X₃ are independently selected from a bond and C₁₋₄alkylene; and R₁₄ and R₁₅ are independently selected from hydrogen and C₁₋₄alkyl; R₁₃ is selected from hydrogen and C₁₋₆alkyl; and R₉ and R₁₀ are independently selected from hydrogen, C₁₋₆alkyl and -OR₁₆; wherein R₁₆ is selected from hydrogen and C₁₋₆alkyl.

In another embodiment, R₁ is independently selected from hydrogen, halo, methoxy, trifluormethoxy and trifluoromethyl; R₃ is selected from isobutyl, cyclopropylmethyl, cyclobutyl-methyl, isopentyl, butyl, cyclopentyl-methyl, 3-methyl-but-2-enyl, pentyl, 2,2-dimethyl-propyl, 4-fluoro-butyl, 2-ethyl-butyl, 2-methyl-pentyl, cyclohexylmethyl, 3,3-dimethyl-2-oxo-butyl, pyrrolyl-propyl, 3-trifluoromethyl-propyl, cyclohexylethyl, 2-ethyl-hexyl, 2-methyl-butyl, 3,4,4-trifluoro-but-3-enyl and 3,3-dimethyl-butyl; R₄ and R₅ are each hydrogen or R₄ and R₅ together with the carbon atom to which R₄ and R₅ are both attached form carbonyl; and Z is selected from a bond, -S(O)₂- and -CH₂-.

In another embodiment, R₈ is selected from -CH₂C(O)OH, - CH(CH₂)C(O)OH, -OC(CH₂)₂C(O)OH, -(CH₂)₂C(O)OH and -OCH₂C(O)OH; and R₉ and R₁₀ are independently selected from hydrogen, halo, methyl, methoxy and trifluoromethyl.

Preferred compounds of the invention are selected from: (3-{3-Isobutyl-1-[2-(4-methoxy-phenyl)-ethyl]-2,4-dioxo-1,3,8-triaza-spiro[4.5]dec-8-ylmethyl}-phenyl)-acetic acid; (3-{3-Isobutyl-1-[2-(4-methoxy-phenyl)-ethyl]-2,4-dioxo-1,3,8-triaza-spiro[4.5]decane-8-sulfonyl}-4-methyl-phenyl)-acetic acid; (3-{3-Isobutyl-1-[2-(4-methoxy-phenyl)-ethyl]-2,4-dioxo-1,3,8-triaza-spiro[4.5]dec-8-yl}-phenyl)-acetic acid; 2-(4-{3-Isobutyl-1-[2-(4-methoxy-phenyl)-ethyl]-2,4-dioxo-1,3-diaza-spiro[4.5]dec-7-ylmethyl}-phenyl)-propionic acid; (3-{3-Cyclopropylmethyl-1-[2-(4-methoxy-phenyl)-ethyl]-2,4-dioxo-1,3,8-triaza-spiro[4.5]dec-8-yl}-phenyl)-acetic acid; {3-[3-Isobutyl-2,4-dioxo-l-(4-trifluoromethoxy-benzyl)-1,3,8-triaza-spiro[4.5]dec-8-yl]-phenyl}-acetic acid; 2-(2-{3-Isobutyl-1-[2-(4-methoxy-phenyl)-ethyl]-2,4-dioxo-1,3,8-triaza-spiro[4.5]dec-8-yl}-pyrimidin-4-yloxy)-2-methyl-propionic acid; 2-(3-{3-Cyclobutylmethyl-1-[2-(2,4-dichloro-phenyl)-ethyl]-2,4-dioxo-1,3-diaza-spiro[4.5]dec-8-yl}-phenoxy)-2-methyl-propionic acid; {3-[3-Cyclopropylmemyl-2,4-dioxo-1-(4-trifluoromethoxy-benzyl)-1,3,8-triaza-spiro[4.5]dec-8-yl]-phenyl}-acetic acid; (3-{3-Cyclobutylmethyl-2,4-dioxo-1-[2-(4-trifluoromethyl-phenyl)-ethyl]-1,3,8-triaza-spiro[4.5]dec-8-yl}-phenyl)-acetic acid; (3-{3-Cyclobutylmethyl-1-[4-(4-methoxy-phenyl)-butyl]-2,4-dioxo-1,3,8-triaza-spiro[4.5]dec-8-yl}-phenyl)-acetic acid; 3-{3-Isobutyl-1-[2-(4-methoxy-phenyl)-ethyl]-2,4-dioxo-1,3,8-triaza-spiro[4.5]dec-8-ylmethyl}-benzoic acid; (2-{3-Isobutyl-1-[2-(4-methoxy-phenyl)-ethyl]-2,4-dioxo-1,3,8-triaza-spiro[4.5]dec-8-ylmethyl}-phenyl)-acetic acid; (3-{3-Isobutyl-1-[2-(4-methoxy-phenyl)-ethyl]-2,4-dioxo-1,3,8-triaza-spiro[4.5]decane-8-sulfonyl}-4-methoxy-phenyl)-acetic acid; 3-(3-{3-Isobutyl-1-[2-(4-methoxy-phenyl)-ethyl]-2,4-dioxo-1,3,8-triaza-spiro[4.5]dec-8-yl}-phenyl)-propionic acid; (3-{3-Isobutyl-1-[2-(4-methoxy-phenyl)-ethyl]-2,4-dioxo-1,3,8-triaza-spiro[4.5]dec-8-yl}-phenoxy)-acetic acid; 2-(3-{3-Isobutyl-1-[2-(4-methoxy-phenyl)-ethyl]-2,4-dioxo-1,3,8-triaza-spiro[4.5]dec-8-yl}-phenoxy)-2-methyl-propionic acid; (5-{3-Isobutyl-1-[2-(4-methoxyphenyl)-ethyl]-2,4-dioxo-1,3,8-triaza-spiro[4.5]dec-8-yl}-2-methyl-phenyl)-acetic acid; (3-{3-Isobutyl-1-[2-(4-methoxy-phenyl)-ethyl]-2,4-dioxo-1,3,8-triaza-spiro[4.5]dec-8-yl}-5-methyl-phenyl)-acetic acid; (2-Fluoro-5-{3-isobutyl-1-[2-(4-methoxy-phenyl)-ethyl]-2,4-dioxo-1,3,8-triza-spiro[4.5]dec-8-yl}-phenyl)-acetic acid; (5-{3-Isobutyl-1-[2-(4-methoxy-phenyl)-ethyl]-2,4-dioxo-1,3,8-triaza-spiro[4-5]dec-8-yl}-2-trifluoromethylphenyl)-acetic acid; (5-{3-Isobutyl-1-[2-4-methoxy-phenyl)-ethyl]-2,4-dioxo-1,3,8-triaza-spiro[4.5]dec-8-yl}-2-methoxy-phenyl)-acetic acid; 2-(3-{3-Isobutyl-1-[2-(4-methoxy-phenyl)-ethyl]-2,4-dioxo-1,3,8-triaza-spiro[4.5]dec-8-yl} phenyl)-2-methyl-propionic acid; (5-{3-Isobutyl-1-[2-(4-methoxy-phenyl)-ethyl]-2,4-dioxo-1,3,8-triaza-spiro[4.5]dec-8-yl}-2-methyl-phenoxy)-acetic acid; (2-Chloro-5- {3-isobutyl-1-[2-(4-methoxy-phenyl)-ethyl]-2,4-dioxo-1,3,8-triaza-spiro[4.5]dec-8-yl}-phenoxy)-acetic acid; 2-(5-{3-Isobutyl-1-[2-(4-methoxy-phenyl)-ethyl]-2,4-dioxo-1,3,8-triaza-spiro[4.5]dec-8-yl}-2-methyl-phenoxy)-2-methyl-propionic acid; 2-(2-Chloro-5-{3-isobutyl-1-[2-(4-methoxy-phenyl)-ethyl]-2,4-dioxo-1,3,8-triaza-spiro[4.5]dec-8-yl}-phenoxy)-2-methyl-propionic acid; 2-(2,3-Difluoro-5-{3-isobutyl-1-[2-(4-methoxy-phenyl)-ethyl]-2,4-dioxo-1,3,8-triaza-spiro[4.5]dec-8-yl}-phenoxy)-2-methyl-propionic acid; (3-{3-Isobutyl-1-[2-(4-medioxy-phenyl)-ethyl]-2-oxo-1,3,8-triaza-spiro[4.5]dec-8-yl}-phenyl)-acetic acid; (6-{3-Isobutyl-1-[2-(4-methoxy-phenyl)-ethyl]-2,4-dioxo-1,3,8-triaza-spiro[4.5]dec-8-yl}-pyridin-2-yl)-acetic acid; (2-{3-Isobutyl-1-[2-(4-methoxy-phenyl)-ethyl]-2,4-dioxo-1,3,8-triaza-spiro[4.5]dec-8-yl}-pyridin-4-yl)-acetic acid; (5-{3-Cyclobutylmethyl-1-[2-(2,4-dichloro-phenyl)-ethyl]-2,4-dioxo-1,3,8-triaza-spiro[4.5]dec-8-yl}-2-methyl-phenyl)-acetic acid; 2-(5-{3-Cyclobutylmethyl-1-[2-(2,4-dichloro-phenyl)-ethyl]-2,4-dioxo-1,3,8-triaza-spiro[4.5]dec-8-yl}-2-methyl-phenoxy)-2-methyl-propionic acid; (2-Chloro-5-{3-cyclobutylmethyl-1-[2-(2,4-dichloro-phenyl)-ethyl]-2,4-dioxo-1,3,8-triaza-spiro[4.5]dec-8-yl}-phenoxy)-acetic acid; 2-(2-Chloro-5-{3-cyclobutylmethyl-1-[2-(2,4-dichlorophenyl)-ethyl]-2,4-dioxo-1,3,8-triaza-spiro[4.5]dec-8-yl}-phenoxy)-2-methyl-propionic acid; (6-{3-Cyclobutylmethyl-1-[2-(2,4-ichloro-phenyl)-ethyl]-2,4-dioxo-1,3,8-triaza-spiro[4.5]dec-8-yl}-pyridin-2-yl)-acetic acid; (4-{3-Isobutyl-1-[2-(4-methoxy-phenyl)-ethyl]-2,4-dioxo-1,3,7-triaza-spiro[4.5]dec-7-ylmethyl}-phenoxy)-acetic acid; (3-{3-Cyclobutylmethyl-1-[2-(4-methoxy-phenyl)-ethyl]-2,4-dioxo-1,3,8-tiaza-spiro[4.5]dec-8-yl}-pbenyl)-acetic acid; {3-[1-[2-(4-Methoxy-phenyl)-ethyl]-3-(3-methyl-butyl)-2,4-dioxo-1,3,8-hiaza-spiro[4.5]dec-8-yl]-phenyl}-acetic acid; (3-{3-Butyl-1-[2-(4-methoxyphenyl)-ethyl]-2,4-dioxo-1,3,8-triaza-spiro[4.5]dec-8-yl}-phenyl)-acetic acid; 2-(4-{3-Isobutyl-1-[2-(4-methoxy-phenyl)-ethyl]-2,4-dioxo-1,3,8-triaza-spiro[4.5]dec-8-yl}-pyrimidin-2-yloxy)-2-methyl-propionic acid; 2-(6-{3-Isobutyl-1-[2-(4-methoxy-phenyl)-ethyl]-2,4-dioxo-1,3,8-triaza-spiro[4.5]dec-8-yl}-pyrimidin-4-yloxy)-2-methyl-propionic acid; 2-(4-{3-Cyclobutylmethyl-1-[2-(2,4-dichloro-phenyl)-ethyl]-2,4-dioxo-1,3,8-triaza-spiro[4.5]dec-8-yl}-pyrimidin-2-yloxy)-2-methyl-propionic acid; 2-(2-{3-Cyclobutylmethyl-1-[2-(2,4-dichloro-phenyl)-ethyl]-2,4-dioxo-1,3,8-triaza-spiro[4.5]dec-8-yl}-pyrimidin-4-yloxy)-2-methyl-propionic acid; 2-(6-{3-Cyclobutylmethyl-1-[2-(2,4-dichloro-phenyl)-ethyl]-2,4-dioxo-1,3,8-triaza-spiro[4.5]dec-8-yl}-pyrimidin-4-yloxy)-2-methyl-propionic acid; {3-[3-Cyclobutylmethyl-2,4-dioxo-1-(4-trifluoromethoxy-benzyl)-1,3,8-triaza-spiro[4.5]dec-8-yl]-phenyl}-acetic acid; {3-[3-Cyclobutylmethyl-2,4-dioxo-1-(4-trifluoromethyl-benzyl)-1,3,8-triaza-spiro[4.5]dec-8-yl]-phenyl}-acetic acid; {3-[3-Cyclopentylmethyl-2,4-dioxo-1-(4-trifluoromethyl-benzyl)-1,3,8-triaza-spiro[4.5]dec-8-yl]-phenyl}-acetic acid; {3-[3-Cyclopentylmethyl-2,4-dioxo-1-(4-trifluoromethoxybenzyl)-1,3,8-triaza-spiro[4.5]dec-8-yl]-phenyl}-acetic acid; {3-[1-(2,4-Bis-trifluoromethyl-benzyl)-3-cyclopentylmethyl-2,4-dioxo-1,3,8-triaza-spiro[4.5]dec-8-yl]-phenyl}-acetic acid; {3-[3-Cyclobutylmethyl-2,4-dioxo-1-(3-trifluoromethoxy-benzyl)-1,3,8-triaza-spiro[4.5]dec-8-yl]-phenyl}-acetic acid; (3-{3-Cyclobutylmethyl-2,4-dioxo-1-[4-(4-trifluoromethyl-phenyl)-thiazol-2-ylmethyl]-1,3,8-triaza-spiro[4.5]dec-8-yl}-phenyl)-acetic acid; (3-{3-Cyclopropylmethyl-2,4-dioxo-1-[4-(4-trifluoromethyl-phenyl)-thiazol-2-ylmethyl]-1,3,8-triaza-spiro[4.5]dec-8-yl}-phenyl)-acetic acid; {3-[3-(3-Methyl-but-2-enyl)-2,4-dioxo-1-(4-trifluoromethoxy-benzyl)-1,3,8-triaza-spiro[4.5]dec-8-yl]-phenyl}-acetic acid; {3-[1-[2-(4-Bromo-phenyl)-2-hydroxy-ethyl]-3-(3-methyl-butyl)-2,4-dioxo-1,3,8-bsaza-spiro[4.5]dec-8-yl]-phenyl}-acetic acid; {3-[1-[2-(4-Chloro-phenyl)-2-hydroxy-ethyl]-3-(3-methyl-butyl)-2,4-dioxo-1,3,8-triaza-spiro[4.5]dec-8-yl]-phenyl}-acetic acid; {3-[2,4-Dioxo-3-pentyl-1-(4-trifluoromethoxy-benzyl)-1,3,8-triaza-spiro[4.5]dec-8-yl]-phenyl}-acetic acid; {3-[3-(2,2-Dimethyl-propyl)-2,4-dioxo-1-(4-trifluoromethoxy-benzyl)-1,3,8-triaza-spiro[4.5]dec-8-yl]-phenyl}-acetic acid; {3-[3-(2-Ethyl-butyl)-2,4-dioxo-1-(4-trifluoromethoxy-benzyl)-1,3,8-triaza-spiro[4.5]dec-8-yl]-phenyl}-acetic acid; {3-[3-(4-Fluoro-butyl)-2,4-dioxo-1-(4-trifluoromethoxy-benzyl)-1,3,8-triaza-spiro[4.5]dec-8-yl]-phenyl}-acetic acid; {3-[3-(4-Methyl-pentyl)-2,4-dioxo-1-(4-trifluoromethoxy-benzyl)-1,3,8-triaza-spiro[4.5]dec-8-yl]-phenyl}-acetic acid; {3-[3-Cyclohexylmethyl-2,4-dioxo-1-(4-trifluoromethoxy-benzyl)-1,3,8-triaza-spiro[4,5]dec-8-yl]-phenyl}-acetic acid; {3-[2,4-Dioxo-3-(3-pyrrol-1-yl-propyl)-1-(4-trifluoromethoxybenzyl)-1,3,8-triaza-spiro[4.5]dec-8-yl]-phenyl}-acetic acid; {3-[3-(3,3-Dimethyl-2-oxobutyl)-2,4-dioxo-1-(4-trifluoromethoxy-benzyl)-1,3,8-triaza-spiro[4.5]dec-8-yl]-phenyl}-acetic acid; {3-[2,4-Dioxo-3-(4,4,4-trifluoro-butyl)-1-(4-trifluoromethoxy-benzyl)-1,3,8-triaza-spiro[4.5]dec-8-yl]-phenyl}-acetic acid; {3-[3-(2-Cyclohexyl-ethyl)-2,4-dioxo-1-(4-trifluoromethoxy-benzyl)-1,3,8-triaza-spiro[4.5]dec-8-yl]-phenyl}-acetic acid; {3-[3-(2-Ethyl-hexyl)-2,4-dioxo-1-(4-trifluoromethoxy-benzyl)-1,3,8-triaza-spiro[4.5]dec-8-yl]-phenyl}-acetic acid; {3-[3-(2-Methyl-butyl)-2,4-dioxo-1-(4-trifluoromethoxy-benzyl)-1,3,8-triaza-spiro[4.5]dec-8-yl]-phenyl}-acetic acid; {3-[2,4-Dioxo-3-(3,4,4-trifluoro-but-3-enyl)-1-(4-trifluoromethoxy-benzyl)-1,3,8-triaza-spiro[4.5]dec-8-yl]-phenyl}-acetic acid; {3-[3-(3,3-Dimethyl-butyl)-2,4-dioxo-1-(4-trifluoromethoxy-benzyl)-1,3,8-triaza-spiro[4.5]dec-8-yl]-phenyl}-acetic acid; {3-[1-(2,4-Dichloro-5-fluoro-benzyl)-3-(3,3-dimethyl-butyl)-2,4-dioxo-1,3,8-triaza-spiro[4.5]dec-8-yl]-phenyl}-acetic acid; {3-[1-(2,4-Dichloro-5-fluom-benzyl)-3-(4-fluoro-butyl)-2,4-dioxo-1,3,8-triaza-spiro[4.5]dec-8-yl]-phenyl}-acetic acid; (3-{3-Cyclobutylmethyl-1-[2-(2,4-dichloro-phenyl)-ethyl]-2,4-dioxo-1,3,8-triaza-spiro[4.5]dec-8-yl}-phenyl)-acetic acid; (3-{3-Cyclopentylmethyl-2,4-dioxo-1-[2-(4-trifluoromethyl-phenyl)-ethyl]-1,3,8-triaza-spiro[4.5]dec-8-yl}-phenyl)-acetic acid; (3-{3-Cyclopentylmethyl-1-[2-(2,4-dichloro-phenyl)-ethyl]-2,4-dioxo-1,3,8-triaza-spiro[4.5]dec-8-yl}-phenyl)-acetic acid; (3-{3-Cyclohexylmethyl-1-[2-(2,4-dichlorophenyl)-ethyl]-2,4-dioxo-1,3,8-triaza-spiro[4.5]dec-8-yl}-phenyl)-acetic acid; and (3-{1-[2-(4-Chloro-phenyl)-ethyl]-3-cyclopentylmethyl-2,4-dioxo-1,3,8-triaza-spiro[4.5]dec-8-yl}-phenyl)-acetic acid.

Further preferred compounds of the invention are detailed in the Examples, *infra.*

### Pharmacology and Utility

Compounds of the invention modulate the activity of PPARs and, as such, are useful for treating diseases or disorders in which PPARs contributes to the pathology and/or symptomology of the disease. This invention further provides compounds of this invention for use in the preparation of medicaments for the treatment of diseases or disorders in which PPARs contributes to the pathology and/or symptomology of the disease.

Such compounds may therefore be employed for the treatment of prophylaxis, dyslipidemia, hyperlipidemia, hypercholesteremia, atherosclerosis, atherogenesis, hypertriglyceridemia, heart failure, hyper cholesteremia, myocardial infarction, vascular diseases, cardiovascular diseases, hypertension, obesity, cachexia, HIV wasting syndrome, inflammation, arthritis, cancer, Alzheimer's disease, anorexia, anorexia nervosa, bulimia, skin disorders, respiratory diseases, ophthalmic disorders, IBDs (irritable bowel disease), ulcerative colitis and Crohn's disease. Preferably for the treatment of prophylaxis, dyslipidemia, hyperlipidemia, hypercholesteremia, atherosclerosis, atherogenesis, hypertriglyceridemia, cardiovascular diseases, hypertension, obesity, inflammation, cancer, skin disorders, IBDs (irritable bowel disease), ulcerative colitis and Crohn's disease.

Compounds of the invention can also be employed to treat long term critical illness, increase muscle mass and/or muscle strength, increase lean body mass, maintain muscle strength and function in the elderly, enhance muscle endurance and muscle function, and reverse or prevent frailty in the elderly.

Further, the compounds of the present invention may be employed in mammals as hypoglycemic agents for the treatment and prevention of conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome X. Preferably type-1 and type-2 diabetes, Impaired Glucose Metabolism (IGM), Impaired Glucose Tolerance (IGT) and Impaired Fasting Glucose (IFG).

In accordance with the foregoing, the compounds of the present invention may be used to treat or prevent any of the diseases or disorders described above *(*See, *"Administration and Pharmaceutical Compositions", infra)* through the administration of a compound of the invention or a pharmaceutically acceptable salt thereof. For any of the above uses, the required dosage will vary depending on the mode of administration, the particular condition to be treated and the effect desired. The present invention also concerns: i) a compound of the invention or a pharmaceutically acceptable salt thereof for use as a medicament; and ii) the use of a compound of the invention or a pharmaceutically acceptable salt thereof for the manufacture of a medicament for preventing or treating any of the diseases or disorders described above.

### Administration and Pharmaceutical Compositions

In general, compounds of the invention will be administered in therapeutically effective amounts via any of the usual and acceptable modes known in the art, either singly or in combination with one or more therapeutic agents. A therapeutically effective amount can vary widely depending on the severity of the disease, the age and relative health of the subject, the potency of the compound used and other factors. In general, satisfactory results are indicated to be obtained systemically at daily dosages of from about 0.03 to 2.5mg/kg per body weight. An indicated daily dosage in the larger mammal, e.g. humans, is in the range from about 0.5mg to about 100mg, conveniently administered, e.g. in divided doses up to four times a day or in retard form. Suitable unit dosage forms for oral administration comprise from ca. 1 to 50mg active ingredient.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrollidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions can be sterilized and/or contain adjuvants, such as preserving, stabilizing, wetting or emulsifying agents, solution promoters, salts for regulating the osmotic pressure and/or buffers. In addition, they can also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations can also be used. Suitable formulations for topical application, e.g., to the skin and eyes, are preferably aqueous solutions, ointments, creams or gels well-known in the art. Such can contain solubilizers, stabilizers, tonicity enhancing agents, buffers and preservatives.

This invention also concerns a pharmaceutical composition comprising a therapeutically effective amount of a compound as described herein in combination with one or more pharmaceutically acceptable carriers.

Compounds of the invention can be administered in therapeutically effective amounts in combination with one or more therapeutic agents (pharmaceutical combinations).

Thus, the present invention also relates to pharmaceutical combinations, such as a combined preparation or pharmaceutical composition (fixed combination), comprising: 1) a compound of the invention as defined above or a pharmaceutical acceptable salt thereof; and 2) at least one active ingredient selected from:
a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; DPPIV (dipeptidyl peptidase IV) inhibitors such as DPP728, LAF237 (vildagliptin - Example 1 of WO 00/34241), MK-0431, saxagliptin, GSK23A ; an AGE breaker; a thiazolidone derivative (glitazone) such as pioglitazone, rosiglitazone, or *(R)*-1-{4-[5-methyl-2-(4-trifluoromethyl-phenyl)-oxazol-4-ylmethoxy]-benzenesulfonyl}-2,3-dihydro-1*H*-indole-2-carboxylic acid described in the patent application WO 03/043985, as compound 19 of Example 4, a non-glitazone type PPARγ agonist e.g. GI-262570;
b) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin;
c) an anti-obesity agent or appetite regulating agent such as phentermine, leptin, bromocriptine, dexamphetamine, amphetamine, fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;
d) anti-hypertensive agents, e.g., loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;
e) a HDL increasing compound;
f) Cholesterol absorption modulator such as Zetia® and KT6-971;
g) Apo-A1 analogues and mimetics;
h) thrombin inhibitors such as Ximelagatran;
i) aldosterone inhibitors such as anastrazole, fadrazole, eplerenone;
j) Inhibitors of platelet aggregation such as aspirin, clopidogrel bisulfate;
k) estrogen, testosterone, a selective estrogen receptor modulator, a selective androgen receptor modulator;
l) a chemotherapeutic agent such as aromatase inhibitors e.g. femara, anti-estrogen, topoisomerase I inhibitors, topoisomerase II inhibitors, microtubule active agents, alkylating agents, antineoplastic antimetabolites, platin compounds, compounds decreasing the protein kinase activity such as a PDGF receptor tyrosine kinase inhibitor preferably Imatinib ({N-{5-[4-(4-methyl-pipeiazino-methyl)-benzoylamido]-2-methylphenyl}-4-(3-pyridyl)-2-pyrimidine-amine }) described in the European patent application EP-A-0 564 409 as example 21 or 4-Methyl-N-[3-(4-methyl-imidazol-1-yl)-5-trifluoromethyl-phenyl]-3-(4-pyridin-3-yl-pyrimidin-2-ylamino)-benzamide described in the patent application WO 04/005281 as example 92; and
m) an agent interacting with a 5-HT₃ receptor and/or an agent interacting with 5-HT₄ receptor such as tegaserod described in the US patent No. 5510353 as example 13, tegaserod hydrogen maleate, cisapride, cilansetron;
or, in each case a pharmaceutically acceptable salt thereof; and optionally a pharmaceutically acceptable carrier.

Most preferred combination partners are tegaserod, imatinib, vildagliptin, metformin, a thiazolidone derivative (glitazone) such as pioglitazone, rosiglitazone, or (*R*)-1-{4-[5-methyl-2-(4-trifluoromethyl-phenyl)-oxazol-4-ylmethoxy]-benzenesulfonyl}-2,3-dihydro-1*H*-indole-2-carboxylic acid, a sulfonylurea receptor ligand, aliskiren, valsartan, orlistat or a statin such as pitavastatin, simvastatin, fluvastatin or pravastatin.

Preferably the pharmaceutical combinations contains a therapeutically effective amount of a compound of the invention as defined above, in a combination with a therapeutically effective amount of another therapeutic agent as described above, e.g., each at an effective therapeutic dose as reported in the art. Combination partners (1) and (2) can be administered together, one after the other or separately in one combined unit dosage form or in two separate unit dosage forms. The unit dosage form may also be a fixed combination.

The structure of the active agents identified by generic or trade names may be taken from the actual edition of the standard compendium "The Merck Index" or the Physician's Desk Reference or from databases, e.g. Patents International (e.g. IMS World Publications) or Current Drugs.
Any person skilled in the art is fully enabled to identify the active agents and, based on these references, likewise enabled to manufacture and test the pharmaceutical indications and properties in standard test models, both in vitro and in vivo.

In another preferred aspect the invention concerns a pharmaceutical composition (fixed combination) comprising a therapeutically effective amount of a compound as described herein, in combination with a therapeutically effective amount of at least one active ingredient selected from the above described group a) to m), or, in each case a pharmaceutically acceptable salt thereof.

A pharmaceutical composition or combination as described herein for the manufacture of a medicament for the treatment of for the treatment of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

Such therapeutic agents include estrogen, testosterone, a selective estrogen receptor modulator, a selective androgen receptor modulator, insulin, insulin derivatives and mimetics; insulin secretagogues such as the sulfonylureas, e.g., Glipizide and Amaryl; insulinotropic sulfonylurea receptor ligands, such as meglitinides, e.g., nateglinide and repaglinide; insulin sensitizers, such as protein tyrosine phosphatase-1B (PTP-1B) inhibitors, GSK3 (glycogen synthase kinase-3) inhibitors or RXR ligands; biguanides, such as metformin; alpha-glucosidase inhibitors, such as acarbose; GLP-1 (glucagon like peptide-1), GLP-1 analogs, such as Exendin-4, and GLP-1 mimetics; DPPIV (dipeptidyl peptidase IV) inhibitors, e.g. isoleucin-thiazolidide; DPP728 and LAF237, hypolipidernic agents, such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin, fluindostatin and rivastatin, squalene synthase inhibitors or FXR (liver X receptor) and LXR (farnesoid X receptor) ligands, cholestyramine, fibrates, nicotinic acid and aspirin. A compound of the present invention may be administered either simultaneously, before or after the other active ingredient, either separately by the same or different route of administration or together in the same pharmaceutical formulation.

The invention also provides for pharmaceutical combinations, e.g. a kit, comprising: a) a first agent which is a compound of the invention as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.
The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g. a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Processes for Making Compounds of the Invention

The present invention also includes processes for the preparation of compounds of the invention. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

Compounds of Formula 4 can be prepared by proceeding as in reaction scheme 1: 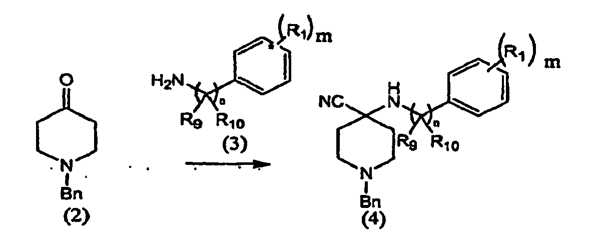 in which m, R₁, R₉, R₁₀ and n are as defined for Formula I. Compounds of Formula 4 are prepared by reacting a compound of formula 2 with a compound of formula 3 in the presence of a suitable solvent (for example, Acetic Acid, and the like) and a suitable reagent (for example, trimethyl-silyl-cyanide, and the like). The reaction is carried out in the temperature range of about 0 to about 50°C and takes up to about 24 hours to complete.

Compounds of Formula 5 can be prepared by proceeding as in reaction scheme 2: 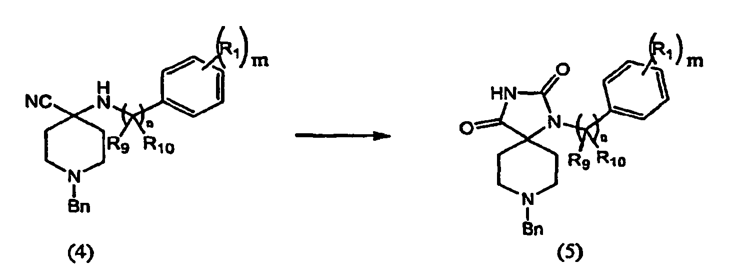 in which m, R₁, R₉, R₁₀ and n are as defined for Formula I. Compounds of Formula 5 are prepared by first forming an intermediate by reacting a compound of formula 4 with a suitable reagent (for example, chlorosulfonylisocyanate, and the like) and a suitable solvent (for example, DCM, and the like). The reaction is carried out at a temperature range of about 0 to about 50°C and takes up to about 2 hours to complete. Secondly, the intermediate is treated with a suitable acid (for example, 1M HCl in water, and the like) in a temperature range of about 80 to about 120°C and takes up to about 6 hours to complete.
Compounds of Formula 6 can be prepared by proceeding as in reaction scheme 3: 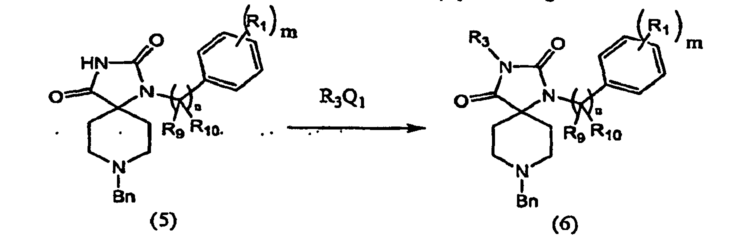 in which m, R₁, R₃, R₉, R₁₀ and n are as defined for Formula I; and Q₁ is a halogen, preferably Cl, I or Br. Compounds of formula 6 are formed by reacting a compound of formula 5 with R₃Q₁ in the presence of a suitable solvent (for example, DMSO, and the like) and a suitable base (for example, potassium carbonate, and the like). The reaction is carried out in the temperature range of about 25 to about 75°C and takes up to about 24 hours to complete.

Compounds of Formula 7 can be prepared by proceeding as in reaction scheme 4: 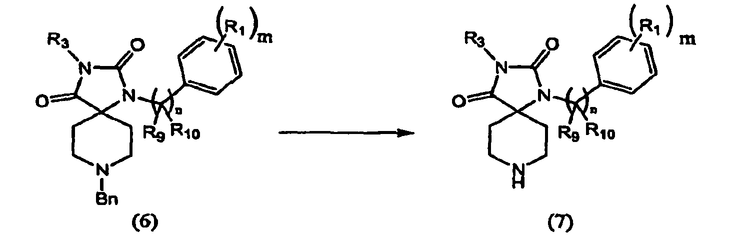 in which m, R₁, R₃, R₉, R₁₀ and n are as defined for Formula **I.** Compounds of Formula 7 are prepared by deprotecting a compound of formula 6 in the presence of a suitable solvent (for example, methanol, and the like), a suitable catalyst (for example, palladium on charcoal, and the like), a suitable acid (for example, HCl, and the like) and a suitable reducing agent (for example, hydrogen, and the like). The reaction is carried out in the temperature range of about 0 to about 50°C and takes up to about 24 hours to complete.
Compounds of Formula 9 can be prepared by proceeding as in reaction scheme 5: 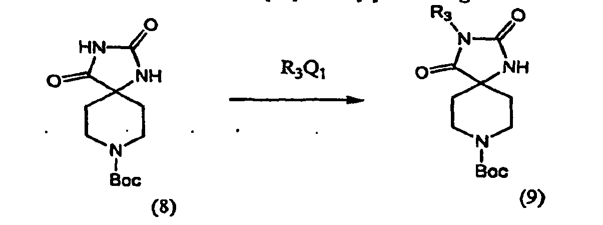 in which R₃ is as defined for Formula I; and Q₁ is a halogen, preferably Cl, I or Br. Compounds of Formula 9 are formed by reacting a compound of formula 8 with R₃Q₁ in the presence of a suitable solvent (for example, DMF, and the like) and a suitable base (for example, cesium bicarbonate, and the like). The reaction is carried out in the temperature range of about 25 to about 75°C and takes up to about 24 hours to complete.

Compounds of Formula 11 can be prepared by proceeding as in reaction scheme 6: 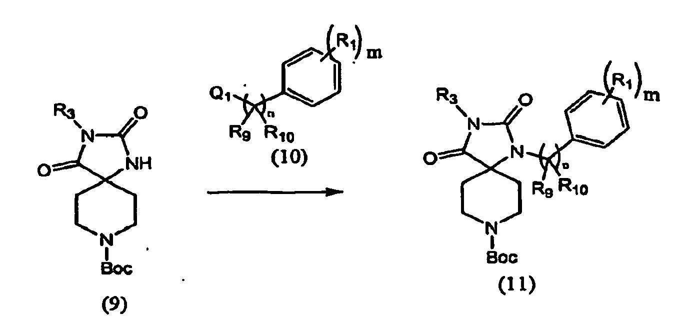 in which m, R₁, R₃, R₉, R₁₀ and n are as defined for Formula I; and Q₁ is a halogen, preferably Cl, I or Br. Compounds of formula 11 are formed by reacting a compound of formula 9 with a compound of formula 10 in the presence of a suitable solvent (for example, DMF, DME, and the like) and a suitable base (for example, cesium carbonate, KF-Al₂O₃. and the like). The reaction mixture can be subjected to microwave radiation. The reaction is carried out in the temperature range of about 100 to about 150°C and takes up to about 30 minutes to complete.

Compounds of Formula 7 can be prepared by proceeding as in reaction scheme 6: 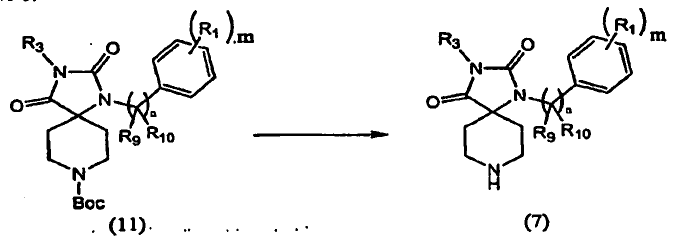 in which R₁, R₂, R₃, R₉, R₁₀ and n are as defined for Formula I. Compounds of Formula 7 are prepared by deprotecting a compound of formula 11 in the presence of a suitable solvent (for example, DCM, and the like) and a suitable acid (for example, TFA, and the like). The reaction is carried out in the temperature range of about 0 to about 50°C and takes up to about 5 hours to complete.

Compounds of Formula I, wherein Z is a bond, can be prepared by proceeding as in reaction scheme 7: 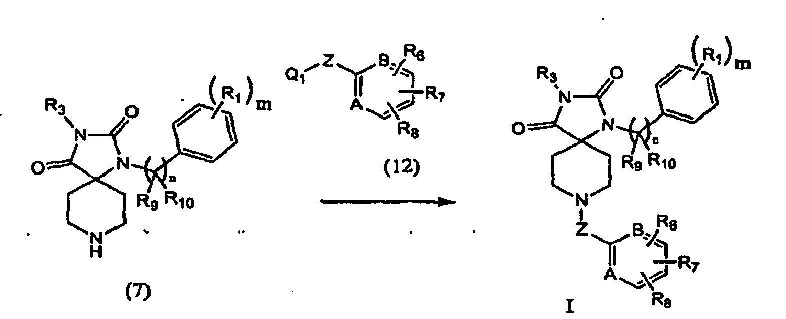 in which n, m, A, B, R₁, R₃, R₆, R₇, R₈, R₉ and R₁₀ are as defined for Formula I; and Q₁ is preferably chloro, iodo or bromo. Compounds of Formula I are prepared by reacting a compound of formula 7 with a compound of formula 12 in the presence of a suitable solvent (for example, 1,4-dioxane, and the like), a suitable catalyst (for example, Pd₂(dba)₃, and the like), a suitable ligand (for example, phosphine ligands such as (tBU)₃PHBF₃, and the like), a suitable inorganic base (for example, Cesium carbonate, and the like) under a suitable protective atmosphere (for example, argon, and the like). The reaction is carried out in the temperature range of about 80 to about 150°C and takes up to about 24 hours to complete.

Compounds of Formula I, in which Z is -S(O)₀₋₂- (SO₂ shown), can be prepared by proceeding as in reaction scheme 8: 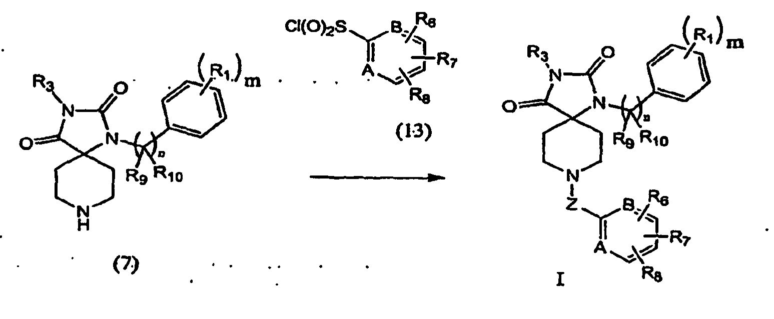 in which n, m, A, B, R₁, R₃, R₆, R₇, R₈, R₉ and R₁₀ are as defined for Formula L Compounds of Formula I are prepared by reacting a compound of formula 7 with a compound of formula 13 in the presence of a suitable solvent (for example, DCM, and the like), a suitable organic base (for example, triethylamine, and the like). The reaction is carried out in the temperature range of about 0 to about 50°C and takes up to about 24 hours to complete.

Compounds of Formula I, wherein Z is methylene, can be prepared by proceeding as in reaction scheme 9: 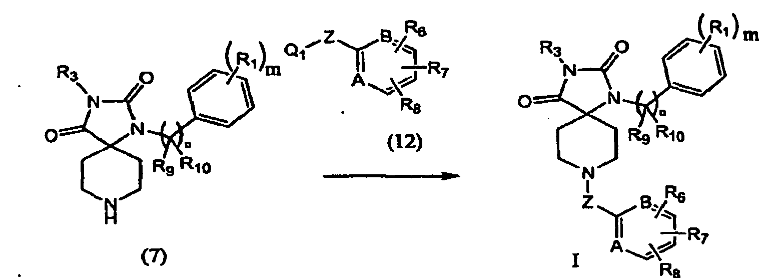 in which n, m, A, B, R₁, R₃, R₆, R₇, R₈, R₉ and R₁₀ are as defined for Formula I; and Q₁ is chloro, bromo or iodo. Compounds of Formula I are prepared by reacting a compound of formula 7 with a compound of formula 12 in the presence of a suitable solvent (for example, DCM, and the like) and a suitable base (for example, triethylamine, and the like). The reaction is carried out in the temperature range of about 0 to about 50°C and takes up to about 24 hours to complete.

Compounds of Formula I, wherein Z is a bond, can be prepared by proceeding as in reaction scheme 10: 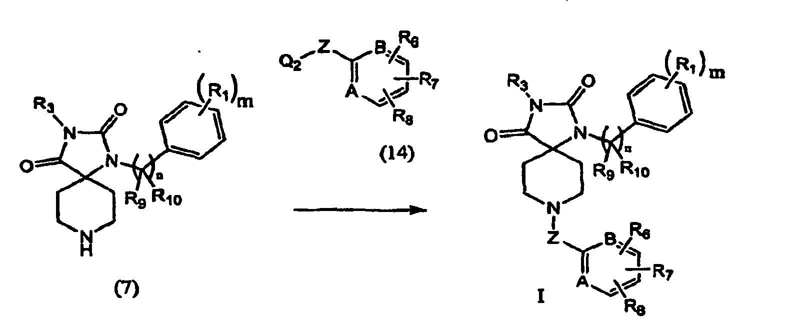 in which n, m, A, B, R₁, R₃, R₆, R₇, R₈, R₉ and R₁₀ are as defined for Formula I; and Q₂ is chloro, bromo, iodo or SO₂Me. Compounds of Formula I are prepared by reacting a compound of formula 7 with a compound of formula 14 in the presence of a suitable solvent (for example, n-butanol, and the like) and a suitable base (for example, diisopropylethylamine, and the like). The reaction is carried out in the temperature range of about 25 to about 75°C and takes up to about 24 hours to complete.

Compounds of Formula I, where R₁ is selected from -X₁CO₂R₁₃, - X₁CR₁₁R₁₂X₂CO₂R₁₃, -X₁SCR₁₁R₁₂X₂CO₂R₁₃ and -X₁OCR₁₁R₁₂X₂CO₂R₁₃ (and R₁₃ is C₁₋₆alkyl), are converted to their corresponding acids (where R₁₃ is hydrogen) via a saponification reaction. The reacting proceeds in the presence of a suitable base (e.g., lithium hydroxide, or the like) and a suitable solvent mixture (e.g., THF/water, or the like) and is carried out in the temperature range of about 0°C to about 50°C, taking up to about 30 hours to complete.

Detailed reaction conditions are described in the examples, *infra*.

### Additional Processes for Making Compounds of the Invention

A compound of the invention can be prepared as a pharmaceutically acceptable acid addition salt by reacting the free base form of the compound with a pharmaceutically acceptable inorganic or organic acid Alternatively, a pharmaceutically acceptable base addition salt of a compound of the invention can be prepared by reacting the free acid form of the compound with a pharmaceutically acceptable inorganic or organic base. Alternatively, the salt forms of the compounds of the invention can be prepared using salts of the starting materials or intermediate.

The free acid or free base forms of the compounds of the invention can be prepared from the corresponding base addition salt or acid addition salt from, respectively. For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Prodrug derivatives of the compounds of the invention can be prepared by methods known to those of ordinary skill in the art (e.g., for further details see Saulnier et al., (1994), Bioorganic and Medicinal Chemistry Letters, Vol. 4, p. 1985). For example, appropriate prodrugs can be prepared by reacting a non-derivatized compound of the invention with a suitable carbamylating agent (e.g., 1,1-acyloxyalkylcarbanochloridate, paranitrophenyl carbonate, or the like).

Protected derivatives of the compounds of the invention can be made by means known to those of ordinary skill in the art. A detailed description of techniques applicable to the creation of protecting groups and their removal can be found in T. W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:
(a) that of the reaction schemes detailed above; and
(b) optionally converting a compound of the invention into a pharmaceutically acceptable salt;
(c) optionally converting a salt form of a compound of the invention to a non-salt form;
(d) optionally converting an unoxidized form of a compound of the invention into a pharmaceutically acceptable N-oxide;
(e) optionally converting an N-oxide form of a compound of the invention to its unoxidized form;
(f) optionally resolving an individual isomer of a compound of the invention from a mixture of isomers;
(g) optionally converting a non-derivatized compound of the invention into a pharmaceutically acceptable prodrug derivative; and
(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### Examples

The present invention is further exemplified, but not limited, by the following intermediates and examples that illustrate the preparation of compounds of Formula I according to the invention.

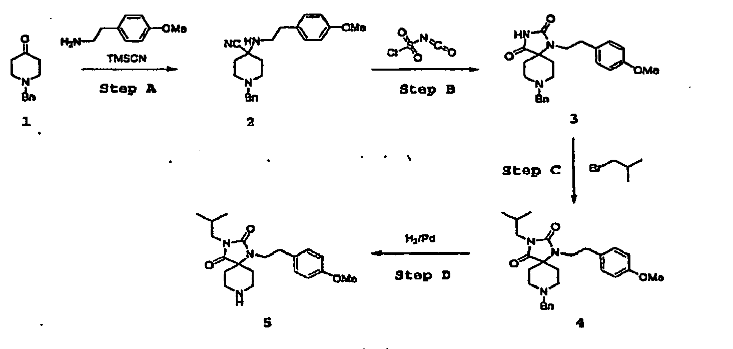

### Intermediate 5. 3-Isobutyl-1-[2-(4-methoxy-phenyl)-edxyl]-1,3,8-triaza-spho[4.5]decane-2,4-dione.

Step A: 1-Benzyl-piperidine4-one 1 (9.5 g, 50 mmol) is dissolved in AcOH (75 mL) and cooled to 0°C. 4-Methoxyphenethylamine (8.1 mL, 55 mmol) is added followed bv trimethylsilylcyanide (6.7 mL, 50 mmol). The ice-bath is removed and the mixture is stirred at rt for 20 h. Then the mixture is poured on ice-water, adjusted to pH 9 with aqueous ammonia and extracted with DCM twice. The organic layers are combined and concentrated. Recrystallization from ether affords **2** as a white solid. ¹H-NMR (400MHz, CDCl₃) δ = 7.26 (m, 5H), 7.07 (d, J = 8.5 Hz, 2H), 6.77 (d, J = 8.5 Hz, 2H), 3.72 (s, 3H), 3.46 (s, 2H), 2.90 (t, J = 6.9 Hz, 2H), 2.70 (m, 4H), 2.27 (m, 2H), 1.91 (m, 2H), 1.69 (m, 2H). MS calcd for C₂₂H₂₈N₃O (M+H⁺) 350.2, found 350.3.

Step B: 1-Bonzyl-4-[2-(4-methoxy-phenyl)-ethylamino]-pipendine-4-carbonitrile **2** (8.0 g, **23** mmol) is dissolved in DCM (100 mL) and cooled to 0°C. Chlorosulfonylisocyanate (2.2 mL, 25 mmol) is added, the ice-bath is removed and the mixture is stirred at rt for 1 h. Then the solvent is removed, 1 M HCl (100 mL) is added and the mixture is heated to reflux for **3** h. After adjusting the pH to 7, the mixture is extracted with DCM three times. The solvent is removed, and the remainder is triturated with MeCN to yield 8-benzyl-1-[2-(4-methoxy-phenyl)-ethyl]-1,3,8-triaza-spiro[4.5]decane-2,4-dione 3 as a colorless solid. ¹H-NMR (400MHz, CDCl₃) δ = 7.33 (m, 5H), 7.15 (d, J = 8.5 Hz, 2H), 6.84 (d, J = 8.5 Hz, 2H), 3.79 (s, 3H), 3.58 (s, 2H), 3.36 (t, J = 7.9 Hz, 2H), 2.91 (t, J = 7.9 Hz, 2H), 2.76 (m, 4H), 1.88 (m, 2H), 1.60. (m, 2H). MS calcd. for C₂₃H₂₈N₃O₃ (M+H⁺) 394.3, found 394.2.

**Step C:** The hydantoin **3** (8.2 g, 20.8 mmol), 1-bromo-2-methylpropane (2.83 mL, 26.1 mmol) and potassium carbonate (3.7 g, 27.1 mmol) in DMSO (50 mL) are stirred for 12 h at 50°C. The mixture is cooled to rt, diluted with EtOAc and washed with H₂O three times and with brine once. The organic layer is dried (MgSO₄), filtered and concentrated. The remainder is purified by flash chromatography (EtOAc/Hexanes gradient) to afford 8-benzyl-3-isobntyl-1-[2-(4-methoxy-phenyl)-ethyl]-1,3,8-triaza-spiro[4.5]decane-2,4-dione **4** () as a white solid: ¹H-NMR (400MHz, CDCl₃) δ = 7.32 (m, 5H), 7.13 (d, J = 8.7 Hz, 2H), 6.83 (d, J = 8.7 Hz, 2H), 3.78 (s, 3H), 3.58 (s, 2H), 3.38 (t, J = 7.8 Hz, 2H), 3.30 (d, J = 7.4 Hz, 2H), 2.92 (t, J = 7.8 Hz, 2H), 2.75 (m, 4H), 2.08 (m, 1H), 1.87 (m, 2H), 1.49 (m, 2H), 0.90 (d, J = 6.7 Hz, 6H). MS calcd. for C₂₇H₃₆N₃O₃ (M+H⁺) 450.3, found 450.2.

**Step D:** The hydantoin **4** (0.25 g, 0.56 mmol) is dissolved in MeOH (25 mL). A catalytic amount of palladium (10% on charcoal, 50 mg) is added followed by a catalytic amount (3 drops) of HCl cone. The mixture is put under 1 atm of hydrogen and stirred at rt for 20 h. The mixture is filtered over celite, washed with MeOH and dried in vacuo to yield 3-isobutyl-1-[2-(4-methoxy-phenyl)-ethyl]-1,3,8-triaza-spiro[4.5]decane-2,4-dione 5 (220 mg, quant) as a colorless glass: ¹H-NMR (400MHz, CDCl₃) δ = 7.14 (d, J = 8.5 Hz, 2H), 6.78 (d, J = 8.5 Hz, 2H), 3.72 (s, 3H), 3.57 (m, 2H). 3.42 (t, J = 7.3 Hz, 2H), 3.34 (m, 2H), 3.27 (d, J = 7.4 Hz, 2H), 2.93 (t, J = 7.3 Hz, 2H), 2.36 (m, 2H), 2.04 (m, 1H), 1.45 (m, 2H), 0.86 (d, J = 6.7 Hz, 6H). MS calcd. for C₂₀H₃₀N₃O₃ (M+H⁺) 360.2, found 360.2.

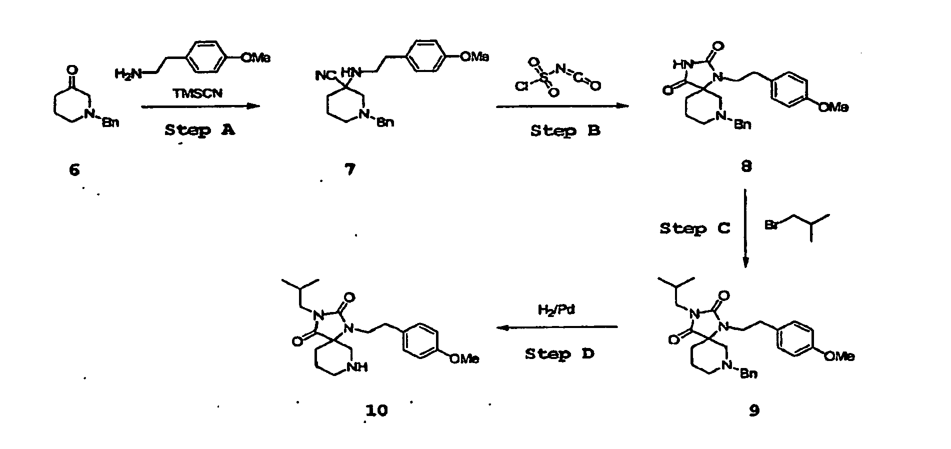

### Intermediate 10. 3-Isobutyl-1-[2-(4-methoxy-phenyl)-ethyl]-1,3,7-triaza-spiro[4.5]decane-2,4-dione.

Step A: 1-Benzy1-3-piperidinone hydrochloride hydrate **6** (3.0 g, 13.3 mmol) is dissolved in AcOH (30 mL) and cooled to 0°C. 4-Methoxyphenethylamine (2.1 mL, 14.6 mmol) is added followed by trimethylsilylcyanide (2.4 mL, 13.3 mmol). The ice-bath is removed and the mixture is stirred at rt for 20 h. Then the mixture is poured on ice-water, adjusted to pH 9 with aqueous ammonia and extracted with DCM twice. The organic layers are combined and concentratedto give a brown oil which is used directly in the next step without purification. MS calcd. for C₂₂H₂₈N₃O (M+H⁺) 350.2, found 350.2.

Step B: 1-Benzyl-3-[2-(4-methoxy-phenyl)-ethylamino]-piperidine-3-carbonitrile 7 (13.3 mmol) is dissolved in DCM (50 mL) and cooled to 0°C. Chlorosulfonylisocyanate (1.3 mL, 14.6 mmol) is added, the ice-bath is removed and the mixture is stirred at rt for 2 h. Then the solvent is removed, 1 M HCl (100 mL) is added and the mixture is heated to reflux for 3 h. After adjusting the pH to 7, the mixture is extracted with DCM three times. The solvent is removed, and the residue is purified on reverse phase HPLC (H₂O/MeCN gradient) to afford 7-Benzyl-1-[2-(4-methoxy-phenyl)-ethyl]-1,3,7-triaza-spiro[4.5]decane-2,4-dione 8 as a colorless oil. ¹H-NMR (400MHz, CDCl₃) δ = 7.16 (m, 5H), 7.08 (d, J = 8.8 Hz, 2H). 6.76 (d, J = 8.8 Hz, 2H), 3.98 (m, 1H), 3.70 (s, 3H), 3.65 (m, 1H), 3.39 (d, J = 13.3 Hz, 1H), 3.33 (d, J = 13.3 Hz, 1H), 2.90 (m, 1H), 2.78 (m, 1H), 2.67 (m, 1H). 2.53 (d, J = 12.0 Hz, 1H), 2.46 (d, J = 12.0 Hz, 1H), 2.13 (m, 1H), 1.79 (m, 3H), 1.62 (m, 1H). MS calcd. for C₂₃H₂₈N₃O₃ (M+H⁺) 394.3, found 394.2.

**Step C:** The hydantoin **8** (1.2 g, 3.0 mmol), 1-bromo-2-methylpropane (0.39 mL, 3.6 mmol) and potassium carbonate (0.54 g, 3.9 mmol) in DMSO (10 mL) are stirred for 12 h at 50°C. The mixture is cooled to rt, diluted with EtOAc and washed with H₂O three times and with brine once. The organic layer is dried (MgSO₄), filtered and concentrated to afford 7-Benzyl-3-isobutyl-1-[2-(4-methoxy-phenyl)-ethyl]-1,3,7-triaza-spiro[4.5]decane-2,4-dione **9** as a colorless oil which is used directly in Step D: ¹H-NMR (400MHz, CDCl₃) δ = 7.26 (m, 5H), 7.16 (d, J = 8.4 Hz, 2H), 6.84 (d, J = 8.4 Hz, 2H), 4.00 (m, 1H), 3.80 (s, 3H), 3.79 (m, 1H), 3.52 (d, J = 13.2 Hz, 1H), 3.40 (d, J = 13.2 Hz, 1H), 3.29 (d, J =7.6 Hz, 2H), 3.00 (m, 1H), 2.89 (m, 1H), 2.76 (m, 1H), 2.55 (m, 2H), 2.05 (m, 1H), 1.91 (m, 3H), 1.62 (m, 2H), 0.87 (d, J = 6.4 Hz, 6H). MS calcd. for C₂₇H₃₆N₃O₃ (M+H⁺) 450.3, found 450.3.

**Step D:** The hydantoin **9** (0.25 g, 0.56 mmol) is dissolved in AcOH (20 mL). A catalytic amount of palladium (10% on charcoal, 50 mg) is added and the mixture is pressurized to 60 psi of hydrogen and shaken for 20 h. The mixture is filtered over celite, neutralized with aqueous sodium bicarbonate and extracted with ethyl acetate. The organic fraction is washed with brine, dried (MgSO₄), filtered, and evaporated to yield 3-Isobutyl-1-[2-(4-methoxy-phenyl)-ethyl]-1,3,7-triaza-spiro[4.5]decane-2,4-dione **10** as a colorless glass: ¹H-NMR (400MHz, CDCl₃) δ = 7.06 (d, J = 8.8 Hz, 2H), 6.77 (d, J = 8.8 Hz, 2H), 3.72 (s, 3H), 3.63 (s, 1H), 3.35 (m, 2H), 3.28 (d, J = 7.2 Hz, 2H), 3.02 (m, 1H), 2.85 (t, J = 7.6 Hz, 2H), 2.68 (m, 2H), 2.47 (m, 1H), 2.05 (m, 2H), 1.65 (m, 2H), 0.83 (d, J = 6.8 Hz, 6H). MS calcd. for C₂₀H₃₀N₃O₃ (M+H⁺) 360.2, found 360.2.

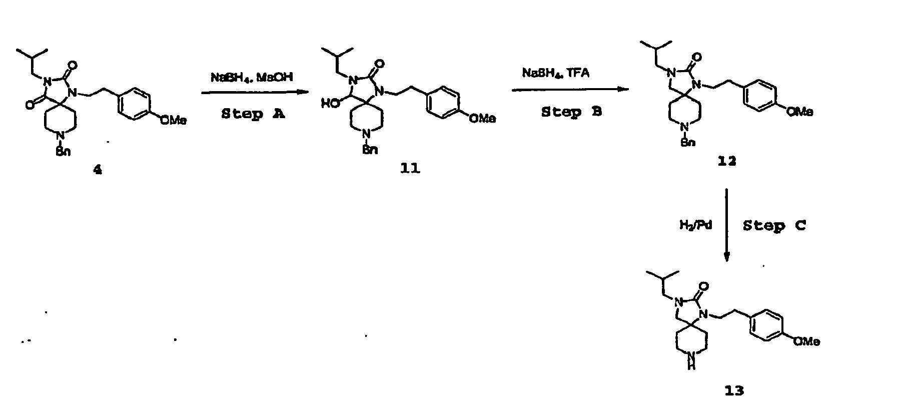

### Intermediate 13. 3-Isobutyl-1-[2-(4-methoxy-phenyl)-ethyl]-1,3,8-triaza-spiro[4.5]decan-2-one.

Step A: Intermediate 4 (45 mg, 0.1 mmol) is dissolved in MeOH (1.5 mL) and cooled to 0°C. Sodium borohydride (100 mg, 2.5 mmol) is added, and the mixture is stirred at 0°C for 30 min, then stirred for 48 h at room temperature. The crude 8-benzyl-4-hydroxy-3-isobutyl-1-[2-(4-methoxy-phenyl)-ethyl]-1,3,8-triaza-spiro[4.5]decan-2-one **11** is used in the next step without further purification. MS calcd. for C₂₇H₃₈N₃O₃ (M+H⁺) 452.3, found 452.3.

Step B: Intermediate 11 is dissolved in trifluoroacetic acid (1.5 mL) and cooled to 0°C. Sodium borohydride (40 mg, 1.0 mmol) is added, and the mixture is stirred at room temperature for 5 h. Then the reaction mixture is poured into ice water and extracted with EtOAc twice. The organic layers are combined, washed with water and concentrated to afford 12 as a white solid. ¹H-NMR (400MHz, CDCl₃) δ = 7.38-7.30 (m, 5H), 7.14 (d, J = 8.6 Hz, 2H), 6.81 (d, J =8.6 Hz, 2H), 3.77 (s, 3H), 3.52 (s, 2H), 3.21 (t, J = 8.0 Hz, 2H), 3.09 (s, 2H), 2.98 (d, J = 7.5 Hz, 2H), 2.88 (m, 2H), 2.80 (d, J = 8.0 Hz, 2H), 1.99 (m, 2H), 1.84 (m, 3H), 1.37 (m, 2H), 0.90 (d, J = 6.7 Hz, 6H). MS calcd. for C₂₂H₂₈N₃O (M+H⁺) 436.3, found 436.3.

**Step C:** The hydantoin **12** (35 mg, 0.08 mmol) is dissolved in MeOH. A catalytic amount of palladium (10% on charcoal, 50 mg) is added followed by a catalytic amount (3 drops) of HCl conc. The mixture is put under 1 atm of hydrogen and stirred at rt for 20 h. The mixture is filtered over celite, washed with MeOH and dried in vacuo to yield 3-Isobutyl-1-[2-(4-methoxy-phenyl)-ethyl]-1,3,8-triaza-spiro[4.5]decan-2-one 13 (22 mg, quant.) as a colorless glass: MS calcd. for C₂₀H_{32:}N₃O₂ (M+H⁺) 346.2, found 346.2.

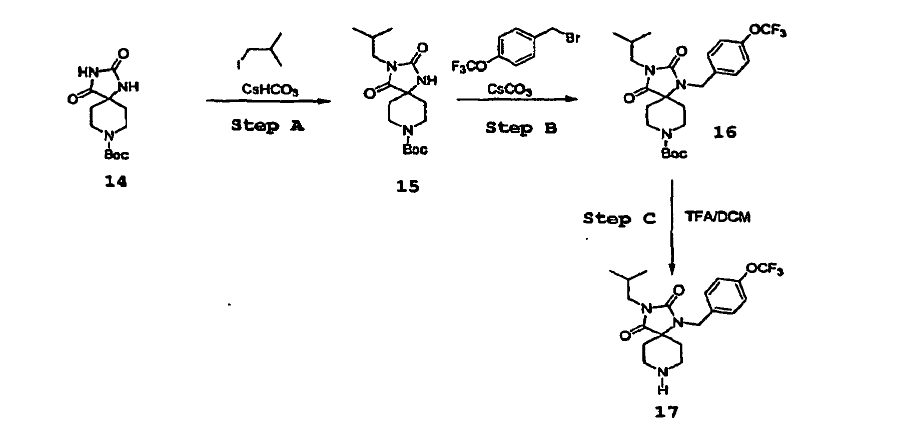

### Intermediate 17. 3-Propyl-1-(4-trifluoromethoxy-benzyl)-1,3,8-triaza-spiro[4.5]decane-2,4-dione.

**Step A.** A well stirred solution of **14** (2 g, 7.4 mmol) in anhydrous DMF (10 mL) is treated with CsHCO₃ (2.16 g, 11.1 mmol) and 1-Iodo-2-methyl-propane (2.0 g, 11.1 mmol). The reaction mixture is heated at 65°C for 8 hours. The reaction mixture is cooled down and quenched with water and extracted with EtOAc. The organic layer is washed once with 3N NaOH, water, brine, dried over Na₂SO₄, and concentrated to afford 3-Isobutyl-2,4-dioxo-1,3,8-triaza-spiro[4.5]decane-8-carboxylic acid tert-butyl ester **15** as white solid. LC/MS (M+H⁺) = 326.2.

**Step B.** A well stirred solution of **15** (0.15 g, 0.46 mmol) in anhydrous DMF (1 mL) is treated with Cs₂CO₃ (0.18 g, 0.55 mmol) and 1-bromomethyl-4-trifluoromethoxy-benzene (0.176 g, 0.69 mmol). The reaction mixture is irradiated in a microwave oven at 120°C for 20 min. The reaction mixture is directly purified by preparative LC/MS using a MeCN/water gradient 90-10%. The solvent is removed under vacuum to afford 3-Isobutyl-2,4-dioxo-1-(4-trifluoromethoxy-benzyl)-1,3,8-triaza-spiro[4.5]decane-8-carboxylic acid *tert*-butyl ester 16. ¹HMR (400 MHz, CDCl₃) δ 7.23 (d, J = 8.0 Hz, 2H), 7.10(d, J = 8.0 Hz, 2H), 4.42 (s, 2H), 3.97-3.94 (bm, 2H), 3.42-3.30 (m, 2H), 3.30 (d, J = 8.0 Hz, 2H) 2.05 (quint, J = 8.0 Hz, 1H), 1.68-1.64 (m, 2H) 1.49-1.46 (m, 2H), 1.38 (s, 9H), 0.85 (d, J = 8.0Hz, 6H). MS (M+H⁺) = 500.3.

**Step C.** 3-Isobutyl-2,4-dioxo-1-(4-trifluoromethoxy-benzyl)-1,3,8-triaza-spiro[4.5]decane-8-carboxylic acid *tert*-butyl ester **16** (0.15 g, 0.3 mmol), is dissolved in DCM (1 mL) and treated with a 50% solution of TFA /DCM (2 mL). The reaction mixture is stirred at room temperature for 1 h. The solvent is removed under vacuum to afford 17 as a TFA salt in quantitative yield. LC/MS(M+H⁺)= 400.2.

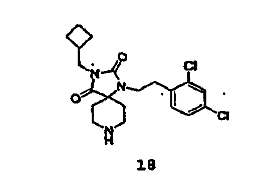

### Intermediate 18. 3-Cyclobutylmethyl-1-[2-(2,4-dichloro-phenyl)-ethyl]-1,3,8-triaza-spiro[4.5]decane-2,4-dione.

Following the procedure of Intermediate 5, except substituting 2,4-dichlorophenethylamine for 4-methoxyphenethylamine, and substituting (bromomethyl)cyclobutane for 1-bromo-2-methylpropane the title compound is prepared as a clear liquid: ¹H-NMR (400MHz, CDCl₃) δ = 7.38 (s, 1H), 7.18 (s, 2H), 3.52 (d, J = 7.4 Hz, 2H), 3.41 (t, J = 7.5 Hz, 2H), 3.34 (m, 2H), 3.11 (t, J = 7.3 Hz, 2H), 2.97 (m, 2H), 2.68 (m, 1H), 1.99 (m, 4H), 1.75 (m, 4H), 1.44 (d, J = 13.7 Hz, 2H). MS calcd. for C₂₀H₂₆Cl₂N₃O₂ (M+H⁺) 410.1, found 410.1.

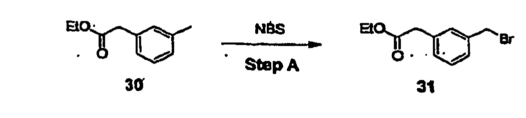

### Intermediate 31. (3-Bromomethyl-phenyl)-acetic acid ethyl ester.

Ethyl-m-tolylacetate 30 (2.00 g, 11.2 mmol) is dissolved in carbontetrachloride (30 mL). NBS (1.90 g, 10.7 mmol) is added followed by benzoyl peroxide (266 mg, 1.1 mmol). The mixture is heated to 75°C overnight. The mixture is diluted with DCM and washed with water and saturated aqueous NaHCO₃. The remainder is purified by flash chromatography (EtOAc/Hexanes gradient) to afford (3-bromomethyl-phenyl)-acetic acid ethyl ester 31 as a colorless oil: ¹H-NMR (400MHz, CDCl₃) δ = 7.36-7.22 (m, 4H), 4.48 (s, 2H), 4.16 (q, J = 7.1 Hz, 2H), 3.61 (s, 2H), 1.26 (t, J = 7.1 Hz, 3H). MS calcd. for C₁₁H₁₃BrO₂ (M+H⁺) 257.0, found 257.0.

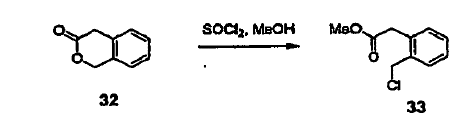

### Intermediate 33. (2-Chloromethyl-phenyl)-acetic acid methyl ester.

Isochromanone 32 (1.9 g, 13 mmol) is dissolved in MeOH (15 mL) and cooled to 0°C. Thionyl chloride (2 mL, 27.3 mmol) is added and the solution is stirred at rt for 48 h. The solvent is removed in vacuo, the remainder is dissolved in DCM and washed with water and saturated aqueous NaHCO₃. The organic layer is dried (MgSO₄), filtered and concentrated. Purification by flash chromatography (EtOAc/Hexanes gradient) affords the (2-chloromethyl-phenyl)-acetic acid methyl ester 33 as a colorless oil: ¹H-NMR (400MHz, CDCl₃) δ = 7.39-7.26 (m, 4H), 4.68 (s, 2H), 3.82 (s, 2H), 3.70 (s, 3H). MS calcd. for C₁₀H₁₁O₂ (M-Cl⁺) 163.1, found 163.1.

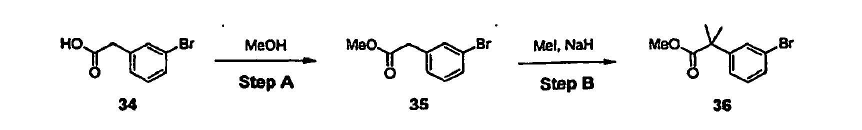

### Intermediate 36. 2-(3-Bromo-phenyl)-2-methyl-propionic acid methyl ester.

Step A: 3-Bromophenyl acetic acid 34 (1.17 g, 5.44 mmol) is dissolved in MeOH (15 mL) containing catalytic amounts of thionyl chloride (0.2 mL). The solution is stirred at rt overnight. The solvent is evaporated, the remainder is dissolved in DCM and washed with water and saturated aqueous NaHCO₃. The organic layer is dried (MgSO₄) filtered and concentrated to afford the methyl ester 35 as an oil: ¹H-NMR (400MHz, CDCl₃) δ = 7.44 (s, 1H), 7.40 (ddd, J = 2.0, 2.4, 6.8 Hz, 1H), 7.20 (m, 2H), 3.70 (s, 3H), 3.59 (s, 2H). MS calcd. for C₉H₁₀BrO₂ (M+H⁺) 229.1, found 229.0.

Step B: Intermediate 35 (1.0 g, 4.4 mmol) is dissolved in DMF (10 mL) and cooled to 0°C. Sodium hydride (60% dispersion, 1.6 g, 22.0 mmol) is added slowly and the mixture is stirred at 0°C until the gas evolution ceases. Then methyl iodide (1.5 mL, 22.0 mmol) is added and the mixture is stirred at ambient temperature for 2 h. The reaction mixture is carefully quenched with MeOH (5 mL) while stirring on an ice-bath. Water is added and the mixture is extracted with EtOAc twice. The combined organic layers are washed with water and brine, dried over MgSO₄ and concentrated. The crude remainder is purified by flash silica chromatography (EtOActhexanes gradient) to afford 2-(3-bromophenyl)-2-methyl-propionic acid methyl ester 36 as a clear liquid: ¹H-NMR (400MHz, CDCl₃) δ = 7.48 (t, J =1.9 Hz, 1H), 7.37 (m, 1H), 7.25 (m, 1H), 7.19 (t, J = 7.8 Hz, 1H), 3.66 (s, 3H), 1.56 (s, 6H). MS calcd. for C₁₁H₁₄BrO₂ (M+H⁺) 257.0, found 257.0.

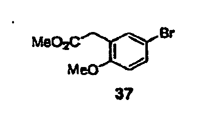

### Intermediate 37. (5-Bromo-2-methoxy-phenyl)-acetic acid methyl ester.

Following the procedure of Intermediate 36, Step A, except substituting (5-bromo-2-methoxy-phenyl)-acetic acid for 3-bromophenyl acetic acid, the title compound is prepared as a clear liquid: ¹H-NMR (400MHz, CDCl₃) δ = 7.35 (dd, J = 2.5 Hz, J = 8.7 Hz, 1H), 7.30 (d, J= 2.5 Hz, 1H), 6.74 (d, J = 8.7 Hz, 1H), 3.80 (s, 3H), 3.69 (s, 3H), 3.59 (s, 2H). MS calcd. for C₁₀H₁₂BrO₃ (M+H⁺) 259.0, found 259.0.

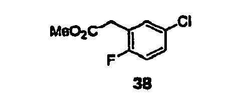

### Intermediate 38. (5-Chloro-2-fluoro-phenyl)-acetic acid methyl ester.

Following the procedure of Intermediate 36, Step A, except substituting (5-chloro-2-fluoro-phenyl)-acetic acid for 3-bromophenyl acetic acid, the title compound is prepared as a clear liquid: ¹H-NMR (400MHz, CDCl₃) δ = 7.27-7.20 (m, 2H), 7.00 (t, J = 8.9 Hz, 1H), 3.72 (s, 3H), 3.64 (s, 2H). MS calcd. for C₉H₉ClFO₂ (M+H⁺) 203.0, found 203.0.

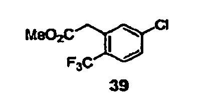

### Intermediate 39. (5-Chloro-2-trffluoromethyl-phenyl)-acetic acid methyl ester.

Following the procedure of Intermediate 36, Step A, except substituting (5-chloro-2-trifluoromethyl-phenyl)-acetic acid for 3-bromophenyl acetic acid, the title compound is prepared as a clear liquid: ¹H-NMR (400MHz, CDCl₃) δ = 7.59 (d, J = 8.4 Hz, 1H), 7.40 (s, 1H), 7.37 (d, J = 8.4 Hz, 1H), 3.80 (s, 2H), 3.72 (s, 3H). MS calcd. for C₁₀H₉ClF₂O₂ (M-F⁺) 233.0, found 233.0.

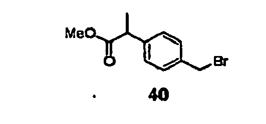

### Intermediate 40. 2-(4-Bromomethyl-phenyl)-propionic acid methyl ester.

Following the procedure of Intermediate 36, Step A, except substituting 2-(4-bromomethyl-phenyl)-propionic acid for 3-bromophenyl acetic acid, the title compound is prepared as a clear liquid: ¹H-NMR (400MHz, CDCl₃) δ = 7.31 (d, J = 8.1 Hz, 2H), 7.23 (d, J = 8.1 Hz, 2H), 4.43 (s, 2H), 3.68 (q, J = 7.2 Hz, 1H), 3.61 (s, 3H), 1.45 (d, J = 7.2 Hz, 3H). MS calcd. for C₁₁H₁₃BrO₂ (M+H⁺) 257.0, found 257.0.

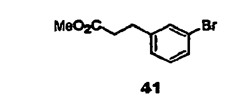

### Intermediate 41. 3-(3-Bromo-phenyl)-propionic acid methyl ester.

Following the procedure of Intermediate 36, Step A, except substituting 3-(3-bromo-phenyl)-propionic acid for 3-bromophenyl acetic acid, the title compound is prepared as a clear liquid: MS calcd. for C₁₀H₁₀BrO₂ (M+H⁺) 243.0, found 243.0.

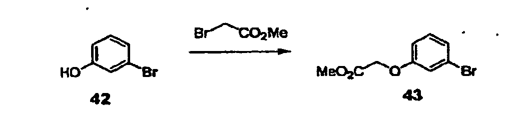

### Intermediate 43. (3-Bromo-phenoxy)-acetic acid methyl ester.

3-Bromo-phenol 42 (1.72 g, 10 mmol) together with methyl-bromoacetate (1.01 mL, 11 mmol) are dissolved in MeCN (600 mL). K₂CO₃ (2.07 g, 15 mmol) is added and the mixture is stirred at 50°C overnight. After insoluble salts are filtered and washed with MeCN, the solvent is removed and the remainder is taken up in EtOAc and washed subsequently with water and brine. The organic layer is dried (MgSO₄), filtered and concentrated to afford 43 as a colorless semi-solid: MS calcd. for C₉H₁₀BrO₃ (M+H⁺) 245.0, found 244.9.

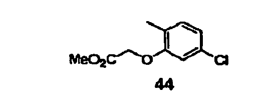

### Intermediate 44. (5-Chloxo-2-methyl-phenoxy)-acetic acid methyl ester.

Following the procedure of Intermediate 43, except substituting 5-chloro-2-methyl-phenol for 3-bromo-phenol, the title compound is prepared as a white solid: ¹H-NMR (400MHz, CDCl₃) δ = 7.07 (d, J = 8.0 Hz, 1H), 6.89 (dd, J = 1.9 Hz, J = 8.0 Hz, 1H), 6.68 (d, J = 1.9 Hz, 1H), 4.64 (s, 2H), 3.82 (s, 3H), 2.24 (s, 3H). MS calcd. for C₁₀H₁₂ClO₃ (M+H⁺) 215.0, found 215.0.

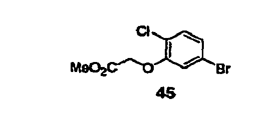

### Intermediate 45. (5-Bromo-2-chloro-phenoxy)-acetic acid methyl ester.

Following the procedure of Intermediate 43, except substituting 5-bromo-2-chloro-phenol for 3-bromo-phenol, the title compound is prepared as a white solid: ¹H-NMR (400MHz, CDCl₃) δ = 7.27 (d, J = 8.4 Hz, 1H), 7.11 (dd, J = 2.1 Hz, J = 8.4 Hz, 1H), 6.99 (d, J = 2.1 Hz, 1H), 4.73 (s, 2H), 3.85 (s, 3H). MS calcd. for C₉H₉BrClO₃ (M+H⁺) 278.9, found 279.0.

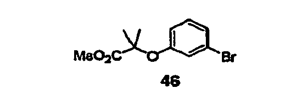

### Intermediate 46. 2-(3-Bromo-phenoxy)-2-methyl-propionic acid methyl ester.

Following the procedure of Intermediate 43, except substituting □□-dimethyl-methyl-bromoacetate for methyl-bromoacetate and heating to reflux, the title compound is prepared as a clear liquid: MS calcd. for C₁₁H₁₄BrO₃ (M+H⁺) 273.0, found 273.0.

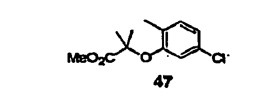

### Intermediate 47. 2-(5-Chloro-2-methyl-phenoxy)-2-methyl-propionic acid methyl ester.

Following the procedure of Intermediate 43, except substituting □□-dimethyl-methyl-bromoacetate for methyl-bromoacetate and heating to reflux, the title compound is prepared as a clear liquid: ¹H-NMR (400MHz, CDCl₃) δ = 7.06 (d, J = 8.0 Hz, 1H), 6.87 (dd, J = 2.0 Hz, J = 8.0 Hz, 1H), 6.62 (d, J = 2.0 Hz, 1H), 3.80 (s, 3H), 2.18 (s, 3H) , 1.60 (s, 6H). MS calcd. for C₁₂H₁₆ClO₃ (M+H⁺) 243.1, found 243.1.

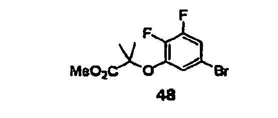

### Intermediate 48. 2-(5-Bromo-2,3-difluoro-phenoxy)-2-methyl-propionic acid methyl ester.

Following the procedure of Intermediate 43, except substituting 5-bromo-2,3-difluoro-phenol for 5-chloro-2-methyl-phenol, the title compound is prepared as a clear liquid: ¹H-NMR (400MHz, CDCl₃) δ = 7.05 (m, 1H), 6.91 (m, 1H), 3.80 (s, 3H), 1.60 (s, 6H). MS calcd. for C₁₁H₁₂BrF₂O₃ (M+H⁺) 309.0, found 309.0.

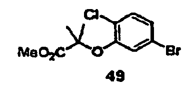

### Intermediate 49. 2-(5-Bromo-2-chloro-phenoxy)-2-methyl-propionic acid methyl ester.

Following the procedure of Intermediate 43, except substituting 5-bromo-2-chloro-phenol for 5-chloro-2-methyl-phenol, the title compound is prepared as a clear liquid: ¹H-NMR (400MHz, CDCl₃) δ = 7.23 (d, J = 8.5 Hz, 1H), 7.09 (dd, J = 2.0 Hz, J = 8.5 Hz, 1H), 7.03 (d, J = 2.0 Hz, 1H), 3.81 (s, 3H), 1.62 (s, 6H). MS calcd. for C₁₁H₁₃BrClO₃ (M+H⁺) 307.0, found 307.0.

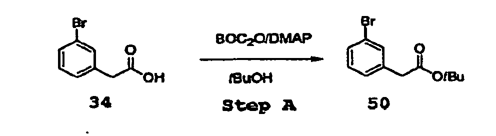

### Intermediate 50. (3-Bromo-phenyl)-acetic acid tert-butyl ester.

To a solution of 34 (11.2 g, 51.6 mmol) and Boc₂O (25 g, 114.5 mmol) in 525 mL of *t*BuOH is added DMAP (1.9 g). The reaction mixture is stirred overnight and checked by TLC till completion. After evaporation of the solvent the product is purified by a short column of silica gel (5-10 cm) up to 10% EtOAc to afford pure (3-bromo-phenyl)-acetic acid *tert*-butyl ester 50 as a colorless oil. ¹H-NMR (400MHz, CDCl₃) δ 7.3 6(m, 1H),7.34-7.30 (m, 1H),7.15-7.11 (m, 2H), 3.42 (s, 2H), 1.17 (s, 9H).

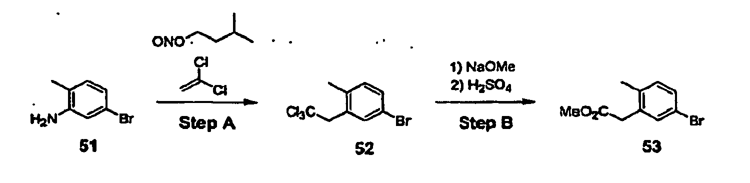

### Intermediate 53. (5-Bromo-2-methyl-phenyl)-acetic acid methyl ester.

Step A: In a flame-dried flask isoamyl nitrate (2.16 mL, 16 mmol) is dissolved in dry MeCN (6 mL). Then copper chloride (Cu(II)Cl₂, 1.74 g, 13 mmol) and vinylidene chloride (12.9 mL, 16 mmol) are added. 5-Bromo-2-methyl-aniline 51 (2.00 g, 11 mmol) is added slowly over a period of 10 min, while the mixture is kept at ambient temperature with a waterbath. The reaction mixture is stirred at room temperature overnight, then poured into ice-cold 20% aqueous HCl (80 mL). After stirring for 30 min it is extracted with ether twice, the combined organic layers are washed with 20% aqueous HCl, water and brine, dried over MgSO₄ and concentrated.

Step B: The crude 4-bromo-1-methyl-2-(2,2,2-thchloro-ethyl)-benzene **52** from Step A is dissolved in MeOH (2 mL) and cooled to 0°C. A solution of 30% NaOMe in MeOH (8.5 mL) is added slowly, then the mixture is heated to reflux for 5 h. After cooling back down to 0°C, H₂SO₄ conc. (1.6 mL) is added, and the mixture is heated to reflux for 1 h. The reaction mixture is cooled to room temperature, water is added and it is extracted with DCM three times. The combined organic layers are washed with water and brine, dried over MgSO₄ and concentrated. The residue is purified by flash silica chromatography (EtOAc/hexanes gradient) to yield (5-bromo-2-methyl-phenyl)-acetic acid methyl ester **53** as an oil: ¹H-NMR (400MHz, CDCl₃) δ = 7.34 (d, J = 2.0 Hz, 1H), 7.30 (dd, J = 2.0 Hz, J = 8.1 Hz, 1H), 7.05 (d, J = 8.1 Hz, 1H), 3.70 (s, 3H), 3.60 (s, 2H), 2.25 (s, 3H). MS calcd. for C₁₀H₁₂BrO₂ (M+H⁺) 243.0, found 243.0.

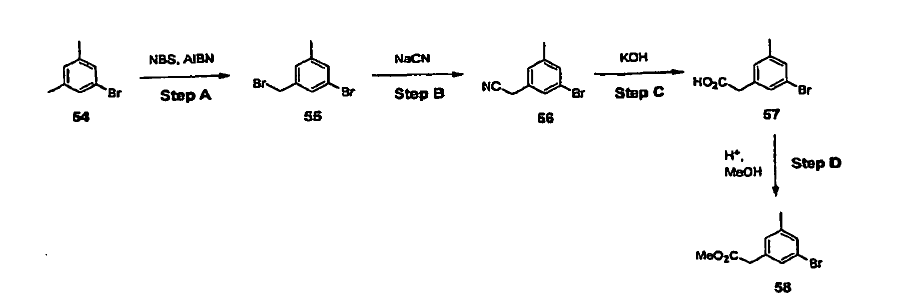

### Intermediate 58. (3-Bromo-5-methyl-phenyl)-acetic acid methyl ester.

**Step A:** 5-Bromo-meta-xylene **54** (1.85 g, 10 mmol), N-bromosuccinimide (1.78 g, 10 mmol) and AIBN (0.11 g, 0.7 mmol) are suspended in CCl₄ (20 mL). The reaction mixture is heated to reflux for 2 h, then the solids are filtered and the remainder is concentrated to give 1-bromo-3-bromomethyl-5-methyl-benzene 55 (2.7 g, quant.) as a white solid: MS calcd. for C₈H₉Br₂ (M+H⁺) 262.9, found 281.0.

**Step B:** Intermediate **55** (2.70 g, 10 mmol) is dissolved in DMSO (10 mL) and cooled to 0°C. Then sodium cyanide (0.98 g, 20 mmol) is added and the mixture is stirred at room temperature for 1 h. Acetonitrile (10 mL) is added and the mixture is heated to reflux for 90 min. Then it is diluted with H₂O and extracted with ether three times. The combined organic layers are washed with H₂O and brine, dried over MgSO₄ and concentrated to yield (3-bromo-5-methyl-phenyl)-acetonitrile 56 as a reddish oil. MS calcd. for C₉H₉BrN (M+H⁺) 210.0, found 210.0.

**Step C**: A high pressure tube is charged with KOH (2.24 g, 40 mmol) dissolved in H₂O (20 mL). Intermediate **56** (∼10 mmol) dissolved in isopropanol (10 mL) is added, the tube is sealed and heated to 120°C overnight. The mixture is then stirred at room temperature for 62 h. After the isopropanol is evaporated, the remainder is acidified with 6 M HCl to pH 2 and extracted with ether three times. The combined organic layers are washed with H₂O, dried over MgSO₄ and concentrated to yield (3-bromo-5-methyl-phenyl)-acetic acid 57 as a reddish solid. MS calcd. for C₉H₁₀BrO₂ (M+H⁺) 229.0, found 228.9.

Step D: (3-Bromo-5-methyl-phenyl)-acetic acid 57 is dissolved in MeOH (20 mL) containing catalytic amounts of thionyl chloride (0.2 mL). The solution is stirred at rt overnight. The solvent is evaporated, the remainder is dissolved in DCM and washed with water and saturated aqueous NaHCO₃. The organic layer is dried (MgSO₄), filtered and concentrated. The remainder is purified by flash silica chromatography (EtOAc/hexanes gradient) to afford (3-bromo-5-methyl-phenyl)-acetic acid methyl ester 58 as an oil: ¹H-NMR (400MHz, CDCl₃) δ = 7.24 (s, 2H), 7.02 (s, 1H), 3.71 (s, 3H), 3.56 (s, 2H), 2.32 (s, 3H). MS calcd. for C₁₀H₁₂BrO₂ (M+H⁺) 243.0, found 243.0.

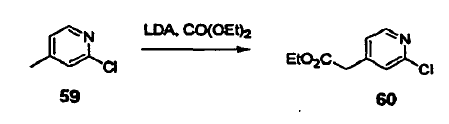

### Intermediate 59. (2-Chloro-pyridin-4-yl)-acetic acid ethyl ester.

2-Chlom-4-methyl-pyridine 59 (1.06 g, 8.33 mmol) is dissolved in THF (18 mL) and cooled to -78°C. LDA (10 mL, 20 mmol) is slowly added over a period of 15 min and stirred at -78°C for another 15 min. Then diethylcarbonate (1.2 mL, 10 mmol) is slowly added over a period of 5 min and stirred at -78°C for another 15 min. The mixture is then warmed to 0°C and stirred at that temperature for 4 h. After quenching with saturated ammonium chloride solution (250 mL) the solution is extracted with EtOAc three times. The combined organic layers are washed with H₂O, dried over Na₂SO₄ and concentrated. The remainder is purified by flash silica chromatography (EtOAc/hexanes gradient) to afford (2-chloro-pyridin-4-yl)-acetic acid ethyl ester **60** as an orange liquid: ¹H-NMR (400MHz, CDCl₃) δ = 832 (d, J = 5.1 Hz, 1H), 7.27 (d, J = 4.0 Hz, 1H), 7.15 (d, J = 5.0 Hz, 1H), 4.17 (q, J = 7.1 Hz, 2H), 3.60 (s, 2H), 1.26 (t, J = 7.1 Hz, 3H). MS calcd. for C₉H₁₁ClNO₂ (M+H⁺) 200.0, found 200.1.

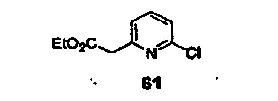

### Intermediate 61. (6-Chloro-pyridin-2-yl)-acetic acid ethyl ester.

Following the procedure of Intermediate **60,** except substituting 6-Chloro-2-methyl-pyridine for 2-Chloro-4-methyl-pyridine, the title compound is prepared as a clear liquid: ¹H-NMR (400MHz, CDCl₃) δ = 7.63 (t, J = 7.8 Hz, 1H), 7.24 (m, 2H), 4.18 (q, J = 7.1 Hz, 2H), 3.82 (s, 2H), 1.26 (t, J = 7.1 Hz, 3H). MS calcd. for C₉H₁₁ClNO₂ (M+H⁺) 200.0, found 200.1.

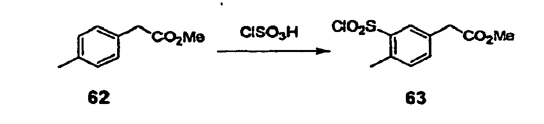

### Intermediate 63. (3-Chlorosulfonyl-4-methyl-phenyl)-acetic acid methyl ester.

p-Tolyl-acetic acid methyl ester **62** (1.0 g, 6.09 mmol) is dissolved in dichloromethane (4 mL) and cooled to 0°C. Chlorosulfonic acid (10 mL) is added dropwise while stirring during the period of 1 h. The mixture is warmed to rt and stirred for 1 h. The reaction mixture is diluted with EtOAc, and washed with saturated Na₂CO₃ and brine. The organic layer is separated, dried (MgSO₄), filtered and concentrated to give crude product, which is purified from silic gel chromatography (EtOAc/hexane gradient) to give the title compound 63 as an oil: ¹H-NMR (400MHz, CDCl₃) δ = 7.89 (d, J=1.6 Hz, 1H), 7.48 (dd, J = 1.6 Hz, J=7.6 Hz, 1H), 7.32 (d, 1=7.6 Hz, 1H), 3.66 (s, 3H), 3.63 (s, 2H), 2.70 (s, 3H); MS calcd. for C₁₀H₁₀O₄S (M-Cl⁺) 227.04, found 227.00.

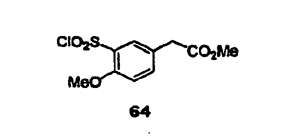

### Intermediate 64. (3-Chlorosulfonyl-4-methoxy-phenyl)-acetic acid methyl ester.

Intermediate **64** is prepared according to patent literature GB 2378179.

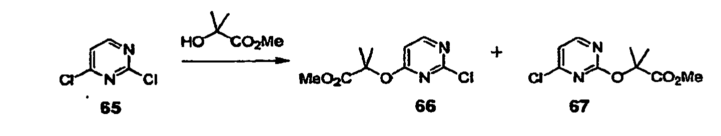

### Intermediates 66 and 67. 2-(2-Chloro-pyrimidin-4-yloxy)-2-methyl-propionic acid methyl ester and. 2-(4-Chloro-pyrimidin-2-yloxy)-2-methyl-propionic acid methyl ester.

2,4-Dichloropyrimidine **65** (0.90 g, 6.0 mmol) is dissolved in DMF (36 mL). 2-Hydroxy isobutyrate methylester (2.13 g, 18.0 mmol) and Cs2C03 (7.8 g, 24 mmol) are added and the mixture is subjected to microwave irradiation (120°C, 5 min). Then it is diluted with EtOAc and washed with H₂O three times, then with brine. The organic layer is dried over MgSO₄ and concentrated. The remainder is purified by flash silica chromatography (EtOAc/hexanes gradient) to afford regioisomers **66** and **67** in a 3:1 ratio as clear oils: **66:** ¹H-NMR (400MHz, CDCl₃) δ = 8.24 (d, J = 5.8 Hz, 1H), 6.61 (d, J = 5.8 Hz, 1H), 3.66 (s, 3H), 1.63 (s, 6H). MS calcd. for C₉H₁₂ClN₂O₃ (M+H⁺) 231.1, found 231.0. **67:** ¹H-NMR (400MHz, CDCl₃) δ = 8.26 (d, J = 5.4 Hz, 1H), 6.90 (d, J = 5.4 Hz, 1H), 3.61 (s, 3H), 1.65 (s, 6H). MS calcd. for C₉H₁₂ClN₂O₃ (M+H⁺) 231.1, found 231.0.

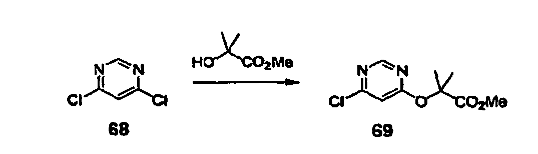

### Intermediate 69. 2-(6-Chloro-pyrimidin-4-yloxy)-2-methyl-propionic acid methyl ester.

4,6-Dichloropyrimidine **68** (0.90 g, 6.0 mmol) is dissolved in DMF (36 mL). 2-Hydroxy isobutyrate methylester (2.13 g, 18.0 mmol) and Cs₂CO₃ (7.8 g, 24 mmol) are added and the mixture is heated to 50°C for 12 h. Then it is diluted with EtOAc and washed with H₂O three times, then with brine. The organic layer is dried over MgSO₄ and concentrated. The remainder is purified by flash silica chromatography (EtOAc/hexanes gradient) to afford 69 as a clear oil: ¹H-NMR (400MHz, CDCl₃) δ = 8.48 (s, 1H), 6.79 (s, 1H), 3.67 (s, 3H), 1.68 (s, 6H). MS calcd. for C₉H₁₂ClN₂O₃ (M+H⁺) 231.1, found 231.0.

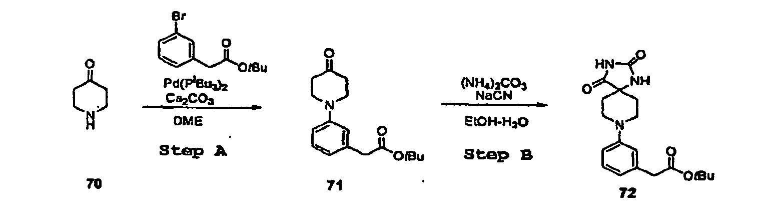

### Intermediate 72. [3-(2,4-Dioxo-1,3,8-triaza-spiro[4.5]dec-8-yl)-phenyl]-acetic acid tert-butyl ester.

**Step A:** To a solution of 4-piperidone monohydrate hydrochloride **70** (2.5 g, 16.42 mmol) in anydrous dioxane (50 mL) and under a nitrogen atmosphere, Cs₂CO₃ (11.75g, 36.13 mmol), (3-Bromo-phenyl)-acetic acid *tert*-butyl ester (4.9 g, 18.1 mmol), and bis (tri t-butyl phosphine) palladium are added. The flask is capped with septa and evacuated three times. The reaction mixture is stirred in oil bath at 85°C for 12 hours, after this time the reaction mix is cooled down, diluted with a saturated solution of NH₄Cl (80 mL) and extracted with EtOAc (2x100 mL). The combined organic layers are washed once with NH₄Cl, brine and dried over Na₂SO₄. The crude is purified by short SiO₂ chromatography (hexane-EtOAc 9:1 to 8:2 as eluant) to afford 1.27g [3-(4-Oxo-piperidin-1-yl)-phenyl]-acetic acid *tert*-butyl ester 71 as a yellow oil. ¹HNMR (400MHz, CDCl₃) δ 7.24 (t, J = 8.0Hz, 1H), 6.92-6.86 (m, 2H), 6.80 (d, J = 4.0 Hz, 1H), 3.61 (t, J = 8.0 Hz, 4H), 3.49 (s, 2H), 2.55 (t, J = 8.0 Hz, 4H). MS (m/z) (M+1)⁺ 290.2.

**Step B:** A well stirred solution of **71** (0.3g, 1.04 mmol) in 6.5 mL of 95% EtOH and 0.5 mL of H₂O, is treated with (NH₄)₂CO₃ (1.84g, 19.2 mmol) and NaCN (0.2g, 4.1 mmol). The reaction mix is heated in a sealed tube at 85°C for 12 hours. After this time the reaction is let to cool down, diluted with H₂O, and extracted with EtOAc (2x 60 mL). The combined organic layers are washed once with brine, dried over Na₂SO₄, and evaporated to yield 0.36g of **72** as a white solid that is used without further purification. ¹HNMR (400MHz, CD₃OD) δ 7.19 (t, J = 8.0Hz, 1H), 6.93-6.90 (m, 2H), 6.75 (d, J = 8.0 Hz, 1H), 3.68-3.63 (m, 2H), 3.48 (s, 2H), 3.12 (m, 2H), 2.17-2.09 (m, 2H), 1.43 (s, 9H). MS (m/z) (M+1)⁺ 360.2.

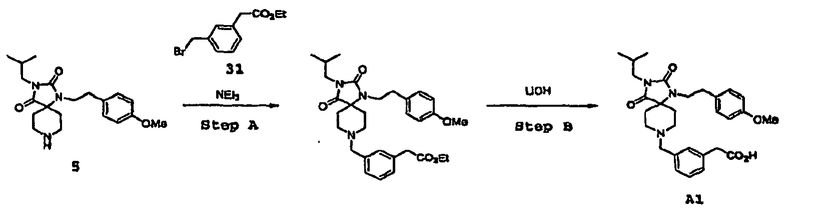

### Example A1. (3-{3-Isobutyl-1-[2-(4-methoxy-phenyl)-ethyl]-2,4-dioxo-1,3,8-triaza-spiro[4.5]dec-8-ylmethyl}-phenyl)-acetic acid.

**Step A:** The 3-isobutyl-1-[2-(4-methoxy-phenyl)-ethyl]-1,3,8-triaza-spiro[4.5]decane-2,4-dione **5** (30 mg, 0.08 mmol) is dissolved in DCM (2.5 mL). Triethylamine (53 uL, 0.24 mmol) and (3-bromomethyl-phenyl)-acetic acid methyl ester 31 (22 mg, 0.09 mmol) are added successively and the mixture is stirred at rt overnight. The solvent is removed in vacuo to afford crude (3-{3-isobutyl-1-[2-(4-methoxy-phenyl)-ethyl]-2,4-dioxo-1,3,8-triaza-spiro[4.5]dec-8-ylmethyl}-phenyl)-acetic acid ethyl ester which is used.without further purification in Step B.
**Step B:** The crude (3-{3-isobutyl-1-[2-(4-methoxy-phenyl)-ethyl]-2,4-dioxo-1,3,8-triaza-spiro[4.5]dec-8-ylmethyl}-phenyl)-acetic acid ethyl ester is dissolved in THF (1 mL), a solution of 1 M LiOH in H₂O (0.6 mL) is added and the mixture is stirred for 12 h at 50°C. The mixture is acidified with 1 M HCl (0.8 mL) and extracted with DCM twice. The organic layer is washed with brine, dried (MgSO₄), filtered, concentrated and purified on reverse phase HPLC (H₂O/MeCN gradient) to afford the title compound as a colorless solid: ¹H-NMR (400MHz, CDCl₃) δ = 7.35 (m, 3H), 7.23 (s, 1H), 7.07 (d, J = 8.6 Hz, 2H), 6.79 (d, J = 8.6 Hz, 2H), 4.11 (s, 2H), 3.75 (s, 3H), 3.62 (s, 2H), 3.44 (m, 4H), 3.35 (t, J = 7.1 Hz, 2H), 3.30 (d, J = 7.4 Hz, 2H), 2.90 (m, 2H), 2.33 (m, 2H), 2.06 (m, 1H), 1.40 (m, 2H), 0.89 (d, J = 6.7 Hz, 6H). MS calculated for C₂₉H₃₈N₃O₅ (M+H⁺) 508.3, found 508.4.

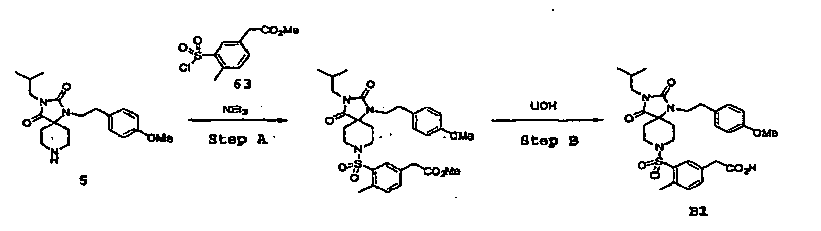

### Example B1. (3-{3-Isobutyl-1-[2-(4-methoxy-phenyl)-ethyl]-2,4-dioxo-1,3,8-triaza-spiro[4.5]decane-8-sulfonyl}-4-methyl-phenyl)-acetic acid.

**Step A:** The 3-isobutyl-1-[2-(4-methoxy-phenyl)-ethyl]-1,3,8-triaza-spiro[4.5]decane-2,4-dione 5 (18 mg, 0.05 mmol) is dissolved in DCM (0.5 mL). Triethylamine (14 uL, 0.10 mmol) and (3-Chlorosulfonyl-4-methyl-phenyl)-acetic acid methyl ester 63 (16 mg, 0.06 mmol) are added successively and the mixture is stirred at rt for 8 h. The solvent is removed in vacuo to afford crude (3-{3-Isobutyl-1-[2-(4-methoxyphenyl)-ethyl]-2,4-dioxo-1,3,8-triaza-spiro[4.5]decane-8-sulfonyl}-4-methyl-phenyl)-acetic acid methyl ester which is used without further purification in Step B.

**Step B:** The crude (3-{3-Isobutyl-1-[2-(4-methoxy-phenyl)-ethyl]-2,4-dioxo-1,3,8-triaza-spiro[4.5]decane-8-sulfonyl}-4-methyl-phenyl)-acetic acid methyl ester is dissolved in THF (1 mL), a solution of 1 M LiOH in H₂O (0.6 mL) is added and the mixture is stirred for 12 h at rt. The mixture is acidified with 1 M HCl (0.8 mL) and extracted with DCM twice. The organic layer is washed with brine, dried (MgSO₄), filtered, concentrated and purified on reverse phase HPLC (H₂O/MeCN gradient) to afford the title compound as a colorless solid: ¹H-NMR (400MHz, CDCl₃) δ = 7.80 (d, J = 1.6 Hz, 1H), 7.37 (dd, J = 1.6 Hz, J = 7.8 Hz, 1H), 7.28 (d, J = 7.8 Hz, 1H), 7.12 (d, J = 8.5 Hz, 2H), 6.84 (d, J = 8.5 Hz, 2H), 3.79 (s, 3H), 3.75 (m, 2H), 3.68 (s, 2H), 3.40 (m, 2H), 3.34 (t, J = 7.0 Hz, 2H), 3.28 (d, J = 7.4 Hz, 2H), 2.93 (t, J = 7.4 Hz, 2H), 2.58 (s, 3H), 2.04 (m, 1H), 1.90 (m, 2H), 1.43 (d, J = 13.7 Hz, 2H), 0.87 (d, J = 6.7 Hz, 6H). MS calculated for C₂₉H₃₈N₃O₇S (M+H⁺) 572.2, found 572.2.

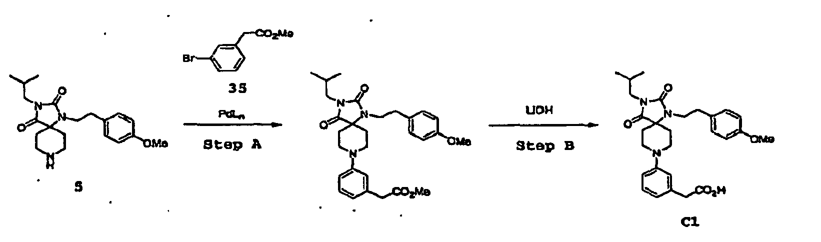

### Example C1. (3-{3-Isobutyl-1-[2-(4-methoxy-phenyl)-ethyl]-2,4-dioxo-1,3,8-triaza-spiro[4.5]dec-8-yl}-phenyl)-acetic acid.

**Step A:** A flame-dried, sealed tube is charged with 3-isobutyl-1-[2-(4-methoxy-phenyl)-ethyl]-1,3,8-triaza-spiro[4.5]decane-2,4-dione **5** (75 mg, 0.21 mmol), (3-bromo-phenyl)-acetic acid methyl ester **35** (72 mg, 0.31 mmol), (tBu)₃PHBF₃ (6 mg, 0.021 mmol) and Cs₂CO₃ (137 mg, 0.42 mmol). 1,4-Dioxane (1.1 mL) is added and the tube is purged with argon. Then Pd₂(dba)₃ (10 mg, 0.011 mmol) is added and the mixture is heated at 120°C overnight. The mixture containing crude (3-{3-isobutyl-1-[2-(4-methoxy-phenyl)-ethyl]-2,4-dioxo-1,3,8-triaza-spiro[4.5]dec-8-yl}-phenyl)-acetic acid methyl ester is used without further purification in Step B.

**Step B:** To the reaction mixture of Step A is added THF (3 mL), a solution of 1 M LiOH in H₂O (1 mL) is added and the mixture is stirred for 12 h at rt. The mixture is acidified with I M HCl (1.2 mL) and extracted with DCM twice. The organic layer is washed with brine, dried (MgSO₄), filtered, concentrated and purified on reverse phase HPLC (H₂O/MeCN gradient) to afford the title compound as a colorless solid: ¹H-NMR (400MHz, CDCl₃) δ = 7.40-7.18 (m, 4H), 7.13 (d, J = 8.5 Hz, 2H), 6.82 (d, J = 8.5 Hz, 2H), 3.93 (t, J = 11.9 Hz, 2H), 3.77 (s, 3H), 3.65 (s, 2H), 3.54 (m, 2H), 3.43 (t, J = 7.1 Hz, 2H), 3.34 (d, J = 7.4 Hz, 2H), 2.96 (t, J = 7.4 Hz, 2H), 2.43 (m, 2H), 2.11 (m, 1H), 1.51 (d, J = 14.0 Hz, 2H), 0.92 (d, J = 6.7 Hz, 6H). MS calculated for C₂₈H₃₆N₃O₅ (M+H⁺) 494.3, found 494.2.

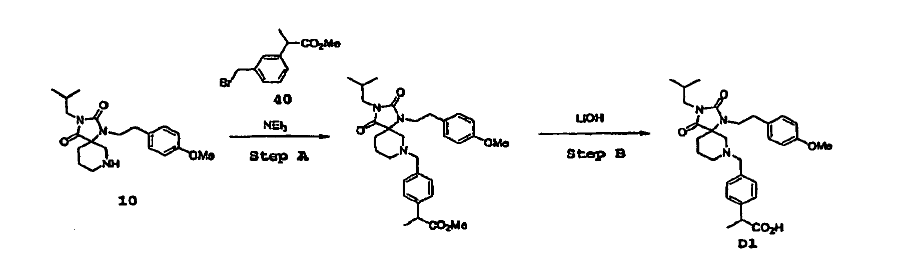

### Example D1. 2-(4-{3-Isobutyl-1-[2-(4-methoxy-phenyl)-ethyl]-2,4-dioxo-1,3-diaza-spiro[4.5]dec-7-ylmethyl}-phenyl)-propionic acid.

**Step A:** The 3-Isobutyl-1-[2-(4-methoxy-phenyl)-ethyl]-1,3,7-triaza-spiro[4.5]decane-2,4-dione **10** (15 mg, 0.04 mmol) is dissolved in DCM (2.5 mL). Triethylamine (17 uL, 0.12 mmol) and 2-(3-bromomethyl-phenyl)-propionic acid methyl ester 40 (12 mg, 0.04 mmol) are added successively and the mixture is stirred at rt overnight. The solvent is removed in vacuo to afford crude 2-(4-{3-Iscbutyl-1-[2-(4-methoxy-phenyl)-ethyl]-2,4-dioxo-1,3-diaza-spiro[4.5]dec-7-ylmethyl}-phenyl)-propionic acid methyl ester which is used without further purification in Step B.

**Step B:** The crude 2-(4-{3-Isobutyl-1-[2-(4-methoxy-phenyl)-ethyl]-2,4-dioxo-1,3-diaza-spiro[4.5]dec-7-ylmethyl}-phenyl)-propionic acid methyl ester is dissolved in THF (1 mL), a solution of 1 M LiOH in H₂O (0.6 mL) is added and the mixture is stirred for 12 h at 50°C. The mixture is acidified with 1 M HCl (0.8 mL) and extracted with DCM twice. The organic layer is washed with brine, dried (MgSO₄), filtered, concentrated and purified on reverse phase HPLC (H₂O/MeCN gradient) to afford the title compound as a colorless solid: ¹H-NMR (400MHn CDCl₃) δ = 7.40 (d, J = 6.8 Hz, 2H), 7.27 (d, J = 6.0 Hz, 2H), 7.13 (d, J = 6.8 Hz, 2H), 6.85 (d, J = 6.0 Hz, 2H), 4.45 (m, 1H), 4.33 (m, 1H), 3.87 (s, 3H), 3.64 (m, 1H), 3.40 (m, 4H), 3.10 (m, 2H), 2.93 (m, 1H), 2.78 (s, 3H), 2.52 (m, 1H), 2.15 (m, 1H), 2.00 (m, 2H), 1.72 (m, 1H), 1.61 (m, 3H), 0.95 (s, 6H). MS calculated for C₃₀H₄₀N₃O₅ (M+H⁺) 522.3, found 522.3.

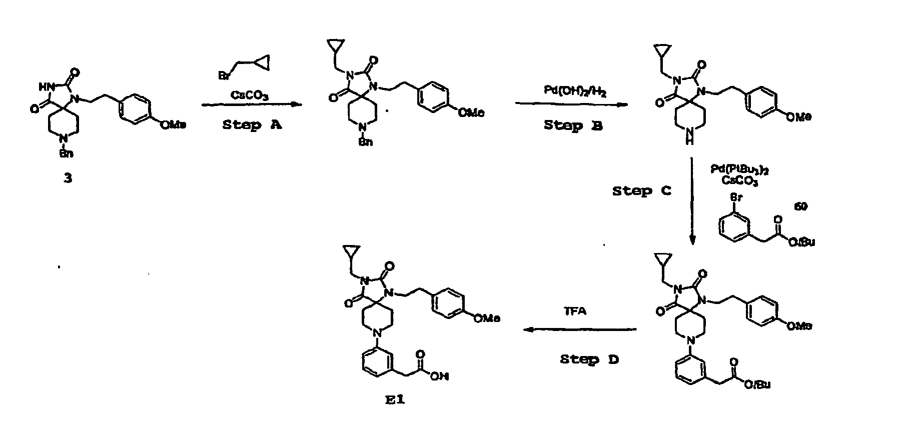

### Example E1. (3-{3-Cyclopropylmethyl-1-[2-(4-methoxy-phenyl)-ethyl]-2,4-dioxo-1,3,8-triaza-spiro[4.5]dec-8-yl}-phenyl)-acetic acid.

**Step A:** 8-Benzyl-1-[2-(4-methoxy-phenyl)-ethyl]-1,3,8-triaza-spiro[4.5]decane-2,4-dione (39 mg, 0.1 mmol) is dissolved in acetonitrile (0.5 mL). Cyclopropylmethyl bromide (0.2 mmol), sodium iodide (30 mg, 0.2 mmol) and cesium carbonate (65 mg, 0.2 mmol) are added at ambient temperature. The mixture is heated in oil at 80 °C for 16 h. The reaction is judged complete by LC/MS. Solid is filtered off and solvent is removed from the mixture to afford the crude 8-Benzyl-3-cyclopropylmethyl-1-[2-(4-methoxy-phenyl)-ethyl]-1,3,8-triaza-spiro[4.5]decane-2,4-dione which is used without further purification in Step B.

**Step B:** The crude 8-Benzyl-3-cyclopropylmethyl-1-[2-(4-methoxyphenyl)-ethyl]-1,3,8-triaza-spiro[4.5]decane-2,4-dione is dissolved in MeOH (1 mL) and stirred with Pd(OH)₂ (∼10 mg) in the presence of 1 atm hydrogen for 16 h at ambient temperature. After filtration and concentration, the crude product 3-Cyclopropylmethyl-1-[2-(4-methoxy-phenyl)-ethyl]-1,3,8-triaza-spiro[4.5]decane-2,4-dione is obtained and used without further purification in Step C.

**Step C:** The crude 3-Cyclopropylmethyl-1-[2-(4-methoxy-phenyl)-ethyl]-1,3,8-triaza-spiro[4.5]decane-2,4-dione is dissolved in 1,4-dioxane (0.3 mL). *tert*-Butyl 3-bromophenylacetate **50** (40 mg, 0.15 mmol) and cesium carbonate (65 mg, 0.2 mmol) are added at ambient temperature. The resultant mixture is purged under a stream of nitrogen and Pd(PtBu₃)₂ (5 mg, 0.01 mmol) is introduced under nitrogen. The reaction mixture is heated in oil at 110 °C for 16 h. The mixture is purified by silica gel flash chromatography (15% EtOAc/hexanes) to yield (3-{3-Cyclopropylmethyl-1-[2-(4-methoxy-phenyl)-ethyl]-2,4-dioxo-1,3,8-triaza-spiro[4.5]dec-8-yl}-phenyl)-acetic acid *tert*-butyl ester as colorless oiL

**Step D**: The (3-{3-Cyclopropylmethyl-1-[2-(4-methoxy-phenyl)-ethyl]-2,4-dioxo-1,3,8-triaza-spiro[4.5]dec-8-yl}-phenyl)-acetic acid *tert*-butyl ester is treated with trifluoroacetic acid at ambient temperature to afford (3-{(3-{3-Cyclopropylmethyl-1-[2-(4-methoxy-phenyl)-ethyl]-2,4-dioxo-1,3,8-triaza-spiro[4.5]dec-8-yl}-phenyl)-acetic acid as a trifluoroacetic acid salt which is purified by preparative LC/MS (20-100 %MeCN/H₂O).¹H NMR (400 MHz, CDCl₃) □ 7.62 (s, 1H), 7.53 (d, *J* = 8.3 Hz, 1H), 7.48 (t, *J* = 8.2 Hz, 1H), 7.39 (d, *J* = 7.6 Hz, 1H), 7.15 (d, *J* = 8.5 Hz, 2H), 6.83 (d, *J* = 8.5 Hz, 2H), 4.16 (t, *J* = 11.7 Hz, 2H), 3.77 (s, 3H), 3.72 (s, 2H), 3.58 (d, *J* = 12 Hz, 2H), 3.47 (t, , *J* = 7.2 Hz, 2H), 3.41 (d, *J* = 7.3 Hz, 2H), 2.99 (t, *J* = 7 Hz, 2H), 2.64 (t, *J* = 13 Hz, 2H), 1.56 (dt, *J* = 14.2, 2 Hz, 2H), 1.2 (m, 1H), 0.55 (m, 2H), 0.37 (m, 2H). LC/MS (M+H⁺): 492.2.

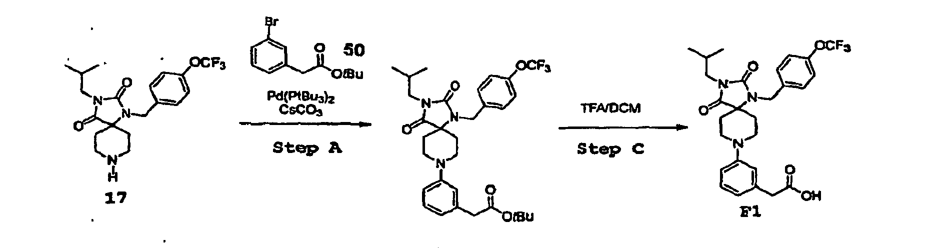

### Example F1. {3-[3-Isobutyl-2,4-dioxo-l-(4-trifluoromethoxy-benzyl)-1,3,8-triaza-spiro[4.5]dec-8-yl]-phenyl}-acetic acid.

**Step A.** To a solution of **17**, (0.103 g, 0.19 mmol) in anhydrous dioxane (1 mL) and under a nitrogen atmosphere, Cs₂CO₃ (0.16 g, 0.49 mmol), (3-Bromo-phenyl)-acetic acid *tert*-butyl ester **50** (0.074 g, 0.27 mmol), and Pd(PtBu₃)₂ (0.03 g, 0.06 mmol) are added. The vial is capped with septa and evacuated three times. The reaction mixture is stirred in an oil bath at 85°C for 12 hours. The reaction mix is cooled down, diluted with a saturated solution of ammonium chloride (5 mL) and extracted with EtOAc (2x10 mL). The organic layer is washed once with NH₄Cl, brine and dried over Na₂SO₄. The crude is purified by preparative LC/MS (20-100 % MeCN/H₂O) to afford {3-[3-Isobutyl-2,4-dioxo-1-(4-trifluoromethoxy-benzyl)-1,3,8-triaza-spiro[4.5]dec-8-yl]-phenyl}-acetic acid *tert* butyl ester. LC/MS (M+H⁺) = 590.3.

**Step B.** 3-[3-Isobutyl-2,4-dioxo-1-(4-trifluoromethoxy-benzyl)-1,3,8-triaza-spiro[4.5]dec-8-yl]-phenyl}-acetic acid tert butyl ester is dissolved in DCM (1 mL) and treated with a 50% solution of TFA /DCM (2 mL). The reaction mixture is stirred at room temperature for 3h. The solvent is removed under vacuum to afford **F1** as a TFA salt in quantitative yield. ¹HNMR (400 MHz, CD₃OD) δ 7.45-7.21 (m, 8H), 4.63 (s, 2H), 4.04-3.98 (bm, 2H), 3.67 (m, 4H).3.40 (d, J = 8.0 Hz, 2H), 2.38 (dt, J = 4.0 and 16.0 Hz, 2H), 2.10 (quint, J = 8.0 Hz, 1H), 1.94-1.90 (m, 2H), 0.95 (d, J = 8.0Hz, 6H). LC/MS (M+H⁺)= 534.3.

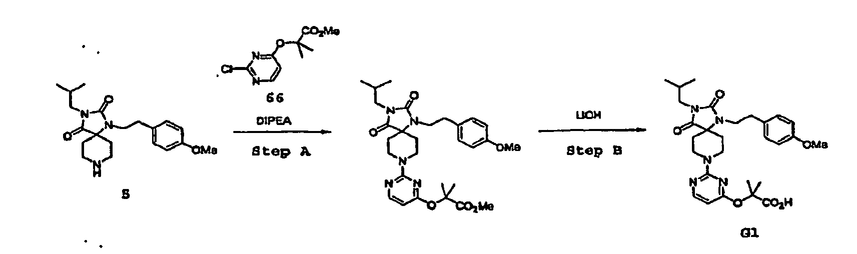

### Example G1. 2-(2-{3-Isobutyl-1-[2-(4-methoxy-phenyl)-ethyl]-2,4-dioxo-1,3,8-triaza-spiro[4.5]dec-8-yl}-pyrimidin-4-yloxy)-2-methyl-propionic acid.

**Step A:** 3-Isobutyl-1-[2-(4-methoxy-phenyl)-ethyl]-1,3,8-triaza-spiro[4.5]decane-2,4-dione **5** (72 mg, 0.20 mmol) is dissolved together with 2-(2-Chloro-pyrimidin-4-yloxy)-2-methyl-propionic acid methyl ester **66** (48 mg, 0.20 mmol) and diisopropylethylamine (52 □L, 0.30 mmol) in n-butanol (0.8 mL). The solution is heated to 50°C for 12 h, then diluted with EtOAc and washed with water twice. The organic layer is seperated and concentrated to give crude 2-(2-{3-isobutyl-1-[2-(4-methoxy-phenyl)-ethyl]-2,4-dioxo-1,3,8-triaza-spiro[4.5]dec-8-yl}-pyrimidin-4-yloxy)-2-methyl-propionic acid methyl ester.

**Step B**: To the crude product of Step A is added THF (3 mL), a solution of 1 M LiOH in H₂O (1 mL) is added and the mixture is stirred for 12 h at rt. The mixture is acidified with 1 M HCl (1.2 mL) and extracted with DCM twice. The organic layer is washed with brine, dried (MgSO₄), filtered, concentrated and purified on reverse phase HPLC (H₂O/MeCN gradient) to afford the title compound as a white solid: ¹H-NMR (400MHz, CDCl₃) δ = 8.12 (d, J = 6.4 Hz, 1H), 7.08 (d, J = 8.5 Hz, 2H), 6.84 (d, J = 8.5 Hz, 2H), 6.21 (d, J = 6.4 Hz, 1H), 4.34 (m, 2H), 3.80 (s, 3H), 3.65 (m, 2H), 3.50-3.00 (m, 4H), 2.95 (m, 2H). 2.07 (m, 1H), 2.00-1.20 (m, 4H), 1.70 (s, 6H), 0.91 (d, J = 6.7 Hz, 6H). MS calculated for C₂₈H₃₈N₅O₆ (M+H⁺) 540.3, found 540.3.

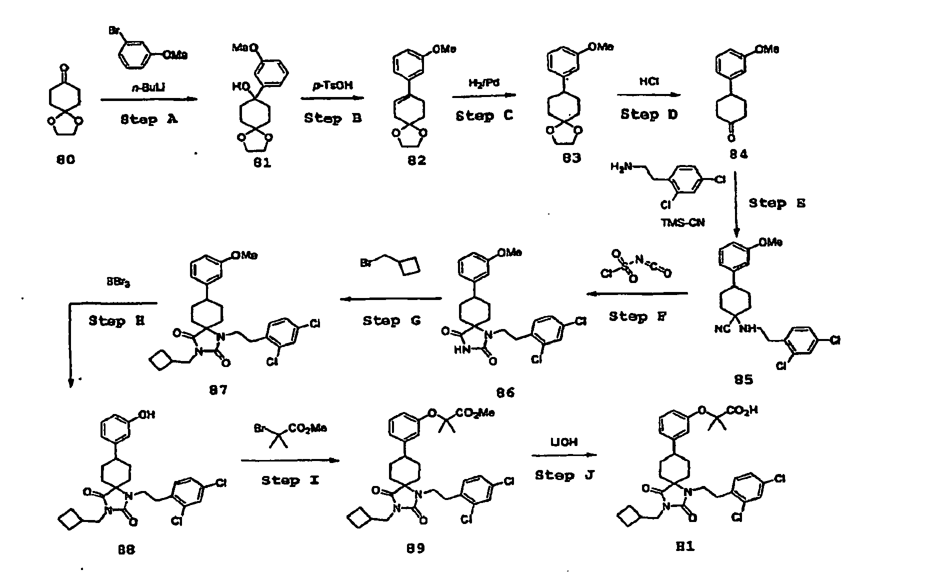

### Example H1. 2-(3-{3-Cyclobutylmethyl-1-[2-(2,4-dichloro-phenyl)-ethyl]-2,4-dioxo-1,3-diaza-spiro[4.5]dec-8-yl}-phenoxy)-2-methyl-propionic acid.

**Step A:** 3-Bromoanisole (2.0 mL, 15.8 mmol) is dissolved in dry THF (20 mL) and cooled to -78°C. *n*-Butyllithium (1.6 M solution in hexane; 10.5 mL, 16.8 mmol) is added dropwise, with stirring, over 5 min. Stirring is continued at -78°C for another 45 min to yield a suspension. In a separate, dry flask 1,4-dioxa-spiro[4.5]decan-8-one **80** (2.67 g, 17.1 mmol) is dissolved in dry THF (15 mL) and cooled to -78°C. The suspension prepared above is added cold via a cannula to the ketone solution; the resulting mixture is stirred at -78°C for 15 min, then at rt for 30 min. Treatment with 5 mL saturated aqueous NH₄Cl solution, followed by concentration, treatment with 1N HCl and extraction with ethyl acetate, then washing with water and brine, drying over MgSO₄, concentration and silica gel chromatography (10-90% EtOAc/Hex) yields 8-(3-methoxy-phenyl)-1,4-dioxa-spiro[4.5]decan-8-ol 81 as a clear, thick oil: ¹H-NMR (400 MHz, CDCl₃) δ = 7.27 (t, *J* = 7.9 Hz, 1H), 7.10 (m, 2H), 6.80 (dd, *J* = 2.4, 8.2 Hz, 1H), 3.99 (m, 4H), 3.82 (s, 3H), 2.13 (m, 4H), 1.81 (d, *J* = 12.1 Hz, 2H), 1.69 (d, *J* = 12.0, 2H).

**Step B**: 8-(3-Methoxy-phenyl)-1,4-dioxa-spiro[4.5]decan-8-ol **81** (1.57 g, 5.9 mmol) is dissolved in benzene (40 mL). *p*-Toluenesulfonic acid monohydrate (0.14 g, 0.74 mmol) is added; the flask is fitted with a Dean-Stark trap and heated to 105°C (bath temperature). After 3 h, the mixture is cooled, diluted with ethyl acetate and washed with sat. aqueous NaHCO₃ and brine, dried over MgSO₄ and concentrated to yield 8-(3-methoxyphenyl)-1,4-dioxa-spiro[4.5]dec-7-ene **82** as an oil (quant): MS calcd. for C₁₅H₁₉O₃ (M+H⁺) 247.1, found 247.1; ¹H-NMR (400 MHz, CDCl₃) δ = 7.22 (t, *J* = 7.9 Hz, 1H), 6.98 (d, *J* = 7.8 Hz, 1H), 6.93 (t, *J* = 2.2 Hz, 1H), 6.78 (dd, *J* = 2.2, 7.9 Hz, 1H), 5.99 (m, 1H), 4.03 (s, 4H), 3.81 (s, 3H), 2.65 (m, 2H), 2.47 (m, 2H), 1.92 (m, 2H).

**Step C**: 8-(3-Methoxy-phenyl)-1,4-dioxa-spiro[4.5]dec-7-ene **82** (from Step B above) is dissolved in ethyl acetate (60 mL). Palladium black (5% on C; 0.22 g, 21 mol%) is added, the mixture is degassed and shaken under 50 psi of hydrogen for 3 h. Filtration and concentration yields 8-(3-methoxy-phenyl)-1,4-dioxa-spiro[4.5]decane **83** as an oil (1.31 g, quant.): MS calcd. for C₁₅H₂₁O₃ (M+H⁺) 249.1, found 249.1; ¹H-NMR (400 MHz, CDCl₃) δ = 7.21 (t, *J* = 7.9 Hz, 1H), 6.83 (d, *J* = 7.9 Hz, 1H), 6.79 (t, *J* = 2.2 Hz, 1H), 6.74 (dd, *J* = 2.2, 7.9 Hz, 1H), 3.98 (s, 4H), 3.80 (s, 3H), 2.53 (m, 1H), 1.85 (m, 4H), 1.69 (m, 4H).

**Step D**: 8-(3-Methoxy-phenyl)-1,4-dioxa-spiro[4.5]decane **83** (1.3 g, 5 mmol) is dissolved in acetone (30 mL) and 4 N aqueous HCl (10.0 mL, 40 mmol). The mixture is heated to reflux for 2.5 h. Cooling and concentration, followed by extraction with ethyl acetate, washing the extracts with sat. aqueous NaHCO₃, water, and brine, drying over Na₂SO₄ and concentration yielded an oil. Silica gel purification yields 4-(3-methoxyphenyl)-cyclohexanone **84** as a clear oil that eventually turned into a white solid: MS calcd. for C₁₃H₁₇O₂ (M+H⁺) 205.1, found 205.1; ¹H-NMR (400 MHz, CDCl₃) δ = 7.25 (t, *J* = 7.9 Hz, 1H), 6.84 (d, *J* = 7.9 Hz, 1H), 6.79 (s, 1H), 6.78 (dd, *J* = 2.2, 7.9 Hz, 1H), 3.81 (s, 3H), 3.01 (tt, *J* = 3.4, 12.1 Hz, 1H), 2.51 (m, 4H), 2.23 (m, 2H), 1.93 (m, 2H).

**Step E:** 4-(3-Methoxy-phenyl)-cyclohexanone **84** (0.55 g, 2.7 mmol) is dissolved in AcOH (10 mL) and cooled to 10°C. 2,4-Dichlorophenethylamine (0.50 mL, 3.3 mmol) is added followed by trimethylsilylcyanide (0.50 mL, 3.7 mmol). The ice-bath is removed and the mixture is stirred at rt for 20 h. The mixture is poured on ice-water, adjusted to pH 9 using aqueous ammonia and extracted with EtOAc twice. The organic extracts are combined, then washed with sat. NaHCO₃, water and brine, dried over MgSO₄ and concentrated to yield an oil. Silica gel chromatography (10-50% EtOAc/Hex) yielded **85** as an oil (0.67 g, 1.66 mmol): MS calcd. for C₂₂H₂₅Cl₂N₂O (M+H⁺) 403.1, found 403.0; ¹H-NMR (400 MHz, CDCl₃) δ = 7.39 (s, 1H), 7.23 (t, *J* = 7.9 Hz, 1H), 7.20 (s, 2H), 6.82 (d, *J* = 7.9 Hz, 1H), 6.77 (s, 1H), 6.76 (dd, *J* = 2.2, 7.9 Hz, 1H), 3.80 (s, 3H), 3.06 (t, *J* = 7.2 Hz, 2H), 2.93 (t, *J* = 7.2 Hz, 2H), 1.96 (m, 2H), 1.83 (m, 2H), 1.57 (m, 2H).

**Step F:** Chlorosulfonylisocyanate (0.2 mL, 2.3 mmol)was dissolved in dry DCM (10 mL) and cooled to 0°C. 1-[2-(2,4-Dichloro-phenyl)-ethylamino]-4-(3-methoxyphenyl)-cyclohexanecarbonitrile **85** (0.67 g, 1.66 mmol) is added dropwise, with stirring, as a solution in DCM (10 mL), the ice-bath is removed and the mixture is stirred at rt for 1 h. The solvent is removed, 1 M HCl (40 mL) is added and the mixture is heated to reflux for 3.5 h. Cooling to rt, followed by vacuum filtration, washing of the white solid with water, and airdrying yielded 1-[2-(2,4-dichloro-phenyl)-ethyl]-8-(3-methoxy-phenyl)-1,3-diaza-spiro[4.5]decane-2,4-dione **86** (0.52 g, 1.16 mmol): MS calcd. for C₂₃H₂₅Cl₂N₂O₃ (M+H⁺) 447.1, found 447.1; ¹H-NMR (400 MHz, drnso-d₆) δ = 10.82 (s, 1H), 7.61 (s, 1H), 7.41 (s, 2H), 7.22 (t, *J* = 7.9 Hz, 1H), 6.81 (d, *J* = 7.8 Hz, 1H), 6.72 (m, 2H), 3.73 (s, 3H), 3.36 (t, *J* = 7.2 Hz, 2H), 2.99 (t, *J* = 7.2 Hz, 2H), 2.15 (m, 2H), 1.85 (m, 2H), 1.68 (m, 4H).

**Step G:** 1-[2-(2,4-Dichloro-phenyl)-ethyl]-8-(3-metuoxy-phenyl)-1,3-diaza-spiro[4.5]decane-2,4-dione **86** (0.52 g, 1.16 mmol), bromomethylcyclobutane (0.175 mL, 1.56 mmol) and potassium carbonate (0.32 g, 2.32 mmol) in dry DMSO (5.0 mL) are stirred for 3 h at 50°C. The mixture is cooled to rt, diluted with water and extracted with DCM (3x). The combined extracts are washed with 1 N HCl, H₂O (3x) and brine, dried over MgSO₄, and concentrated to afford 3-cyclobutylmethyl-1-[2-(2,4-dichloro-phenyl)-ethyl]-8-(3-methoxy-pbenyl)-1,3-diaza-spiro[4.5]decane-2,4-dione **87** (0.65 g, quant.) as a clear, thick oil: MS calcd. for C₂₈H₃₃Cl₂N₂O₃ (M+H⁺) 515.1, found 515.1; ¹H-NMR (400 MHz, CDCl₃) δ = 7.40 (d, *J* = 1.7 Hz, 1H), 7.21 (m, 3H), 6.88 (d, *J* = 7.9 Hz, 1H), 6.83 (t, *J* = 2.2 Hz, 1H), 6.75 (dd, *J* = 2.2, 7.9 Hz, 1H), 3.80 (s, 3H), 3.54 (d, *J* = 7.4 Hz, 2H), 3.41 (t, *J* = 7.2 Hz, 2H), 3.13 (t, *J* = 7.2 Hz, 2H), 2.71 (septet, *J* = 7.7 Hz, 1H), 2.38 (m, 3H), 2.02 (m, 2H), 1.88 (m, 2H), 1.77 (m, 6H), 1.62 (m, 2H).

**Step H:** 3-Cyclobutylmethyl-1-[2-(2,4-dichloro-phenyl)-ethyl]-8-(3-methoxy-phenyl)-1,3-diaza-spiro[4.5]decane-2,4-dione **87** (0.65 g, 1.2 mmol) is dissolved in dry dichloromethane. Neat boron tribromide (0.50 mL, 5.2 mmol) is added and the mixture is stirred at rt for 1.5 h. The reaction mixture is poured over ice and extracted with DCM (3x). The combined extracts are washed with aqueous sat. NaHCO₃, dried over MgSO₄, and concentrated to yield a glass. Treatment with acetonitrile and concentration yielded 3-cyclobutylmethyl-1-[2-(2,4-dichloro-phenyl)-ethyl]-8-(3-hydroxy-phenyl)-1,3-diaza-spiro[4.5]decane-2,4-dione **88** as a white solid (0.56 g, quant.): MS calcd. for C₂₇H₃₁Cl₂N₂O₃ (M+H⁺) 501.1, found 501.1; ¹H-NMR (400 MHz, CDCl₃) δ = 7.40 (d, J = 1.7 Hz, 1H), 7.20 (m, 2H), 7.17 (t, *J* = 7.9 Hz, 1H), 6.85 (d, *J* = 7.9 Hz, 1H), 6.77 (t, *J* = 2.2 Hz, 1H), 6.68 (dd, *J* = 2.2, 7.9 Hz, 1H), 3.55 (d, *J* = 7.4 Hz, 2H), 3.41 (t, *J* = 7.2 Hz, 2H), 3.12 (t, *J* = 7.2 Hz, 2H), 2.71 (septet, *J* = 7.7 H₂, 1H), 2.38 (m, 3H), 2.02 (m, 2H), 1.87 (m, 2H), 1.77 (m, 6H), 1.60 (m, 2H).

**Step I:** 3-Cyclobutylmèthyl-1-[2-(2,4-dichloro-phenyl)-ethyl]-8-(3-hydroxy-phenyl)-1,3-diaza-spiro[4.5]decane-2,4-dione **88** (0.28 g, 0.56 mmol) is dissolved in DCM (3 mL) and ACN (6 mL). 2-Bromo-2-methyl-propionic acid methyl ester (0.09 mL, 0.7 mmol) and Cs₂CO₃ (0.38 g, 1.17 mmol) are added and the suspension is vigorously stirred at 60°C for 4 h. Cooling, addition of a small amt. of silica gel, and filtration, followed by concentration yielded the ester **89** as a thick oil: MS calcd. for C₃₂H₃₉O₂N₂O₅ (M+H⁺) 601.1, found 601.0; ¹H-NMR (400 MHz, CDCl₃) δ = 7.40 (d, *J* = 1.7 Hz, 1H), 7.21 (m, 2H), 7.15 (t, *J* = 7.9 Hz, 1H), 6.91 (d, *J* = 7.9 Hz, 1H), 6.80 (t, *J* = 2.2 Hz, 1H), 6.62 (dd, *J* = 2.2, 7.9 Hz, 1H), 3.79 (s, 3H), 3.54 (d, *J* = 7.4 Hz, 2H), 3.41 (t, *J* = 7.2 Hz, 2H), 3.12 (t, *J* = 7.2 Hz, 2H), 2.70 (septet, *J* = 7.7 Hz, 1H), 2.36 (m, 3H), 2.02 (m, 2H), 1.86 (m, 2H), 1.76 (m, 6H), 1.69 (m, 2H), 1.69 (s, 6H).

**Step J:** 2-(3-{3-Cyclobutylmethyl-1-[2-(2,4-dichloro-phenyl)-ethyl]-2,4-dioxo-1,3-diaza-spiro[4.5]dec-8-yl}-phenoxy)-2-methyl-propionic acid methyl ester **89** (from Step I above) is dissolved in DME (2 mL). Solid lithium hydroxide monohydrate (0.10 g, excess) is added, followed by water (0.50 mL). The mixture is stirred at 60°C overnight. Cooling, adjusting the pH to 2 using 1 N HCl, and extraction with DCM (3x), followed by drying over MgSO₄ and concentration yielded a resin. Treatment with diethyl ether and hexane followed by concentration under high vacuum yielded 2-(3-{3-cyclobutylmethyl-1-[2-(2,4-dichloro-phenyl)-ethyl]-2,4-dioxo-1,3-diaza-spiro[4.5]dec-8-yl}-phenoxy)-2-methyl-propionic acid **Example H1** a solid: MS calcd. for C₃₁H₃₇Cl₂N₂O₅ (M+H⁺) 587.1, found 587.1; ¹H-NMR (400 MHz, CDCl₃) δ = 7.41 (d, *J* = 1.7 Hz, 1H), 7.21 (m, 2H), 7.18 (t, *J* = 7.9 Hz, 1H), 6.90 (d, *J* = 7.9 Hz, 1H), 6.82 (t, *J* = 2.2 Hz, 1H), 6.65 (dd, *J* = 2.2, 7.9 Hz, 1H), 3.54 (d, *J* = 7.4 Hz, 2H), 3.41 (t, *J* = 7.2 Hz, 2H), 3.13 (t, *J* = 7.2 Hz, 2H), 2.70 (septet, *J* = 7.7 Hz, 1H), 2.36 (m, 3H), 2.02 (m, 2H), 1.88 (m, 2H), 1.77 (m, 6H), 1.70 (m, 2H), 1.69 (s, 6H).

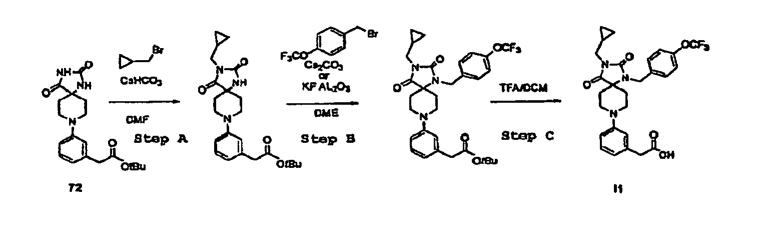

### Example I1: {3-[3-Cyclopropylmethyl-2,4-dioxo-1-(4-trifluoromethoxy-benzyl)-1,3,8-triaza-spiro[4.5]dec-8-yl]-phenyl}-acetic acid.

**Step A:** To a well stirred solution of intermediate **72** (0.17 g, 0.47 mmol) in anhydrous DMF (5 mL)were added CsHCO₃ (0.14 g, 0.7 mmol) and bromomethyl-cyclopropane (0.095 g, 0.7 mmol). The reaction mixture is evacuated three times and irradiated in a microwave oven at 130 °C for 20 minutes. The reaction mix is cooled down, diluted with water and extracted with EtOAc twice. The organic layers are combined, washed with 5% Na₂CO₃ solution, brine and concentrated to afford [3-(3-cyclopropylmethyl-2,4-dioxo-1,3,8-triaza-spiro[4.5]dec-8-yl)-phenyl]-acetic acid tert-butyl ester as a white solid which is used without further purification. MS (m/z) (M+1)⁺ 414.3.

**Step B**: To a well stirred solution of crude [3-(3-cyclopropylmethyl-2,4-dioxo-1,3,8-triaza-spiro[4.5]dec-8-yl)-phenyl]-acetic acid tert-butyl ester (20mg, 0.048 mmol) in anhydrous MeCN (1 mL) are added Cs₂CO₃ (19 mg 0.058 mmol) and 1-bromomethyl-4-trifluoromethoxy-benzene (12.5 uL, 0.77 mmol). The reaction mixture is evacuated three times and irradiated in a microwave oven at 130 °C for 30 minutes. The reaction mix directly purified by preparative LC/MS using a MeCN/H₂O gradient 90-10%. The solvent is removed under vacuum to afford {3-[3-cyclopropylmethyl-2,4-dioxo-1-(4-trifluoromethoxy-benzyl)-1,3,8-triaza-spiro[4.5]dec-8-yl]-phenyl}-acetic acid tert-butyl ester. MS (m/z) (M+1)⁺ 602.3.
NB: KF-Al₂O₃ can be used instead of Cs₂CO₃.

**Step C:** A solution of {3-[3-cyclopropylmethyl-2,4-dioxo-1-(4-trifluoromethoxy-benzyl)-1,3,8-triaza-spiro[4.5]dec-8-yl]-phenyl}-acetic acid tert-butyl ester in DCM (1mL) is treated with a 50% solution of TFA in DCM (2 mL). The reaction mixture is stirred at room temperature for 1 hour. The solvent is removed under vacuum and the crude is purified by preparative LC/MS using a MeCN/H₂O gradient 90-10%. Removal of the solvent affords the title compound **I1** as TFA salt. ¹HNMR (400MHz, CD₃OD) δ 7.41 (d, J = 8.0Hz, 2H), 7.29 (t, J = 8.0Hz, 1H), 7.23 (d, J = 8.0Hz, 2H), 7.07-7.04 (m, 2H), 6.95-6.94 (m, 2H), 4.61(s, 2H), 3.73-3.63 (m, 4H), 3.54 (s, 2H), 3.41 (d, J = 4.0Hz, 2H), 2.24-2.17 (m, 2H), 1.82-1.79 (m, 2H), 1.43 (s, 9H), 1.22-1.18 (m, 2H), 0.55-0.51 (m, 1H), 0.38-0.34 (m, 2H). MS (m/z) (M+1)⁺ 532.0.

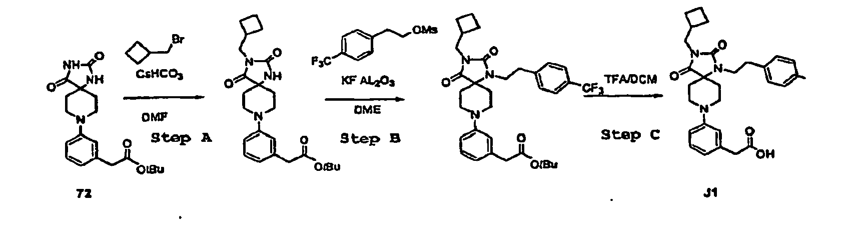

### Example J1: (3-{3-Cyclobutylmethyl-2,4-dioxo-1-[2-(4-trifluoromethyl-phenyl)-ethyl]-1,3,8-triaza-spiro[4.5]dec-8-yl}-phenyl)-acetic acid.

**Step A:** To a well stirred solution of **72** (0.4 g, 1.1 mmol) in anhydrous DMF (5 mL)were added CsHCO₃ (0.32 g 1.6 mmol) and bromomethyl-cyclobutane ( 0.23 g, 1.6 mmol). The reaction mixture is evacuated three times and irradiated in a MW oven at 130 °C for 20 minutes. The reaction mix is cooled down, diluted with water and extracted with EtOAc twice. The organic layers are combined, washed with 5% Na₂CO₃ solution, brine and concentrated to afford [3-(3-cyclobutylmethyl-2,4-dioxo-1,3,8-triaza-spiro[4.5]dec-8-yl)-phenyl]-acetic acid *tert*-butyl ester as a white solid which is used without further purification. ¹HNMR (400MHz, CDCl₃) δ 7.17-7.13 (m, 1H), 6.79-6.73 (m, 3H), 5.89 (bs, 1H), 3.65-3.61 (m, 2H), 3.47 (d, J = 4.0Hz, 2H), 3.42 (s, 2H). 2.94-2.88 (m, 2H), 2.63 (quint. J = 8.0Hz, 1H), 2.19-2.12 (m, 2H), 1.97-1.90 (m, 2H), 1.81-1.65 (m, 6H), 1.37 (s, 9H). MS (m/z) (M+1)⁺ 428.3.

**Step B:** To a solution of [3-(3-cyclobutylmethyl-2,4-dioxo-1,3,8-triaza-spiro[4.5]dec-8-yl)-phenyl]-acetic acid *tert*-butyl ester (22mg, 0.05 mmol) in anhydrous DME (2mL) are added methanesulfonic acid 2-(4-trifluoromethyl-phenyl)-ethyl ester (30 mg 0.1mmol) and KF-Al₂O₃ (0.2g). The reaction mixture is stirred in an oil bath at 80 °C for 8 hours. After this time, the reaction mix is filtered and directly purified by preparative LC/MS using a MeCN/H₂O gradient 90-10% to afford (3-{3-cyclobutylmethyl-2,4-dioxo-1-[2-(4-trifluoromethyl-phenyl)-ethyl]-1,3,8-triaza-spiro[4.5]dec-8-yl}-phenyl)-acetic acid *tert*-butyl ester. MS (m/z) (M+1)⁺ 600.2.

**Step C:** (3-{3-cyclobutylmethyl-2,4-dioxo-1-[2-(4-trifluoromethylphenyl)-ethyl]-1,3,8-triaza-spiro[4.5]dec-8-yl}-phenyl)-acetic acid *tert*-butyl ester is converted in the title compound (3-{3-cyclobutylmethyl-2,4-dioxo-1-[2-(4-trifluoromethylphenyl)-ethyl]-1,3,8-triaza-spiro[4.5]dec-8-yl}-phenyl)-acetic acid as a TFA salt following the same procedure as described in Step C for the preparation of Example **I1**. ¹HNMR (400MHz, CD₃OD) δ 7.59 (d, J = 8.0Hz, 2H), 7.43 (d, J = 8.0H, 1H), 7.22 (t, J = 8.0Hz, 1H), 6.96-6.81 (m, 3H), 3.63-3.57 (m, 2H), 3.52-3.45 (m, 6H), 3.08-3.04 (m, 2H), 2.71-2.63 (m, 1H), 2.05-2.00 (m, 4H), 1.89-1.87 (m, 2H), 1.80-1.77 (m, 2H), 0.91-0.98 (m, 2H). MS (m/z) (M+1)⁺ 544.3.

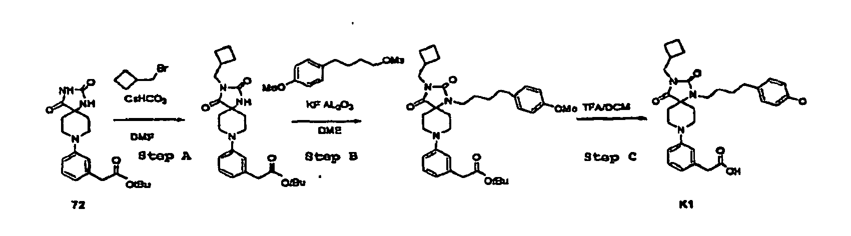

### Example K1: (3-{3-Cyclobutylmethyl-1-[4-(4-methoxy-phenyl)-butyl]-2,4-dioxo-1,3,8-triaza-spiro[4.5]dec-8-yl}-phenyl)-acetic acid.

**Step A:** To a well stirred solution of **72** (0.4 g, 1.1 mmol) in anhydrous DMF (5 mL)were added CsHCO₃ (0.32 g 1.6 mmol) and bromomethyl-cyclobutane ( 0.23 g, 1.6 mmol). The reaction mixture is evacuated three times and irradiated in a MW oven at 130 °C for 20 minutes. The reaction mix is cooled down, diluted with water and extracted with EtOAc twice. The organic layers are combined, washed with 5% Na₂CO₃ solution, brine and concentrated to afford [3-(3-cyclobutylmethyl-2,4-dioxo-1,3,8-triaza-spiro[4.5]dec-8-yl)-phenyl]-acetic acid *tert*-butyl ester as a white solid which is used without further purification. ¹HNMR (400MHz, CDCl₃) δ 7.17-7.13 (m, 1H), 6.79-6.73 (m, 3H), 5.89 (bs, 1H), 3.65-3.61 (m, 2H), 3.47 (d, J = 4.0Hz, 2H), 3.42 (s, 2H), 2.94-2.88 (m, 2H), 2.63 (quint. J = 8.0Hz, 1H), 2.19-2.12 (m, 2H), 1.97-1.90 (m, 2H), 1.81-1.65 (m, 6H), 1.37 (s, 9H). MS (m/z) (M+1)⁺ 428.3.

**Step B:** (3-{3-Cyclobutylmethyl-1-[4-(4-methoxy-phenyl)-butyl]-2,4-dioxo-1,3,8-triaza-spim[4.5]dec-8-yl}-phenyl)-acetic acid *tert*-butyl ester is prepared from [3-(3-cyclobutylmethyl-2,4-dioxo-1,3,8-triaza-spiro[4.5]dec-8-yl)-phenyl]-acetic acid *tert-*butyl ester using the same procedure described in Step B for the preparation of **J1**. The reaction mixture is purified by preparative LC/MS using a MeCN/H₂O gradient 90-10%. The solvent is removed under vacuum to afford the title compound. MS (m/z) (M+1)⁺ 590.2.

**Step C:** (3-{3-Cyclobutylmethyl-1-[4-(4-methoxy-phenyl)-butyl]-2,4-dioxo-1,3,8-triaza-spiro[4.5]dec-8-yl}-phenyl)-acetic acid *tert*-butyl ester is converted in the title compound(3-{3-cydobutylmethyl-1-[4-(4-methoxy-phenyl)-butyl]-2,4-dioxo-1,3,8-triaza-spiro[4.5]dec-8-yl}-phenyl)-acetic acid as a TFA salt following the same procedure as described in Step C for the preparation of Example **I1**. ¹HNMR (400MHz, CDCl₃) δ 7.59 (s, 1H), 7.53-7.50 (m, 1H), 7.45 (t, J = 8.0Hz, 1H), 7..36 (d, J = 8.0Hz, 1H), 7.09 (d, J = 8.0Hz. 2H), 6.82 (d, J = 8.0Hz, 2H), 4.20 (t, J = 12.0Hz, 2H), 3.78 (s, 3H), 3.68-3.66 (m, 3H), 3.55 (d, J = 8.0Hz, 2H), 3.29 (t, J = 8.0Hz, 2H), 2.83 (dt, J = 4.0 and 12.0Hz, 2H), 2.67 (quint. J = 12.0Hz, 1H), 2.58 (t, J = 8.0Hz, 2H), 2.05-1.98 (m, 2H), 1.89-1.73 (m, 6H), 1.66-1.60 (m, 4H). MS (m/z) (M+1)⁺ 534.2.

By repeating the procedures described in the above examples, using appropriate starting materials, the following compounds of Formula I, as identified in Table 1, are obtained.

**Table 1**

| **Compound** **Number** | **Compound** **Structure** | **Physical Data** **¹H NMR 400 MHz (DMSO-*d₆*) and/or MS (m/z)** |
|---|---|---|
| **A2** | 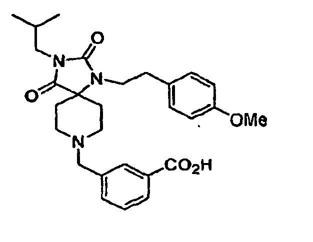 | ¹H-NMR (400 MHz, CDCl₃) δ = 8.18 (s, 1H), 8.04 (d, J = 7.7 Hz, 1H), 7.67 (d, J = 7.7 Hz, 1H), 7.52 (t, J = 7.7 Hz, 1H), 7.08 (d, J = 8.6 Hz, 2H), 6.78 (d, J = 8.6 Hz, 2H), 4.28 (s, 2H), 3.73 (s, 3H), 3.56 (m, 4H), 3.38 (t, J = 7.1 Hz, 2H), 330 (d, J = 7.4 Hz, 2H), 2.92 (t, J = 7.1 Hz, 2H), 2.39 (m, 2H), 2.07 (m, 1H), 1.45 (m, 2H), 0.90 (d, J = 6.7 Hz, 6H). MS calcd. for C₂₈H₃₆N₃O₅ (M+H⁺) 494.3, found 494.3. |
| A3 | 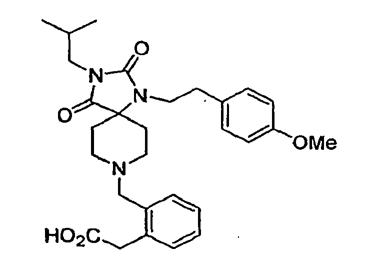 | ¹H-NMR (400 MHz, CDCl₃) δ = 7.34-7.26 (m, 4H), 7.17 (d, J = 8.6 Hz, 2H), 6.84 (d, J = 8.6 Hz, 2H), 3.86 (t, J = 10.8 Hz, 2H), 3.76 (s, 3H), 3.48 (t, J = 7.1 Hz, 2H), 3.35 (d, J = 7.4 Hz, 2H), 3.18 (d, J = 11.5 Hz, 2H), 2.98 (m, 4H), 2.82 (t, J = 6.6 Hz, 2H), 2.32 (m, 2H), 2.11 (m, 1H), 1.55 (d, J = 14.0 Hz, 2H), 0.93 (d, J = 6.7 Hz, 6H). MS calcd. for C₂₉H₃₈N₃O₅ (M+H⁺) 508.3, found 508.2. |
| B2 | 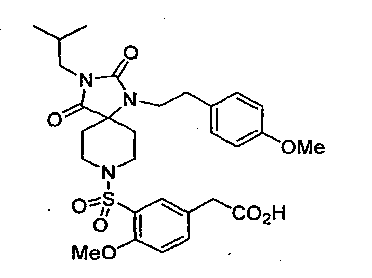 | ¹H-NMR (400 MHz, CDCl₃) δ = 7.78 (d, J = 2.3 Hz, 1H), 7.44 (dd, J = 2.3 Hz, J = 8.5 Hz, 1H), 7.11 (d, J = 8.6 Hz, 2H), 6.98 (d, J = 7.8 Hz, 1H), 6.83 (d, J = 8.6 Hz, 2H), 3.92 (s, 3H), 3.79 (s, 3H), 3.77 (m, 2H), 3.62 (s, 2H), 3.36 (m, 4H), 3.27 (d, J = 7.4 Hz, 2H), 2.93 (t, J = 7.4 Hz, 2H), 2.03 (m, 1H), 1.86 (m, 2H), 1.44 (d, J = 13.7 Hz, 2H), 0.87 (d, J = 6.7 Hz, 6H). MS calcd. for C₂₉H₃₈N₃O₈S (M+H⁺) 588.2, found 588.2. |
| C2 | 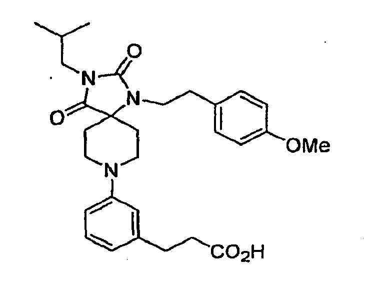 | MS calcd. for C₂₉H₃₈N₃O₅ (M+H⁺) 508.3, found 508.2. |
| C3 | 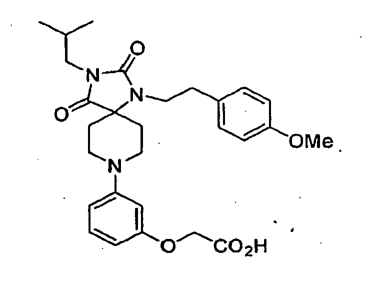 | ¹H-NMR (400 MHz, CDCl₃) δ = 7.27 (m, 1H), 7.11 (d, J = 8.5 Hz, 2H), 6.93 (s, 1H), 6.85 (m, 1H), 6.82 (d, J = 8.5 Hz, 2H), 6.70 (m, 1H), 4.65 (s, 2H), 3.78 (m, 2H), 3.77 (s, 3H), 3.53 (m, 2H), 3.39 (t, J = 7.1 Hz, 2H), 3.34 (d, J = 7.4 Hz, 2H), 2.95 (t, J = 7.4 Hz, 2H), 2.22 (m, 2H), 2.09 (m, 1H), 1.51 (d, J = 13.8 Hz, 2H), 0.92 (d, J = 6.7 Hz, 6H). MS calcd. for C₂₈H₃₆N₃O₆ (M+H⁺) 510.3, found 510.2. |
| C4 | 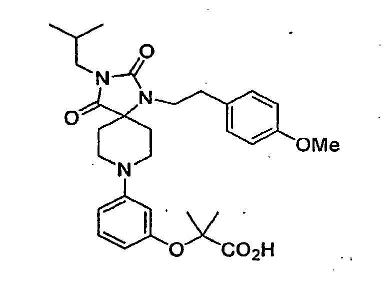 | ¹H-NMR (400 MHz, CDCl₃) δ = 7.31 (m, 2H), 7.13 (d, J = 8.5 Hz, 2H), 7.01 (s, 1H), 6.82 (m, 1H), 6.82 (d, J = 8.5 Hz, 2H), 3.90 (m, 2H), 3.77 (s, 3H), 3.50 (m, 2H), 3.40 (t, J = 7.1 Hz, 2H), 3.34 (d, J = 7.4 Hz, 2H), 2.96 (t, J = 7.4 Hz, 2H), 2.31 (m, 2H), 2.09 (m, 1H), 1.51 (d, J = 13.8 Hz, 2H), 1.60 (s, 6H), 0.92 (d, J = 6.7 Hz, 6H). MS calcd. for C₃₀H₄₀N₃O₆ (M+H⁺) 538.3, found 538.3. |
| C5 | 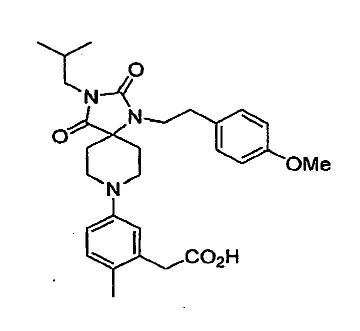 | ¹H-NMR (400 MHz, CDCl₃) δ = 7.45 (s, 1H), 7.34 (d, J = 8.2 Hz, 1H), 7.26 (m, 1H), 7.13 (d, J = 8.5 Hz, 2H), 6.82 (d, J = 8.5 Hz, 2H), 4.05 (t, J = 12.7 Hz, 2H), 3.76 (s, 3H), 3.64 (s, 2H), 3.53 (m, 2H), 3.44 (t, J = 5.0 Hz, 2H), 3.34 (m, 2H), 2.96 (t, J = 5.0 Hz, 2H), 2.58 (t, 12.7 Hz, 2H), 2.30 (s, 3H), 2.10 (m, 1H), 1.50 (d, J = 14.2 Hz, 2H), 0.93 (d, J = 6.7 Hz, 6H). MS calcd. for C₂₉H₃₈N₃O₅ (M+H⁺) 508.3, found 508.3. |
| C6 | 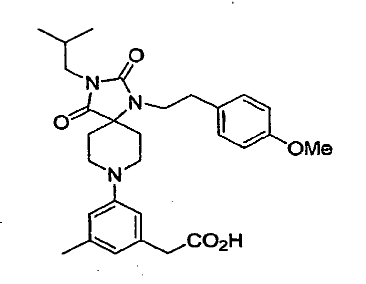 | ¹H-NMR (400 MHz, CDCl₃) δ = 7.20 (s, 1H), 7.14 (s, 1H), 7.12 (d, J = 8.6 Hz, 2H), 7.04 (s, 1H), 6.82 (d, J = 8.6 Hz, 2H), 3.96 (m, 2H), 3.76 (s, 3H), 3.59 (s, 2H), 3.53 (m, 2H), 3.43 (t, J = 7.2 Hz, 2H), 3.35 (d, J = 7.5 Hz, 2H), 2.96 (t, J = 7.2 Hz, 2H), 2.47 (m, 2H), 2.35 (s, 3H), 2.10 (m, 1H), 1.50 (d, J = 14.0 Hz, 2H), 0.93 (d, J = 6.7 Hz, 6H). MS calcd. for C₂₉H₃₈N₃O₅ (M+H⁺) 508.3, found 508.3. |
| C7 | 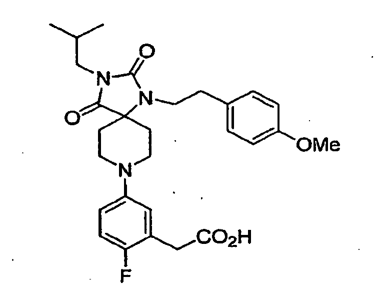 | ¹H-NMR (400 MHz, CDCl₃) δ = 7.39 (m, 1H), 7.31 (m, 1H), 7.13 (d, J = 8.5 Hz, 2H), 7.12 (m, 1H), 6.83 (d, J = 8.5 Hz, 2H), 3.88 (m, 2H), 3.77 (s, 3H), 3.71 (s, 2H), 3.48 (m, 2H), 3.43 (t, J = 7.2 Hz, 2H), 3.34 (d, J = 7.5 Hz, 2H), 2.97 (t, J = 7.2 Hz, 2H), 2.38 (m, 2H), 2.10 (m, 1H), 1.50 (d, J = 14.1 Hz, 2H), 0.93 (d, J = 6.7 Hz, 6H). MS calcd. for C₂₈H₃₅FN₃O₅ (M+H⁺) 512.3, found 512.3. |
| C8 | 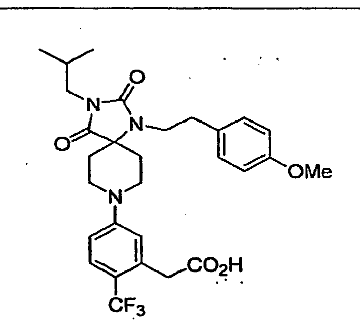 | ¹H-NMR (400 MHz, CDCl₃) δ = 7.58 (d, J = 8.7 Hz, 1H), 7.20-7.10 (m, 2H), 7.10 (d, J = 8.3 Hz, 2H), 7.12 (m, 1H), 6.81 (d, J = 8.3 Hz, 2H), 3.83 (s, 2H), 3.80-3.60 (m, 4H), 3.78 (s, 3H), 3.34 (m, 4H), 2.94 (t, J = 7.2 Hz, 2H), 2.10 (m, 1H), 2.02 (m, 2H), 1.51 (d, J = 13.5 Hz, 2H), 0.93 (d, J = 6.7 Hz, 6H). MS calcd. for C₂₉H₃₅F₃N₃O₅ (M+H⁺) 562.3, found 562.3. |
| C9 | 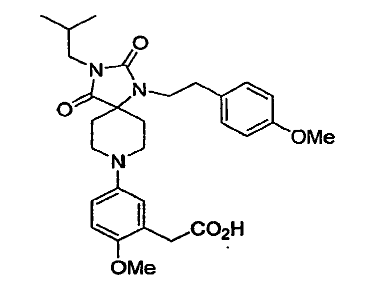 | ¹H-NMR (400 MHz, CDCl₃) δ = 7.59 (d, J = 8.9 Hz, 1H), 7.52 (s, 1H), 7.16 (d, J = 8.4 Hz, 2H), 6.92 (d, J = 8.9 Hz, 1H), 6.82 (d, J = 8.4 Hz, 2H), 4.11 (m, 2H), 3.84 (s, 3H), 3.76 (s, 3H), 3.65 (s, 2H), 3.51 (m, 4H), 3.35 (d, J = 7.4 Hz, 2H), 2.99 (t, J = 7.0 Hz, 2H), 2.76 (m, 2H), 2.10 (m, 1H), 1.48 (d, J = 14.3 Hz, 2H), 0.93 (d, J = 6.7 Hz, 6H). MS calcd. for C₂₉H₃₈N₃O₆ (M+H⁺) 524.3, found 524.3. |
| C10 | 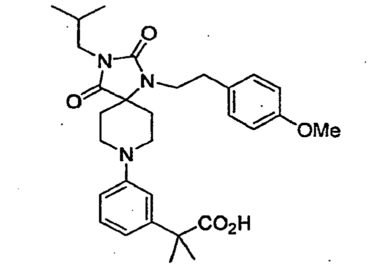 | ¹H-NMR (400 MHz, CDCl₃) δ = 7.34 (m, 2H), 7.18 (d, J = 7.8 Hz, 1H), 7.12 (d, J = 8.4 Hz, 2H), 6.82 (d, J = 8.4 Hz, 2H), 3.77 (m, 2H), 3.77 (s, 3H), 3.52 (m, 2H), 3.41 (t, J = 7.3 Hz, 2H), 3.34 (d, J = 7.4 Hz, 2H), 2.95 (t, J = 7.3 Hz, 2H), 2.29 (m, 2H), 2.10 (m, 1H), 1.58 (s, 6H), 1.52 (d, J = 13.7 Hz, 2H), 0.92 (d, J = 6.7 Hz, 6H). MS calcd. for C₃₀H₄₀N₃O₅ (M+H⁺) 522.3, found 522.3. |
| C11 | 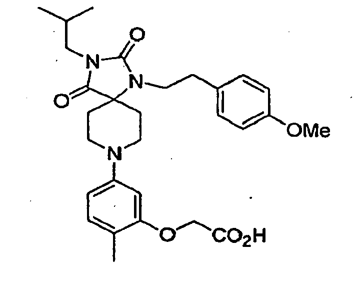 | ¹H-NMR (400 MHz, CDCl₃) δ = 7.20 (m, 2H), 7.12 (d, J = 8.3 Hz, 2H), 7.18 (d, J = 8.1 Hz, 1H), 6.82 (d, J = 8.3 Hz, 2H), 4.71 (s, 2H), 4.01 (m, 2H), 3.76 (s, 3H), 3.49 (m, 2H), 3.42 (t, J = 7.1 Hz, 2H), 3.34 (d, J = 7.4 Hz, 2H), 2.96 (t, J = 7.1 Hz, 2H), 2.49 (m, 2H), 2.25 (s, 3H), 2.10 (m, 1H), 1.49 (d, J = 14.2 Hz, 2H), 0.93 (d, J = 6.7 Hz, 6H). MS calcd. for C₂₉H₃₈N₃O₆ (M+H⁺) 524.3, found 524.3. |
| C12 | 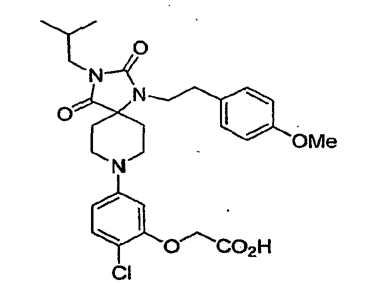 | ¹H-NMR (400 MHz, CDCl₃) δ = 7.43-6.82 (m, 7H), 4.78 (s, 2H), 3.99 (m, 2H), 3.76 (s, 3H), 3.52 (m, 2H), 3.40 (t, J = 7.2 Hz, 2H), 3.34 (d, J = 7.4 Hz, 2H), 2.96 (t, J = 7.2 Hz, 2H), 2.38 (m, 2H), 2.10 (m, 1H), 1.49 (d, J = 14.3 Hz, 2H), 0.92 (d, J = 6.7 Hz, 6H). MS calcd. for C₂₈H₃₅ClN₃O₆ (M+H⁺) 544.2, found 544.2. |
| C13 | 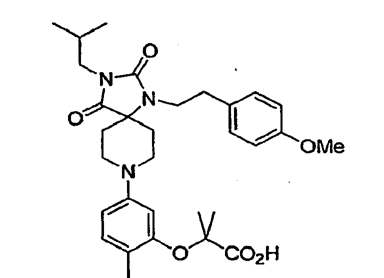 | ¹H-NMR (400 MHz, CDCl₃) δ = 7.19 (d, J = 8.3 Hz, 1H), 7.11 (d, J = 8.2 Hz, 2H), 7.05 (m, 1H), 6.90 (m, 1H), 6.82 (d, J = 8.2 Hz, 2H), 3.93 (m, 2H), 3.76 (s, 3H), 3.47 (m, 2H), 3.39 (t, J = 7.2 Hz, 2H), 3.34 (d, J = 7.4 Hz, 2H), 2.94 (t, J = 7.2 Hz, 2H), 2.38 (m, 2H), 2.21 (s, 3H), 2.09 (m, 1H), 1.61 (s, 6H), 1.49 (d, J = 14.2 Hz, 2H), 0.92 (d, J = 6.7 Hz, 6H). MS calcd. for C₃₁H₄₂N₃O₆ (M+H⁺) 552.3, found 552.3. |
| C14 | 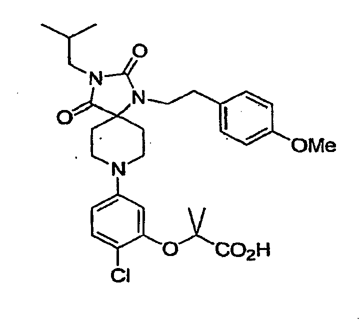 | ¹H-NMR (400 MHz, CDCl₃) δ = 7.43 (d, J = 8.7 Hz, 1H), 7.26 (s, 1H), 7.10 (d, J = 8.3 Hz, 2H), 6.97 (d, J = 8.7 Hz, 1H), 6.82 (d, J = 8.3 Hz, 2H), 3.95 (m, 2H), 3.77 (s, 3H), 3.50 (m, 2H), 3.39 (t, J = 7.2 Hz, 2H), 3.35 (d, J = 7.4 Hz, 2H), 2.96 (t, J = 7.2 Hz, 2H), 2.34 (m, 2H), 2.10 (m, 1H), 1.64 (s, 6H), 1.49 (d, J = 13.7 Hz, 2H), 0.92 (d, J = 6.7 Hz, 6H). MS calcd. for C₃₀H₃₉ClN₃O₆ (M+H⁺) 572.2, found 572.3. |
| C15 | 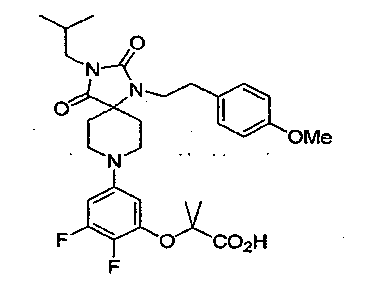 | ¹H-NMR (400 MHz, CDCl₃) δ = 7.10 (d, J = 8.3 Hz, 2H), 6.91 (m, 1H), 6.82 (d, J = 8.3 Hz, 2H), 6.81 (m, 1H), 3.75 (m, 2H), 3.77 (s, 3H), 3.45 (m, 2H), 3.38 (t, J = 7.2 Hz, 2H), 3.34 (d, J = 7.4 Hz, 2H), 2.95 (t, J = 7.2 Hz, 2H), 2.19 (m, 2H), 2.10 (m, 1H), 1.62 (s, 6H), 1.49 (d, J = 14.4 Hz, 2H), 0.92 (d, J = 6.7 Hz, 6H). MS calcd. for C₃₀H₃₈F₂N₃O₆ (M+H⁺) 574.3, found 574.3. |
| C16 | 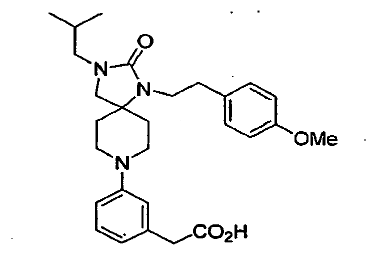 | ¹H-NMR (400 MHz, CDCl₃) δ = 7.25-6.98 (m, 4H), 7.05 (d, J = 8.4 Hz, 2H), 6.82 (d, J = 8.4 Hz, 2H), 3.70 (s, 3H), 3.54 (m, 4H), 3.15 (m, 4H), 2.96 (d, J = 7.3 Hz, 2H), 2.87 (t, J = 11.6 Hz, 2H), 2.75 (t, J = 7.0 Hz, 2H), 2.09 (t, J = 11.6 Hz, 2H), 1.78 (m, 1H), 1.42 (d, J = 13.1 Hz, 2H), 0.83 (d, J = 6.6 Hz, 6H). MS calcd. for C₂₈H₃₈N₃O₄ (M+H⁺) 480.3, found 480.2. |
| C17 | 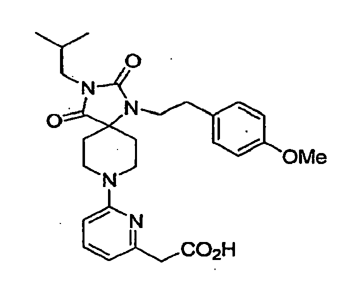 | ¹H-NMR (400 MHz, CDCl₃) δ = 7.69 (t, J = 8.0 Hz, 1H), 7.03 (d, J = 8.5 Hz, 2H), 6.76 (d, J = 9.2 Hz, 1H), 6.73 (d, J = 8.5 Hz, 2H), 6.65 (d, J = 7.2 Hz, 1H), 4.00 (m, 2H), 3.85 (m, 4H), 3.69 (s, 3H), 3.27 (m, 4H), 2.85 (t, J = 7.4 Hz, 2H), 2.03 (m, 1H), 1.88 (m, 2H), 1.55 (d, J = 13.5 Hz, 2H), 0.85 (d, J = 6.7 Hz, 6H). MS calcd. for C₂₇H₃₅N₄O₅ (M+H⁺) 495.3, found 495.2. |
| C18 | 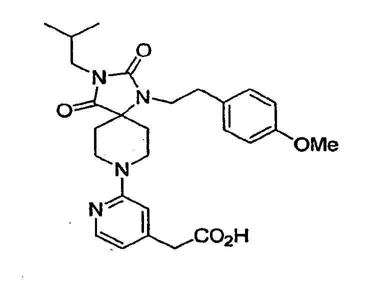 | ¹H-NMR (400 MHz, CDCl₃) δ = 7.90 (d, J = 6.4 Hz, 1H), 7.08 (d, J = 8.5 Hz, 2H), 6.88 (s, 1H), 6.79 (d, J = 8.5 Hz, 2H), 6.73 (d, J = 6.4 Hz, 2H), 4.07 (m, 2H), 3.93 (m, 2H), 3.76 (s, 3H), 3.61 (s, 2H), 3.33 (m, 4H), 2.92 (t, J = 7.4 Hz, 2H), 2.08 (m, 1H), 1.92 (m, 2H), 1.64 (d, J = 14.0 Hz, 2H), 0.91 (d, J = 6.7 Hz, 6H). MS calcd. for C₂₇H₃₅N₄O₅ (M+H⁺) 495.3, found 495.2. |
| C19 | 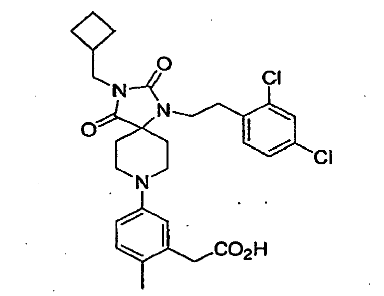 | ¹H-NMR (400 MHz, CDCl₃) δ = 7.48 (d, J = 2.2 Hz, 1H), 7.37 (m, 2H), 7.29 (d, J = 8.4 Hz, 2H), 7.18 (m, 2H), 4.16 (m, 2H), 3.66 (s, 2H), 3.60 (m, 2H), 3.56 (d, J = 7.5 Hz, 2H), 3.49 (t, J = 7.1 Hz, 2H), 3.13 (t, J = 7.1 Hz, 2H), 2.70 (m, 3H), 2.32 (s, 3H), 2.02 (m, 2H), 1.89 (m, 2H), 1.76 (m, 2H), 1.61 (d, J = 14.4 Hz, 2H). MS calcd. for C₂₉H₃₄Cl₂N₃O₄ (M+H⁺) 558.2, found 558.2. |
| C20 | 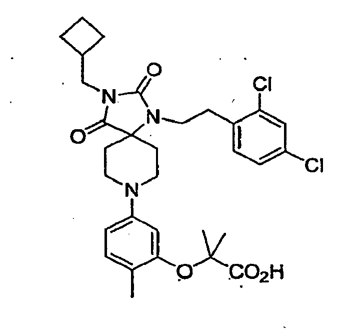 | MS calcd. for C₃₁H₃₈Cl₂N₃O₅ (M+H⁺) 602.2, found 602.2. |
| C21 | 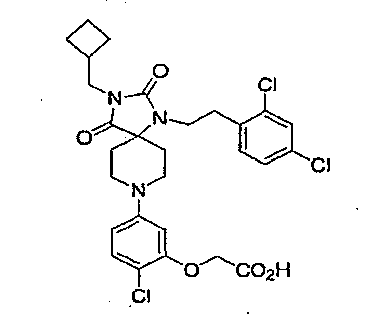 | ¹H-NMR (400 MHz, CDCl₃) δ = 7.46 (d, J = 8.7 Hz, 1H), 7.39 (s, 1H), 7.33 (d, J = 2.2 Hz, 1H), 7.18 (m, 2H), 6.99 (dd, J = 2.2 Hz, J = 8.7 Hz, 1H), 4.79 (s, 2H), 4.07 (m, 2H), 3.59 (m, 2H), 3.56 (d, J = 7.5 Hz, 2H), 3.46 (t, J = 7.2 Hz, 2H), 3.13 (t, J = 7.2 Hz, 2H), 2.69 (m, 1H), 2.49 (m, 2H), 2.02 (m, 2H), 1.90 (m, 2H), 1.76 (m, 2H), 1.60 (d, J = 14.3 Hz, 2H). MS calcd. for C₂₈H₃₁Cl₃N₃O₅ (M+H⁺) 594.1, found 594.1. |
| C22 | 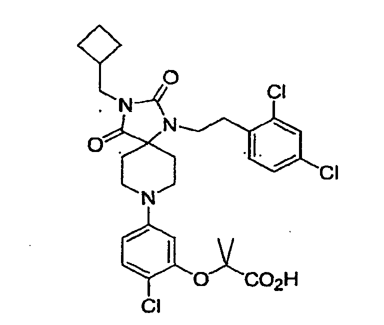 | ¹H-NMR (400 MHz, CDCl₃) δ = 7.48 (d, J = 8.7 Hz, 1H), 7.38 (s, 1H), 7.31 (d, J = 2.1 Hz, 1H), 7.18 (m, 2H), 7.04 (dd, J = 2.2 Hz, J = 8.7 Hz, 1H), 4.79 (s, 2H), 4.05 (m, 2H), 3.58 (m, 2H), 3.56 (d, J = 7.5 Hz, 2H), 3.45 (t, J = 7.2 Hz, 2H), 3.12 (t, J = 7.2 Hz, 2H), 2.69 (m, 1H), 2.47 (m, 2H), 2.03 (m, 2H), 1.90 (m, 2H), 1.76 (m, 2H), 1.65 (s, 6H), 1.60 (d, J = 13.5 Hz, 2H). MS calcd. for C₃₀H₃₅Cl₃N₃O₅ (M+H⁺) 622.2, found 622.1. |
| C23 | 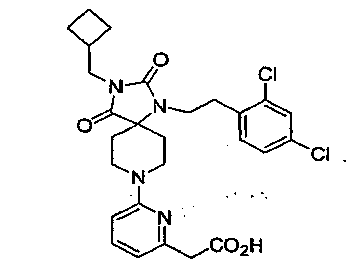 | MS calcd. for C₂₇H₃₁Cl₂N₄O₄ (M+H⁺) 545.2, found 545.2. |
| D2 | 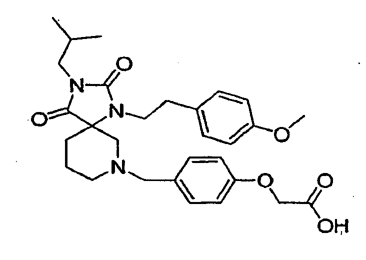 | ¹H-NMR (400 MHz, CDCl3) δ = 7.24 (d, J = 7.6 Hz, 2H), 7.20 (d, J = 8.0 Hz, 2H), 6.93 (s, 4H), 4.79 (s, 2H), 3.92 (s, 3H), 3.62 (m, 1H), 3.45 (m, 3 H), 3.17 (m, 1H), 3.04 (m, 2H), 2.53 (m, 1H), 2.16 (m, 1H), 2.02 (m, 2H), 1.72 (m, 1H), 0.99 (d, J = 6.7 Hz, 6H). MS calcd. for C₂₉H₃₈N₃O₆ (M+H+) 523.3, found 523.3. |
| E2 | 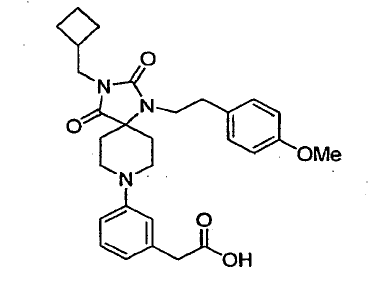 | ¹H NMR (400 MHz, CDCl3) δ 7.62 (s, 1H), 7.54 (d, J = 7.6 Hz, 1H), 7.48 (t, J = 8.2 Hz, 1H), 7.38 (d, J = 7.6 Hz, 1H), 7.14 (d, J = 8.8 Hz, 2H), 6.83 (d, J = 8.6 Hz, 2H), 4.13 (t, J = 11.7 Hz, 2H), 3.76 (s, 3H), 3.72 (s, 2H), 3.53-3.58 (m, 4H), 3.47 (t, , J = 7.2 Hz, 2H), 3.41 (d, J = 7.3 Hz, 2H), 2.99 (t, J = 7.2 Hz, 2H), 2.61-2.75 (m, 3H), 1.73-1.83 (m, 6H), 1.47 (d, J = 14.5 Hz, 2H). LC/MS (M+H⁺): 506.2. |
| E3 | 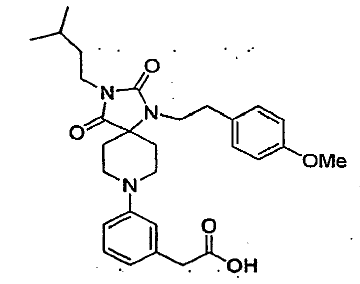 | ¹H NMR (400 MHz, CDCl3) δ 7.62 (s, 1H), 7.5 (m, 1H), 7.46 (t, J = 8.2 Hz, 1H), 7.35 (d, J = 8.3 Hz, 1H), 7.16 (d, J = 8.3 Hz, 2H), 6.83 (d, J = 8.2 Hz, 2H), 4.11 (t, J = 11.7 Hz, 2H), 3.77 (s, 3H), 3.72 (s, 2H), 3.48-3.57 (m, 6H), 2.98(t, J = 6.1 Hz, 2H), 2.68(m, 2), 1.55 ( m, 4H), 1.22 (m, 1H), 0.98 (d, J = 6.2, 6H). LC/MS (M+H⁺): 508.2 |
| E4 | 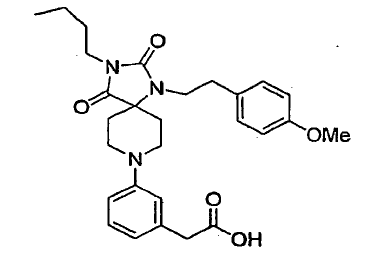 | LC/MS (M+H⁺): 494.2. |
| G2 | 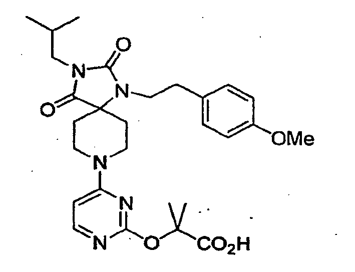 | ¹H-NMR (400MHz, CDCl₃) δ = 7.87 (s, 1H), 7.00 (d, J = 8.5 Hz, 2H), 6.75 (d, J = 8.5 Hz, 2H), 6.32 (s, 1H), 4.53-4.34 (m, 1H), 3.89 (m, 1H), 3.76 (m, 1H), 3.71 (s, 3H), 3.59-3.44 (m, 1H), 3.26 (m, 4H), 2.86 (m, 2H), 2.02 (m, 1H), 1.95-1.62 (m, 2H), 1.64-1.61 (s, 6H), 1.45 (m, 2H), 0.84 (d, J = 6.7 Hz, 6H). MS calculated for C₂₈H₃₈N₅O₆ (M+H⁺) 540.3, found 540.3. |
| G3 | 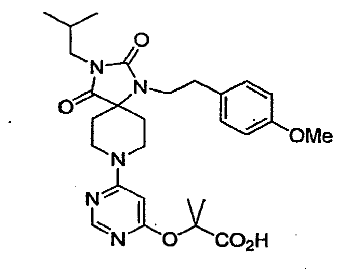 | ¹H-NMR (400MHz, CDCl₃) δ = 8.33 (s, 1H), 7.02 (d, J = 8.5 Hz, 2H), 6.75 (d, J = 8.5 Hz, 2H), 5.92 (s, 1H), 4.04 (m, 2H), 3.77 (m, 2H), 3.71 (s, 3H), 3.27 (m, 4H), 2.86 (t, J = 7.2 Hz, 2H), 2.03 (m, 1H), 1.74 (m, 2H), 1.64 (s, 6H), 1.50 (m, 2H), 0.85 (d, J = 6.7 Hz, 6H). MS calculated for C₂₈H₃₈N₅O₆ (M+H⁺) 540.3, found 540.3. |
| G4 | 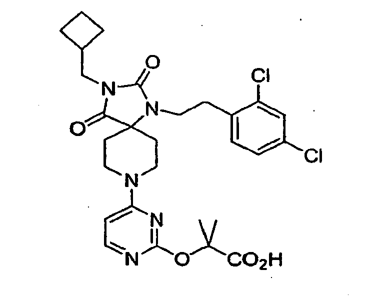 | ¹H-NMR (400 MHz, CDCl₃) δ = 7.94 (d, J = 7.4 Hz, 1H), 7.34 (s, 1H), 7.15 (s, 2H), 6.43 (d, J = 7.4 Hz, 1H), 4.64 (m, 1H), 4.00 (m, 1H), 3.89 (m, 1H), 3.69 (m, 1H), 3.54 (d, J = 7.2 Hz, 2H), 3.36 (m, 2H), 3.08 (t, J = 7.2 Hz, 2H), 2.67 (m, 1H), 2.00-1.62 (m, 16H). MS calcd. for C₂₈H₃₄Cl₂N₅O₅ (M+H⁺) 590.2, found 590.2. |
| G5 | 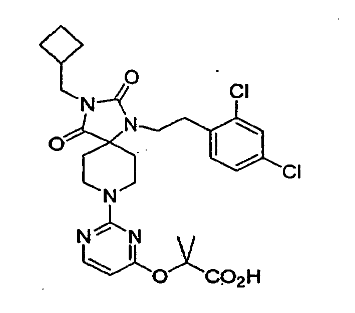 | ¹H-NMR (400 MHz, CDCl₃) δ = 8.13 (d, J = 6.9 Hz, 1H), 7.34 (s, 1H), 7.16 (s, 2H), 6.24 (d, J = 6.9 Hz, 1H), 4.41 (m, 2H), 3.76 (m, 2H), 3.52 (d, J = 7.4 Hz, 2H), 3.31 (m, 2H), 3.05 (m, 2H), 2.66 (m, 1H), 2.02-1.57 (m, 16H). MS calcd. for C₂₈H₃₄Cl₂N₅O₅ (M+H⁺) 590.2, found 590.2. |
| G6 | 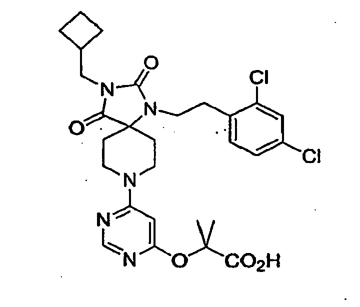 | ¹H-NMR (400 MHz, CDCl₃) δ = 8.42 (s, 1H), 7.37 (s, 1H), 7.17 (s, 2H), 6.03 (s, 1H), 4.16 (m, 2H), 3.88 (m, 2H), 3.54 (d, J = 7.2 Hz, 2H), 3.37 (t, J = 7.2 Hz, 2H), 3.08 (t, J = 7.2 Hz, 2H), 2.68 (m, 1H), 2.00-1.62 (m, 16H). MS calcd. for C₂₈H₃₄Cl₂N₅O₅ (M+H⁺) 590.2, found 590.2. |
| I2 | 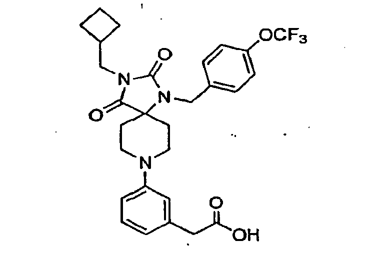 | ¹HNMR (400MHz, CD₃OD) δ 7.48-7.41 (m, 4H), 7.37-7.35 (m, 1H), 7.30-7.24(m, 3H), 4.63 (s, 2H), 4.11-4.05 (m, 2H), 3.69-3.65(m, 2H), 3.61 (d, J = 8.0Hz, 2H), 2.75(quint, J = 8.0Hz, 1H), 2.4. (dt, J = 4.0 and 12.0Hz, 2H), 2.09-2.03 (m, 2H), 1.95-1.80 (m, 6H). MS (m/z) (M+1)⁺ 546.2. |
| I3 | 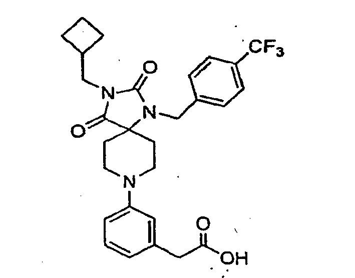 | ¹HNMR (400MHz, CD₃OD) δ 7.60 (d, J = 8.0Hz, 2H), 7.45- (d, J = 8.0Hz, 2H), 7.17(t, J = 8.0 Hz, 1H), 6.89 (s, 1H), 6.85 (d, J = 8.0Hz, 1H), 6.77 (d, J = 8.0Hz, 1H), 4.62 (s, 2H), 3.64-3.57 (m, 4H), 3.54 (bs, 2H), 3.51-3.44 (m, 2H), 2.72 (quint, J = 8.0Hz, 1H), 2.11. (m, 4H), 1.93-1.79 (m, 4H), 1.66-1.63 (m, 2H). MS (m/z) (M+1)⁺ 530.2. |
| I4 | 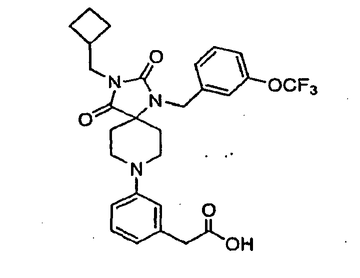 | ¹HNMR (400MHz, CD₃OD) δ 7.48 (t, J = 8.0Hz, 1H), 7.32-7.29 (m, 2H), 7.24 (s, 1H), 7.20-7.18 (m, 1H), 7.11-6.99 (m, 3H), 4.62 (s, 2H), 3.78-3.71 (m, 2H). 3.66-3.59 (m, 6H), 2.75-2.71 (m, 1H), 2.23-2.16 (M, 2H), 2.08-2.01 (m, 2H), 1.93-1.75 (m, 6). MS (m/z) (M+1)⁺ 546.3. |
| I5 | 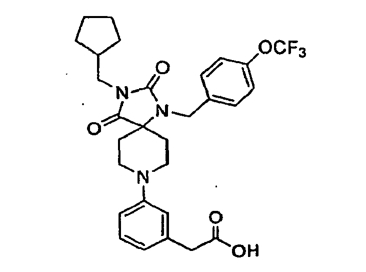 | ¹HNMR (400MHz, CD₃OD) δ 7.41 (d, J = 8.0Hz, 2H), 7.34 (t, J = 8.0Hz, 1H), 7.24 (d, J = 8.0Hz, 2H), 7.18 (s, 1H), 7.14-7.05 (m, 2H), 4.60 (s, 2H), 3.68-3.57 (m, 6H), 2.23 (dt, J = 8.0 and 12.0Hz, 2H), 1.84-1.81 (m, 2H), 1.64-1.52 (m, 3H), 099 (d, J = 8.0 Hz, 6H). MS (m/z) (M+1)⁺ 548.3. |
| I6 | 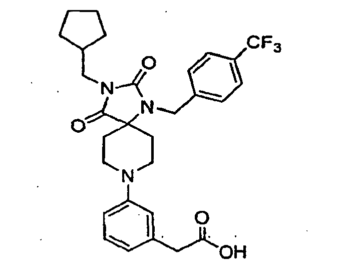 | ¹HNMR (400MHz, CD₃OD) δ 7.64 (d, J = 8.0Hz, 2H), 7.51 (d, J = 8.0Hz, 2H), 7.35 (t, J = 8.0Hz, 1H), 7.21 (s, 1H), 7.15-7.08(m, 2H), 4.67 (s, 2H), 3.89-3.83 (m, 2H), 3.68-3.59 (m, 6H), 2.22 (dt, J = 8.0 and 16Hz, 2H), 1.88-1.84 (m, 2H), 1.63-1.53 (m, 3H), 0.98 (d, J = 8.0Hz, 6H). MS (m/z) (M+1)⁺ 532.3. |
| I7 | 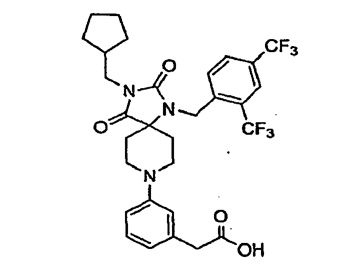 | ¹HNMR (400MHz, CD₃OD) δ 7.99 (s, 1H), 7.93 (d, J = 8.0H, 1H), 7.69 (d, J = 8.0Hz, 1H), 7.33 (t, J = 8.0Hz, 1H), 7.19 (s, 1H), 7.15-7.06 (m, 2H), 4.18 (s, 2H), 3.87-3.84 (m, 2H), 3.68.3.60 (m, 7H), 2.13 (dt J = 8.0 and 16 Hz, 2H), 2.02-1.99 (m, 2H), 1.65-1.55 (m, 3H), 1.00 (d, J = 8.0Hz, 6H). MS (m/z) (M+1)⁺ 600.2. |
| I8 | 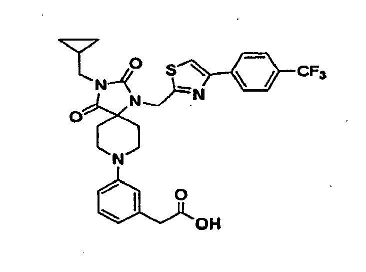 | ¹HNMR (400MHz, CDCl₃) δ 7.98 (d, J = 7.9Hz, 2H), 7.56-7.68 (m, 5H), 7.48 (t, J = 8.1Hz, 1H), 7.38 (d, J = 7.6Hz, 1H), 4.97 (s, 2H), 4.24 (t, J= 11.5Hz, 2H), 3.68 (d, J= 12.1Hz, 2H), 3.47 (d, J= 7Hz, 2H), 3.17 (t, J= 13.1 Hz, 2H), 2.33 (m, 1H), 2.0 (d, J= 14.5Hz, 2H), 1.22 (m, 2H), 0.56 (dd, J = 13.5, 5.4Hz, 2H), 0.4 (dd, J = 9.9, 5.0Hz, 2H). MS (m/z) (M+1)⁺ 655.2. |
| I9 | 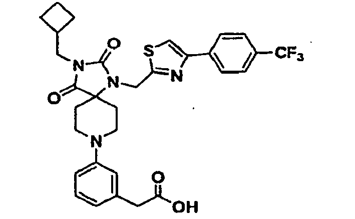 | MS (m/z) (M+1)⁺ 669.2. |
| I10 | 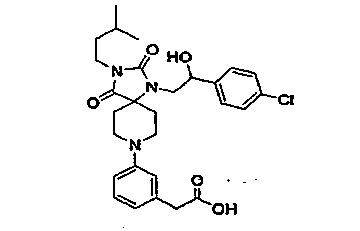 | ¹HNMR (400MHz, CDCl₃) δ 7.73 (s, 1H), 7.65 (d, J = 7.8Hz, 1H), 7.5 (t, J= 7.6Hz, 1H), 7.43 (m, 3 H), 7.35 (s, 2H), 7.34 (d, J= 7.9Hz, 1H), 5.1 (m, 1H), 4.2 (m, 2H), 3.74 (s, 2H), 3.6 (m, 6H), 3.2 (m, 2H), 1.81 (d, J= 12.4Hz, 1H), 1.56 (m, 4H), 0.97 (d, J = 5.2Hz, 6H). MS (m/z) (M+1)⁺ 528.2, 530.2. |
| I11 | 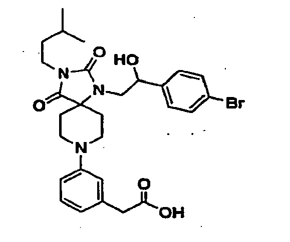 | ¹NMR (400MHz, CDCl₃) δ 7.68 (s, 1H) 7.59 (d, J = 8.2Hz, 1H), 7.49 (m, 3H), 7.41 (d, J = 7.6Hz, 1H), 7.34 (d, J = 8.1Hz, 2H), 5.06 (m, 1H), 4.2 (t, J= 12.4Hz, 2H), 3.72 (s, 2H), 3.61 (m, 6H), 3.08 (m, 2H), 1.81 (d, J= 13.9Hz, 1H), 1.56 (m, 4H), 0.97 (d, J = 6Hz, 6H). MS (m/z) (M+1)⁺ 572.2, 574.2. |
| I12 | 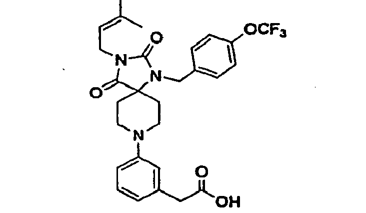 | MS (m/z) (M+1)⁺ 546.2. |
| I13 | 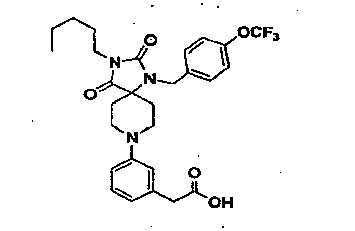 | ¹HNMR (400MHz, CDCl₃) δ 7.78 (s, 1H), 7.69 (d, J = 7.3 Hz, 1H), 7.62 (d, J = 8.1 Hz, 2H), 7.5 (t, J = 7.8Hz, 1H), 7.42 (d, J = 7.5Hz, 1H), 7.18 (d, J = 7.9Hz, 2H), 4.67 (s, 2H), 4.23 (t, J= 12.2Hz, 2H), 3.73 (s, 2H), 3.61 (m, 4H), 3.32 (m, 2H), 1.71 (m, 4H), 1.34 (m, 4H), 0.92 (t, J= 7.2Hz, 3H). MS (m/z) (M+1)⁺ 548.2. |
| I14 | 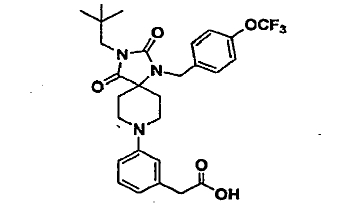 | MS (m/z) (M+1)⁺ 548.2. |
| I15 | 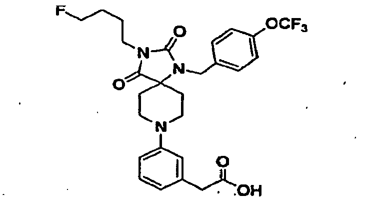 | MS (m/z) (M+1)⁺ 552.2. |
| I16 | 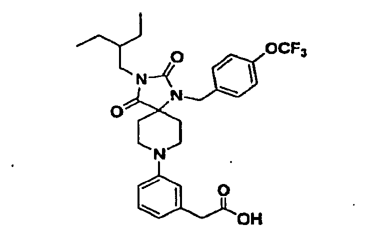 | ¹HNMR (400MHz, CDCl₃) δ 7.78 (s, 1H), 7.7 (d, J = 8.1 Hz, 1H), 7.62 (d, J = 8.1 Hz, 2H), 7.5 (t, J = 7.7Hz, 1H), 7.42 (d, J = 7.6Hz, 1H), 7.18 (d, J = 7.8Hz, 2H), 4.67 (s, 2H), 4.24 (t, J= 11.5Hz, 2H), 3.73 (s, 2H), 3.61 (d, J= 12.5Hz, 2H), 3.49 (d, J= 7.4Hz, 2H), 3.33 (m, 2H), 1.8 (quint, J= 6.7Hz, 1H), 1.72 (d, J= 14Hz, 2H), 1.34 (t, J = 6.5Hz, 4H), 0.94 (m, 6H). MS (m/z) (M+1)⁺ 562.2. |
| I17 | 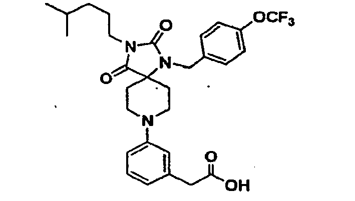 | MS (m/z) (M+1)⁺ 562.2. |
| I18 | 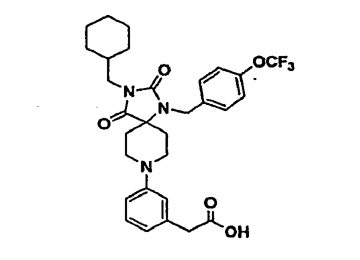 | ¹HNMR (400MHz, CDCl₃) δ 7.69 (s, 1H), 7.61 (d, J = 8.3 Hz, 1H), 7.52 (d, J = 8.3Hz, 2H), 7.48 (t, J = 8.1 Hz, 1H), 7.4 (d, J = 7.5Hz, 1H), 7.18 (d, J = 8.1Hz, 2H), 4.63 (s, 2H), 4.23 (t, J= 11.9Hz, 2H), 3.7 (s, 2H), 3.62 (d, J= 13.2Hz, 2H), 3.42 (d, J= 7.3 Hz, 2H), 3.1(m, 2H), 1.74 (m, 8H), 1.25 (m, 3H), 1.01 (q, J = 12Hz, 2H). MS (m/z) (M+1)⁺ 574.2. |
| I19 | 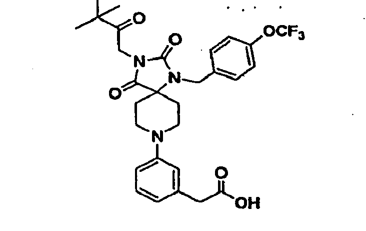 | MS (m/z) (M+1)⁺ 576.2. |
| I20 | 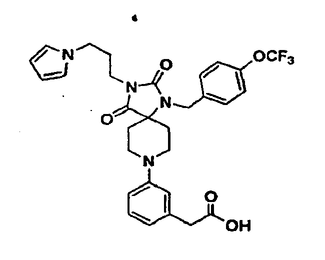 | ¹HNMR (400MHz, CDCl₃) δ 7.81 (s, 1H), 7.72 (d, J = 8.6H, 1H),7.66 (d, J = 8.5Hz, 2H), 7.5 (t, J = 8.0Hz, 1H), 7.43 (d, J = 7.3Hz, 1H), 7.19 (d, J = 8.7Hz, 2H), 6.67(s, 2H), 6.06(s, 2H), 4.64 (s, 2H), 4.5 (t, J = 12.4Hz, 2H), 3.99 (t, J = 6.6Hz, 2H), 3.74 (s, 2H), 3.65 (t, J = 6.6Hz, 2H), 3.56 (d, J = 11.5Hz, 2H), 3.35 (t, J = 11.2Hz, 2H), 2.22 (t, J = 6.6Hz, 2H), 1.6 (d, J= 13.3, 2H). MS (m/z) (M+1)⁺ 585.2. |
| I21 | 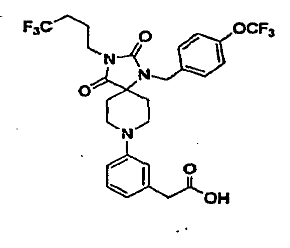 | ¹HNMR (400MHz, CDCl₃) δ 7.81 (s, 1H), 7.73 (d, J = 7.4H, 1H),7.66 (d, J = 8Hz, 2H), 7.51 (t, J = 7.4Hz, 1H), 7.43 (d, J = 8Hz, 1H), 7.19 (d, J = 7.5Hz, 2H), 4.69 (s, 2H), 4.21 (t, J = 11.6Hz, 2H), 3.74 (s, 2H), 3.63 (m, 4H), 3.44 (m, 2H), 1.98 (m, 4H), 1.72 (m, 2H). MS (m/z) (M+1)⁺ 588.2. |
| I22 | 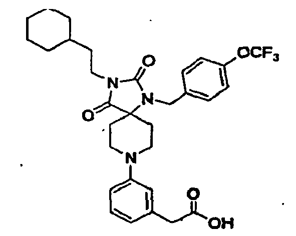 | MS (m/z) (M+1)⁺ 588.2. |
| I23 | 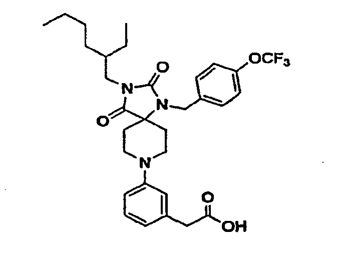 | ¹HNMR (400MHz, CDCl₃) δ 7.74 (s, 1H), 7.66 (d, J = 7.8H, 1H),7.58 (d, J = 8.3Hz, 2H), 7.49 (t, J = 8.0Hz, 1H), 7.41 (d, J = 7.5 Hz, 1H), 7.18 (d, J = 8.1 Hz, 2H), 4.66 (s, 2H), 4.22 (t, J = 11.7Hz, 2H), 3.72 (s, 2H), 3.62 (d, J = 8Hz, 2H), 3.49 (d, J = 7.4Hz, 2H), 3.24 (m, 2H), 1.84 (m, 1H), 1.71 (d, J = 14.6Hz, 2H), 1.29 (m, 10H), 0.93 (t, J = 7.3 Hz, 3H), 0.89 (t, J = 6.7Hz, 3H). MS (m/z) (M+1)⁺ 590.2. |
| I24 | 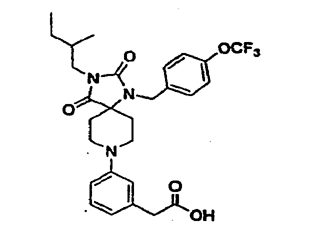 | ¹HNMR (400MHz, CDCl₃) δ 7.79 (s, 1H), 7.7 (d, J = 7.3Hz, 1H), 7.62 (d, J = 8.2Hz, 2H), 7.5 (t, J = 7.9Hz, 1H), 7.42 (d, J = 7.6Hz, 1H), 7.18 (d, J = 7.4Hz, 2H), 4.66 (s, 2H), 4.23 (m, 2H), 3.73 (s, 2H), 3.6 (m, 2H), 3.32 (m, 2H), 1.72 (d, J = 12.9Hz, 2H), 1.58 (m, 3H), 0.97 (m, 6H). MS (m/z) (M+1)⁺ 548.2. |
| I25 | 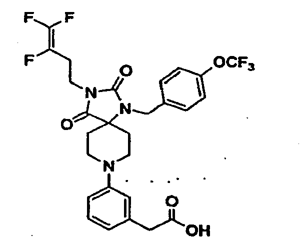 | MS (m/z) (M+1)⁺ 586.2. |
| I26 | 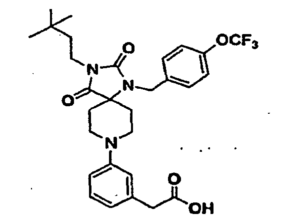 | MS (m/z) (M+1)⁺ 562.2. |
| I27 | 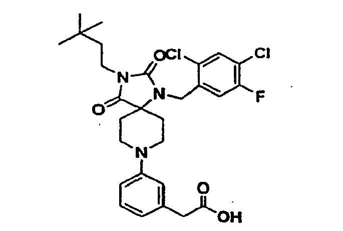 | MS (m/z) (M+1)⁺ 564.2, 565.2, 566.2, 567.2, 568.2. |
| I28 | 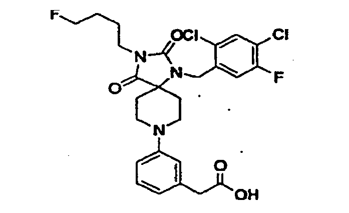 | MS (m/z) (M+1)⁺ 554.2, 555.2, 556.2, 557.2, 558.2. |
| J2 | 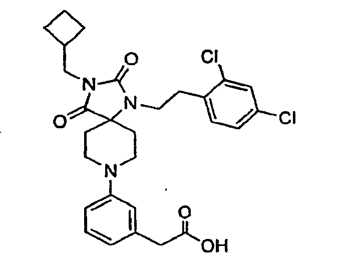 | ¹HNMR (400MHz, CDCl₃) δ 7.42-7.32 (m, 4H), 7.22-7.17 (m, 3H), 3.99 (t, J = 8.0Hz, 2H), 3.66 (s, 2H), 3.63-3.55 (m, 4H), 3.49-3.46 (m, 2H), 3.14-3.11 (m, 2H), 2.68 (quint, J = 8.0Hz, 1H), 2.55-2.48 (m, 2H), 2.05-2.01 (m, 2H), 1.91-1.87 (m, 2H), 1.81-1.74 (m, 2H), 1.59-1.56 (m, 2H). MS (m/z) (M+1)⁺ 545.3. |
| J3 | 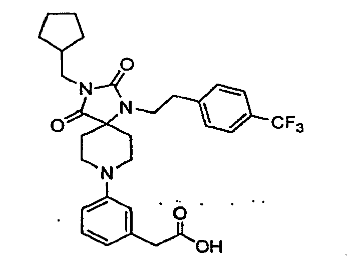 | ¹HNMR (400MHz, CD₃OD) δ 7.60 (d, J = 8.0Hz, 2H), 7.45 (d, J = 8.0H, 1H), 7.34 (t, J = 8.0Hz, 1H), 7.18 (s, 1H), 7.12 (d, J = 8.0Hz, 1H), 7.03 (d, J = 8.0Hz, 1H), 3.82 (t, J = 8.0Hz 2H), 3.68-3.64 (m, 4H), 3.55-3.47 (m, 4H), 3.10-3.04 (m, 2H), 2.18 (dt, J = 4.0 and 12.0 Hz, 2H), 1.72 (m, 2H), 1.58-1.48 (m, 3H), 0.96 (t, J = 4.0Hz,6H). MS (m/z) (M+1)⁺ 546.3. |
| J4 | 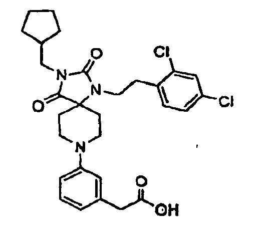 | ¹HNMR (400MHz, CD₃OD) δ 7.45 (s, 1H), 7.31-726 (m, 3H), 7.06 (s, 1H), 7.02 (d, J = 8.0H, 1H), 6.92 (d, J = 8.0Hz, 1H),3.68 (m, 4H), 3.60 (s, 2H), 3.52 (t, J = 8.0Hz 2H),3.42 (t, J = 8.0Hz, 2H), 3.12-3.08 (m, 2H), 2.12-2.04 (m, 2H), 1.65-1.47 (m, 6H), 1.31 (t, J = 8.0Hz, 2H), 0.97 (d, J = 4.0Hz, 6H). MS (m/z) (M+1)⁺ 547.2. |
| J5 | 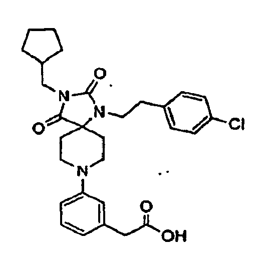 | ¹HNMR (400MHz, CD₃OD) δ 7.42 (t, J = 8.0Hz, 1H), 7.33-7.28 (m, 4H), 7.26-7.18 (m, 3H), 3.94 (dt, J = 4.0 and 12.0Hz, 2H), 3.70-3.64 (m, 4H) 3.54 (t, J = 8.0Hz, 2H), 3.45 (t, J = 8.0Hz, 2H), 2.26 (dt, J = 4.0 and 16.0 Hz, 2H), 1.77.1.74 (m, 2H), 1.58-1.45 (m, 3H), 0.97 (d, J = 4.0Hz, 6H). MS (m/z) (M+1)⁺ 513.2. |
| J6 | 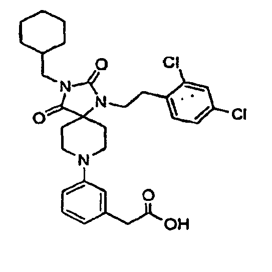 | ¹HNMR (400MHz, CDCl₃) δ 7.49 (s, 1H), 7.44-7.43 (m, 2H), 7.38 (s, 1H), 7.31-7.29 (m, 1H), 7.23-7.16 (m, 2H), 4.13-4.09 (M, 2H), 3.69 (s, 2H), 3.64-3.60 (m, 2H), 3.51 (t, J = 8.0Hz, 2H), 3.35 (m, 2H), 3.16 (t, J = 8.0Hz, 2H), 2.68-2.63 (m, 4H), 1.76-160 (m, 4H), 1.28-1.18 (m, 4H), 0.9-0.87 (m,3H).. MS (m/z) (M+1)⁺ 573.2. |

### Transcriptional Assay

Transfection assays are used to assess the ability of compounds of the invention to modulate the transcriptional activity of the PPARs. Briefly, expression vectors for chimeric proteins containing the DNA binding domain of yeast GAL4 fused to the ligand-binding domain (LBD) of either PPARδ, PPARα or PPARγ are introduced via transient transfection into mammalian cells, together with a reporter plasmid where the luciferase gene is under the control of a GAL4 binding site. Upon exposure to a PPAR modulator, PPAR transcriptional activity varies, and this can be monitored by changes in luciferase levels. If transfected cells are exposed to a PPAR agonist, PPAR-dependent transcriptional activity increases and luciferase levels rise.

293T human embryonic kidney cells (8x10⁶) are seeded in a 175cm² flask a day prior to the start of the experiment in 10% FBS, 1% Penicillin/Streptomycin/Fungizome, DMEM Media. The cells are harvested by washing with PBS (30ml) and then dissociating using trypsin (0.05%; 3ml). The trypsin is inactivated by the addition of assay media (DMEM, CA-dextran fetal bovine serum (5%). The cells are spun down and resuspended to 170,000cells/ml. A Transfection mixture of GAL4-PPAR LBD expression plasmid (1µg), UAS-luciferase reporter plasmid (1µg), Fugene (3:1 ratio; 6µL) and serum-free media (200µL) was prepared and incubated for 15-40 minutes at room temperature. Transfection mixtures are added to the cells to give 0.16M cells/mL, and cells (50µl/well) are then plated into 384 white, solid-bottom, TC-treated plates. The cells are further incubated at 37°C, 5.0% CO₂ for 5-7 hours. A 12-point series of dilutions (3 fold serial dilutions) are prepared for each test compound in DMSO with a starting compound concentration of 10µM. Test compound (500nl) is added to each well of cells in the assay plate and the cells are incubated at 37°C, 5.0% CO₂ for 18-24 hours. The cell lysis/luciferase assay buffer, Bright-Glo^{™} (25%; 25µl; Promega), is added to each well. After a further incubation for 5 minutes at room temperature, the luciferase activity is measured.

Raw luminescence values are normalized by dividing them by the value of the DMSO control present on each plate. Normalized data is analyzed and dose-response curves are fitted using Prizm graph fitting program. EC50 is defined as the concentration at which the compound elicits a response that is half way between the maximum and minimum values. Relative efficacy (or percent efficacy) is calculated by comparison of the response elicited by the compound, with the maximum value obtained for a reference PPAR modulator.

Compounds of Formula I, in free form or in pharmaceutically acceptable salt form, exhibit valuable pharmacological properties, for example, as indicated by the *in vitro* tests described in this application. Compounds of the invention preferably have an EC50 for PPARδ and/or PPARα and/or PPARγ, of less than 5µM, more preferably less than 1µM, more preferably less than 500nm, more preferably less than 100nM. Compounds of the invention preferably have an EC50 for PPARδ that is less than or equal to PPARa which in turn has an EC50 that is at least 10-fold less than PPARγ.

It is understood that the examples and embodiments described herein are for illustrative purposes only.

## Claims

1. A compound selected from Ia, Ib and Ic: 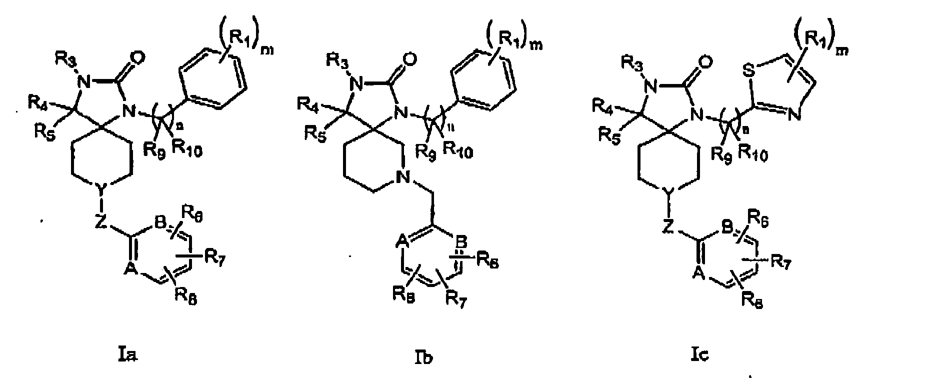 in which:
n is selected from 1 or 2;
m is selected from 1, 2, 3, 4 and 5; each
R₁ is independently selected from hydrogen, halo, C₁₋₆alkyl, halo-substituted C₁₋₆alkyl, C₁₋₆alkoxy and halo-substituted-C₁₋₆alkoxy;
R₃ is selected from C₁₋₆alkyl, C₂₋₈alkenyl, halo-substituted-C₁₋₆alkyl, halo-substituted-C₂₋₆alkenyl, C₅₋₁₀hteteroaryl-C₀₋₄alkyl and C₃₋₁₂cycloalkyl-C₀₋₄alkyl; wherein R₂ is selected from hydrogen and C₁₋₆alkyl;
R₄ is selected from hydrogen and C₁₋₄alkyl;
R₅ is selected from hydrogen and C₁₋₆alkyl; or R₄ and R₅ together with the carbon atom to which R₄ and R₅ are both attached form carbonyl;
Y is N;
Z is selected from a bond, -S(O)₀₋₂- and -CR₁₁R₁₂-; wherein R₁₁ and R₁₂ are independently selected from hydrogen and C₁₋₆alkyl;
A and B are independently selected from CH and N;
R₆ and R₇ are independently selected from hydrogen, halo, C₁₋₆alkyl, halo-substituted C₁₋₆alkyl, C₁₋₆alkoxy and halo-substituted-C₁₋₆alkoxy;
R₈ is selected from -X₂CO₂R₁₃, -X₂CR₁₄R₁₅X₃CO₂R_{13,} - X₂SCR₁₄R₁₅X₃C3CO₂R₁₃ and -X₂OCR₁₄R₁₅X₃CO₂R₁₃; wherein X₂ and X₃ are independently selected from a bond and C₁₋₄alkylene; and R₁₄ and R₁₅ are independently selected from hydrogen, C₁₋₄alkyl and C₁₋₄alkoxy; or R₁₄ and R₁₅ together with the carbon atom to which R₁₄ and R₁₅ are attached form C₃₋₁₂cycloalkyl; and R₁₃ is selected from hydrogen and C₁₋₆alkyl;
R₉ and R₁₀ are independently selected from hydrogen C₁₋₆alkyl and -OR₁₆;
wherein R₁₆ is selected from hydrogen and C₁₋₆alkyl; and the pharmaceutically acceptable salts, hydrates, solvates and isomers thereof.

2. The compound of claim 1 in which:
n is selected from 1 or 2;
m is selected from 1, 2 and 3; each
R₁ is independently selected from hydrogen, halo, C₁₋₆alkyl, halo-substituted C₁₋₆alkyl, C₁₋₆alkoxy and halo-substituted-C₁₋₆alkoxy;
R₃ is selected from C₁₋₈alkyl, C₂₋₈alkenyl, halo-substituted-C₁₋₆alkyl, halo-substituted-C₂₋₆alkenyl, C₅₋₁₀heteroaryl-C₀₋₄alkyl and C₃₋₁₂cycloalkyl-C₀₋₄alkyl; wherein R₂ is selected from hydrogen and C₁₋₆alkyl;
R₄ is selected from hydrogen and C₁₋₆alkyl;
R₅ is selected from hydrogen and C₁₋₆alkyl; or R₄ and R₅ together with the carbon atom to which R₄ and R₅ are both attached form carbonyl;
Y is N;
Z is selected from a bond, -S(O)₀₋₂- and -CR₁₁R₁₂-; wherein R₁₁ and R₁₂ are independently selected from hydrogen and C₁₋₆alkyl;
A and B are independently selected from CH and N;
R₆ and R₇ are independently selected from hydrogen, halo, C₁₋₆alkyl, halo-substituted C₁₋₆alkyl and C₁₋₆alkoxy;
R₈ is selected from -X₂CO₂R₁₃, -X₂CR₁₄R₁₅X₃CO₂R₁₃ and-X₂OCR₁₄R₁₅X₃CO₂R₁₃; wherein X₂ and X₃ are independently selected from a bond and C₁₋₄alkylene; and R₁₄ and R₁₅ are independently selected from hydrogen and C₁₋₄alkyl; R₁₃ is selected from hydrogen and C₁₋₆alkyl, and
R₉ and R₁₀ are independently selected from hydrogen, C₁₋₆alkyl and -OR₁₆;
wherein R₁₆ is selected from hydrogen and C₁₋₆alkyl.

3. The compound of claim 2 in which: R₁ is independently selected from hydrogen, halo, methoxy, trifluormethoxy and trifluoromethyl; R₃ is selected from isobutyl, cyclopropyl-methyl, cyclobutyl-methyl, isopentyl, butyl, cyclopentyl-methyl, 3-methyl-but-2-enyl, pentyl, 2,2-dimothyl-propyl, 4-fluoro-butyl, 2-ethyl-butyl, 2-methyl-pentyl, oyclohexyl-methyl, 3,3-dimethyl-2-oxo-butyl, pyrrolyl-propyl, 3-trifluoromethyl-propyl, cyclohexyl-ethyl, 2-ethyl-hexyl, 2-methyl-butyl, 3,4,4-triflucro-but-3-enyl and 3,3-dimethyl-butyl; R₄ and R₅ are each hydrogen or R₄ and R₅ together with the carbon atom to which R₄ and R₅ are both attached form carbonyl; and Z is selected from a bond, -S(O)₂- and -CH₂-.

4. The compound of claim 3 in which: R₉ is selected from -CH₂C(O)OH, - CH(CH₂)C(O)OH, -OC(CH₂)C(O)OH, -(CH₂)₂C(O)OH and -OCR₂C(O)OH; and R₉ and R₁₀ are independently selected from hydrogen, halo, methyl, methoxy and trifluoromethyl.

5. The compound of claim 1 selected from: (3-{3-Isobutyl-1-[2-(4-methoxyphenyl)-ethyl]-2,4-dioxo-1,3,8-triaza-spiro[4.5]dec-8-ylmethyl}phenyl)-acetic acid; (3-{3-Isobutyl-1-[2-(4-methoxy-phenyl)-ethyl]-2,4-dioxo-1,3,8-triaza-spiro[4.5]decane-8-sulfonyl)-4-methyl-phenyl)-acetic acid; (3-{3-Isobutyl-1-[2-(4-methoxy-phenyl)-ethyl]-2,4-dioxo-1,3,8-triaza-spiro[4.5]dec-8-yl}-phenyl)-acetic acid; 2-(4-{3-Isobutyl-[2-(4-methoxy-phenyl)-ethyl]-2,4-dioxo-1,3-diaza-spiro[4.5]dec-7-ylmethyl}-phonyl)-propionic acid; (3-{3-Cyclopropylmethyl-1-[2-(4-methoxy-phenyl)-ethyl]-2,4-dioxo-1,3,8-triaza-spiro[4.5]dec-8-yl}-phenyl)-acetic acid; {3-[3-Isobutyl-2,4-dioxo-1-(4-trifluoromethoxybenzyl)-1,3,8-triaza-spiro[4.5]dec-8-yl]-phenyl}-acetic acid; 2-(2-{3-Isobutyl-1-[2-(4-methoxy-phenyl)-ethyl]-2,4-dioxo-1,3,8-triaza-spixo[4.5]dec-8-yl}-pyriroidin-4-yloxy)-2-methyl-propionic acid; 2-(3-{3-Cyclobutylmethyl-1-[2-(2,4-dichloro-phenyl)-ethyl]-2,4-dioxo-1,3-diaza-spiro[4.5]dec-8-yl}-phenoxy)-2-methyl-propionic acid; {3-[3-Cyclopropylmethyl-2,4-dioxo-1-(4-trifluoromethox-benzyl)-1,3,8-triaza-spiro[4.5]dec-8-yl]-phenyl}-acetic acid; (3-{3-Cyclobutylmethyl-2,4-dioxo-1--[2-(4-trifluoromethyl-phenyl)-ethyl]-1,3,8-triaza-spiro[4.5]dec-8-yl}-phenyl)-acetic acid; (3-{3-Cyelobutylmethyl-1-[4-(4-methoxy-phenyl)-butyl]-2,4-dioxo-1,3,8-triaza-spiro[4.5]dec-8-yl}-phenyl)-acetic acid; 3-{3-Isobutyl-1-[2-(4-methoxy-phenyl)-ethyl]-2,4-dioxo-1,3,8-triaza-spiro[4.5]dec-8-ylmethyl}-benzoic acid, (2-{3-Isabutyl-1-[2-(4-methoxy-phenyl)-ethyl]-2,4-dioxo-1,3,8-triaza-spiro[4.5]dec-8-ylmethyl}-phenyl)-acetic acid; (3-{3-Isobutyl-1-[2-(4-methoxyphenyl)-ethyl]-2,4-dioxo-1,3,8-triaza-spiro[4.5]decane-8-sulfonyl}-4-methoxy-phenyl)-acetic acid; 3-(3-{3-Isobutyl-1-[2-(4-mothoxy-phenyl)-ethyl]-2,4-dioxo-1,3,8-triaza-spiro[4.5]dec-8-yl}-phanyl)-propionic acid; (3-{3-Isobutyl-1-[2-(4-methoxy-phenyl)-ethyl]-2,4-dioxo-1,3,8-triaza-spiro[4.5]dec-8-yl}-phenoxy)-acetic acid; 2-(3-{3-Isobutyl-1-[2-(4-methoxy-phenyl)-ethyl]-2,4-dioxo-1,3,8-triaza-spiro[4.5]dec-8-yl}-phenoxy)-2-methyl-propionic acid; (5-{3-Isobtrtyl-1-[2-(4-methoxy-phenyl)-ethyl]-2,4-dioxo-1,3,8-triaza-spiro[4.5]dec-8-yl}-2-methyl-phenyl)-acetic acid; (3-{3-Isobutyl-1-[2-(4-methoxy-phenyl)-ethyl]-2,4-dioxo-1,3,8-triaza-spiro[4.5]dec-8-yl}-5-methyl-phenyl)-acetic acid; (2-Fluoro-5-{3-isobutyl-1-[2-(4-methoxy-phenyl)-ethyl]-2,4-dioxo-1,3,8-triaza-spiro[4.5]dec-8-yl}-phenyl)-acetic acid; (5-{3-Isobutyl-1-[2-(4-methoxy-phenyl)-ethyl]-2,4-dioxo-1,3,8-triaza-spiro[4.5]dec-8-yl}-2-trifluoromethyl-phenyl)-acetic acid; (5-{3-Isobutyl-1-[2-(4-methoxyphenyl)-ethyl]-2,4-dioxo-1,3,8-triaza-spiro[4.5]dec-8-yl}-2-inethoxy-phenyl)-acetic acid; 2-(3-{3-Isobutyl-1-[2-(4-methoxy-phenyl)-ethyl]-2,4-dioxo-1,3,8-triaza-spiro[4.5]dec-8-yl}-phenyl)-2-mctltyl-propionic acid; (5-{3-Isobutyl-1-[2-(4-methoxy-phenyl)-ethyl]-2,4-dioxo-1,3,8-triaza-spiro[4.5]dec-8-yl}-2-methyl-phenoxy)-acetic acid; (2-Chloro-5-{3-isobutyl-1-[2-(4-methoxy-phenyl)-ethyl]-2,4-dioxo-1,3,8-triaza-spiro[4.5]dec-8-yl}-phenoxy)-acetic acid; 2-(5-{3-rsobutyl-1-[2-(4-methoxy-phenyl)ethyl]-2,4-dioxo-1,3,8-triaza-spiro[4.5]dec-8-yl}-2-methyl-phenoxy)-2-methyl-propionic acid; 2-(2-Chloro-5-{3-isobutyl-1-[2-(4-methoxy-phenyl)-ethyl]-2,4-dioxo-1,3,8-triaza-spiro[4.5]dec-8-yl}-phenoxy)-2-methyl-propionic acid; 2-(2,3-Difluoro-5-{3-isobutyl-1-[2-(4-methoxy-phenyl)-ethyl]-2,4-dioxo-1,3,8-triaza-spiro[4.5]dec-8-yl}phenoxy)-2-methyl-propionic acid; (3-{3-Isobutyl-1-[2-(4-methoxy-phenyl)-ethyl]-2-oxo-1,3,8-triaxa-spiro[4.5]dec-8-yl}-phenyl)-acetic acid; (6-{3-Isobutyl-[2-(4-methoxy-phenyl)-ethyl]-2,4-dioxo-1,3,8-triaza-spiro[4.5]dec-8-yl}-pyridin-2-yl)-acetic acid; (2-{3-Isobutyl-1-[2-(4-methoxy-phenyl)-ethyl]-2,4-dioxo-1,3,8-triaza-spiro[4.5]dec-8-yl}-pyridin-4-yl)-acotic acid; (5-{3-Cyclobutylmethyl-1-[2-(2,4-dichlorophenyl)-ethyl]-2,4-dioxo-1,3,9-triaza-spiro[4.5]dec-8-yl}-2-methyl-phenyl)-acetic acid; 2-(S-(3-Cyclobutylmethyl-1-[2-(2,4-dichloro-phenyl)-ethyl]-2,4-dioxo-1,3,8-triaza-spiro[4.5]dec-8-yl)-2-methyl-phenoxy)-2-methyl-propionic acid; (2-Chloro-5-{3-cyclobutylmethyl-1-[2-(2,4-dichloro-phenyl)-ethy]-2,4-dioxo-1,3,8-triaza-spiro[4.5]dec-8-yl}-phenoxy)-acetic acid; 2-(2-Chloro-5-{3-cyclobutylmethyl-1-[2-(2,4-dichloro-phenyl)-ethyl]-2,4-dioxo-1,3,8-triaza-spiro[4.5]dec-8-yl}-phenoxy)-2-methyl-propionic acid; (6-{3-Cyclobutylmethyl-1-[2-(2,4-dichloro-phenyl)-ethyl]-2,4-dioxo-1,3,8-triaza-spiro[4.5]dec-8-yl}-pyridin-2-yl)-acetic acid; (4-{3-Isobutyl-1-[2-(4-methoxy-phenyl)-ethyl]-2,4-dioxo-1,3,7-triaza-spiro[4.5]dec-7-ylmethyl}-phenoxy)-acetic acid; (3-{3-Cyclobutylmethyl-1-[2-(4-methoxy-phenyl)-ethyl]-2,4-dioxo-1,3,8-triaza-spiro[4.5]dec-8-yl}-phenyl)-acetic acid; {3-[1-[2-(4-Methoxyphenyl)-ethyl]-3-(3-methyl-butyl)-2,4-dioxo-1,3,8-triaza-spiro[4.5]dec-8-yl]-phenyl}-acetic acid; (3-{3-Butyl-1-[2-(4-methoxy-phenyl)-ethyl]-2,4-dioxo-1,3,8-triaza-spiro[4.5]dec-8-yl}-phenyl)-acetic acid; 2-(4-{3-Isobutyl-1-[2-(4-methoxy-phenyl)-ethyl]-2,4-dioxo-1,3,8-triaza-spiro[4.5]dec-8-yl}-pyimidin-2-yloxy)-2-methyl-propionic acid; 2-(6-{3-Isobutyl-1-[2-(4-methoxy-phenyl)-ethyl]-2,4-dioxo-1,3,8-triaza-spiro[4.5]dec-8-yl}-pyrimidin-4-yloxy)-2-methyl-propionic acid; 2-(4-{3-Cyclobutylmethyl-1-[2-(2,4-dichloro-phenyl)-ethyl]-2,4-dioxo-1,3,8-triaza-spiro[4.5]dec-8-yl}-pyrimidin-2-yloxy)-2-methyl-propionic acid; 2-(2-{3-Cyclobutylmethyl-1-[2-(2,4-dichloro-phonyl)-ethyl]-2,4-dioxo-1,3,8-triaza-spiro[4.5]dec-8-yl}-pyrimidin-4-yloxy)-2-methyl-propionic acid; 2-(6-{3-Cyclobutylmethyl-1-[2-(2,4-dichloro-phenyl)-ethyl]-2,4-dioxo-1,3,8-triaza-spiro[4.5]dec-8-yl}-pyrimidin-4-yloxy)-2-methyl-propionic acid; {3-[3-Cylobutylmethyl-2,4-dioxo-1-(4-trifluoromethoxybenzyl)-1,3,8-triaza-spiro[4.5]dec-8-yl]-phenyl}-acetic acid; {3-[3-Cyclobutylmethyl-2,4-dioxo-1-(4-tritluoromethyl benzyl)-1,3,8-triaza-spiro[4.5]dec-8-yl]-phenyl}-acetic acid; {3-[3-Cyclopentylmethyl-2,4-dioxo-1-(4-trifluoromethyl-benzyl)-1,3,8-triaza-spiro[4.5]dec-8-yl]-phenyl}-acetic acid; {3-[3-Cyclopentylmethyl-2,4-dioxo-1-(4-trifluoromethoxy-benyl)-1,3,8-triaza-spiro[4.5]dec-8-yl]-phenyl}-acetic acid; {3-[1-(2,4-Bis-trifiluoromethyl-benzyl)-3-cyclopentylmethyl-2,4-dioxo-1,3,8-triaza-spiro[4.5]dec-5-yl]phenyl}-acetic acid; {3-[3-Cyclobutylmethyl-2,4-dioxo-1-(3-ttifluoromethoxy-benzyl)-1,3,8-triaza-spiro[4.5]dec-8-yl]-phenyl}-acetic acid; (3-{3-Cyclobutylmethyl-2,4-dioxo-1-[4-(4-trifluoromethyl-phenyl)-thiazol-2-ylmethyl]-1,3,8-triaza-spiro[4.5]dec-8-yl}-phenyl)-acetic acid; (3-{3-Cyclopropylmethyl-2,4-dioxo-1-[4-(4-trifluoromethyhphenyl)-titiazol-2-ylmethyl]-1,3,8-triaza-spiro[4.5]dec-8-yl}phenyl)-acetic acid; {3-(3-(3-Methyl-but-2-enyl)-2,4-dioxo-1-(4-trifluoromethoxy-benzyl)-1,3,8-triaza-spiro[4.5]dec-8-yl)-phenyl}-acetic acid; {3-[1-[2-(4-Bromo-phenyl)-2-hydroxy-ethyl]-3-(3-methyl-butyl)-2,4-dioxo-1,3,8-triaza-spiro[4.5]dec-8-yl]-phenyl}-acetic acid; {3-[1-[2-(4-Chloro-phenyl)-2-hydroxy-ethyl]-3-(3-methyl-butyl)-2,4-dioxo-1,3,8-triaza-sprio[4.5]dec-8-yl]-phenyl}-acetic acid; {3-[2,4-Dioxo-3-pentyl-1-(4-trifluoromethoxy-benzyl)-1,3,8-triaza-spiro[4.5]dec-8-yl]-phenyl}-acetic acid; {3-[3-(2,2-Dimethyl-propyl)-2,4-dioxo-1-(4-trifluoromethoxy-benzyl)-1,3,8-triaza-spiro[4.5]dec-8-yl]-phenyl}-acetic acid; {3-[3-(2-Ethyl-butyl)-2,4-dioxo-1-(4-trifluoromethoxy-benzyl)-1,3,8-triaza-spiro[4.5]dec-8-yl]-phenyl}-acetic acid; {3-[3-(4-Fluoro-butyl)-2,4-dioxo-1-(4-trifluaromethoxy-benzyl)-1,3,8-triaza-spiro[4.5]dec-8-yl]-phenyl}-acetic acid; {3-[3-(4-Methyl-pentyl)-2,4-dioxo-2-(4-trifluoromthoxy-benzyl)-1,3,8-triaza-spiro[4.5]dec-8-yl]-phenyl}-acetic acid; {3-[3-Cyclobexylniethyl-2,4-dioxo-1-(4-trifluoromethoxy-benzyl)-1,3,8-triaza-spiro[4.5]dec-8-yl]-phenyl}-acetic acid; {3-[2,4-Dioxo-3-(3-pyrrol-1-yl-propyl)-1-(4-trifluoro-methoxy-benzyl)-1,3,8-triaza-spiro[4.5]dec-8-yl]-phenyl}-acetic acid; {3-[3-(3,3-Dimethyl-2-oxo-butyl)-2,4-choxo-1-(4-trifluoromethoxy-benzyl)-1,3,8-triaza-spiro[4.5]dec-8-yl]-phenyl}-acetic acid; {3-[2,4-Dioxo-3-(4,4,4--trifluoro-butyl)-1-(4-trifluoromethoxy-benzyl)-1,3,8-triaza-spiro[4.5]dec-8-yl]-phenyl}-acetic acid; {3-[3-(2-Cyclohexyl-ethyl)-2,4-dioxo-1-(4-trifluoromethoxy-benzyl)-1,3,8-triaza-spiro[4.5]dec-8-yl]-phenyl}-acetic acid; {3-[3-(2-Ethyl-hexyl)-2,4-dioxo-1-(4-trifluoromethoxy-benzyl)-1,3,8-triaza-spiro[4.5]dec-8-yl]-phenyl}-acetic acid; {3-[3-(2-Methyl-butyl)-2,4-dioxo-1-(4-trifluoromethoxy-benzyl)-1,3,8-triaza-spiro[4.5]dec-8-yl]-phenyl)-acetic acid; {3-[2,4-Dioxo-3-(3,4,4-trifluoro-but-3-enyl)-1-(4-trifluoromethoxy-benzyl)-1,3,8-triaza-spiro[4.5]dec-8-yl]-phenyl}-acetic acid; {3-[3-(3,3-Dimethyl-butyl)-2,4-dioxo-1-(4-trifluoromethoxy-benzyl)-1,3,8-triaza-spiro[4.5]dec-8-yl]-phenyl}-acetic acid; {3-[1-(2,4-Dichloro-5-fluoro-benzyl)-3-(3,3-dimethyl-butyl)-2,4-dioxo-1,3,8-triaza-spiro[4.5]dec-8-yl]-phenyl}-acetic acid; {3-[1-(2,4-Dichloro-5-fluoro-benzyl)-3-(4-fluoro-butyl)-2,4-dioxo-1.3,8-triaza-spiro[4.5]dec-8-yl]-phenyl}-acetic acid; (3-{3-Cyclobutylmethyl-1-[2-(2,4-dichloro-phenyl)-ethyl]-2,4-dioxo-1,3,5-triaza-spiro[4.5]dec-8-yl}-phenyl)-acetic acid; (3-{3-Cyclopentylmethyl-2,4-dioxo-1-[2-(4-trifluoromethyl-phenyl)-ethyl]-1,3,8-triaza-spiro[4.5]dea-8-yl}-phenyl)-acetic acid; (3-{3-Cyclopentylmethyl-1-[2-(2,4-dichloro-phenyl)-ethyl]-2,4-dioxo-1,3,8-triaza-spiro[4.5]dec-8-yl)-phenyl)-acetic acid; (3-{3-cyclohexylmethyl-1-[2-(2,4-dichloro-phenyl)-ethyl]-2,4-dioxo-1,3,8-triaza-spiro[4.5]dec-8-yl}-phenyl)-acetic acid; and (3-{1-[2-(4-chloro-phenyl)-ethyl]-3-cyclopentylmethyl-2,4- dioxo-1,3,8-triaza-spiro[4.5]dec-8-yl}-phenyl)-acetic acid.

6. A compound any one of claims 1 to 5 for use in a method of treating or preventing dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, cachexia, inflammation, arthritis, cancer, anorexia, anorexia nervosa, bulimia, Alzheimer's disease, skin disorders, respiratory diseases, ophthalmic disorders, irritable bowel diseases, ulcerative colitis, Crohn's disease, type-1 diabetes, type-2 diabetes or Syndrome X.

7. A compound any one of claims 1 to 5 for use in a method of treating or preventing HIV wasting syndrome, long term critical illness, decreased muscle mass and/or muscle strength, decreased lean body mass, maintenance of muscle strength and function in the elderly, diminished muscle endurance and muscle function, or frailty in the elderly.

8. Use of a compound according to any of claims 1 to 5 in the manufacture of a medicament for the treatment or prevention of dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, cachexia, inflammation, arthritis, cancer, anorexia, anorexia nervosa, bulimia, Alzheimer's disease, skin disorders, respiratory diseases, ophthalmic disorders, irritable bowel diseases, ulcerative colitis, Crohn's disease, type-1 diabetes, type-2 diabetes and Syndrome X.

9. Use of a compound according to any of claims 1 to 5 in the manufacture of a medicament for the treatment or prevention of HIV wasting syndrome, long term critical illness, decreased muscle mass and/or muscle strength, decreased lean body mass, maintenance of muscle strength and function in the elderly, diminished muscle endurance and muscle function, and frailty in the elderly.

10. A pharmaceutical composition comprising a therapeutically effective amount of a compound of any of claim 1 to 5 in combination with one or more pharmaceutically acceptable excipients.

11. A pharmaceutical combination, especially a pharmaceutical composition, comprising 1) a compound of any of claims 1 to 5 or a pharmaceutical acceptable salt thereof; and 2) at least one active ingredient selected from:
a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Bxendin-4 and GLP-1 mimetics; dipeptidyl peptidase IV inhibitors such as DPP728, vildagliptin, MK-0431, saxagliptin, GSK23A ; an AGE breaker; a thiazolidone derivative (glitazone) such as pioglitazone, resiglitazone, or (R)-1-{4-[5-methyl-2-(4-trifluoromethylphenyl)-oxazol-4-ylmethoxy]-benzenesulfonyl}-2,3-dihydro-1H-indole-2-carboxylic acid, a non-glitazone type PPARγ agonist e.g. GI-262570;
b) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin;
c) an anti-obesity agent or appetite regulating agent such as phentermine, leptin, bromocriptine, dexamphetamine, amphetamine, fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;
d) anti-hypertensive agents, e.g., loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thioxphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;
e) a HDL increasing compound;
f) a cholesterol absorption modulator such as Zetia® and KT6-971;
g) Apo-Al analogues and mimetics;
h) thrombin inhibitors such as Ximelagatran;
i) aldosterone inhibitors such as anastrazole, fadraxole, eplerenone;
j) Inhibitors of platelet aggregation such as aspirin, clopidogrel bisulfate;
k) estrogen, testosterone, a selective estrogen receptor modulator a selective androgen receptor modulator;
l) a chemotherapeutic agent such as aromatase inhibitors e.g. femara, anti-estrogens, topoisomerase I inhibitors, topoisomerase II inhibitors, microtubule active agents, alkylating agents, antineoplastic antimetabolites, platin compounds, compounds decreasing the protein kinase activity such as a PDGF receptor tyrosine kinase inhibitor preferably Imatinib or 4-Methyl-N-[3-(4-methyl-imidaxol-1-yl)-5-trifluoromethyl-phenyl]-3-(4-pyridin-3-yl-pyrimidin-2-ylamino)-benzamide; and
m) an agent interacting with a 5-HT₃ receptor and/or an agent interacting with 5-HT₄ receptor such as tegaserod, tegaserod hydrogen maleate, cisapride, cilansetron;
or, in each case a pharmaceutically acceptable salt thereof; and optionally a pharmaceutically acceptable carrier.

12. A pharmaceutical composition according to claim 10 or a combination according to claim 11, for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

13. A compound according to any of claims 1 to 5, or a pharmaceutical composition according to claim 10 or a combination according to claim 11, for use as a medicament.

14. Use of a compound according to any of claims 1 to 5, or a pharmaceutical composition according to claim 10 or a combination according to claim 11, for the manufacture of a medicament for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

## Patentansprüche

1. Verbindung, die aus Ia, Ib und Ic ausgewählt ist: 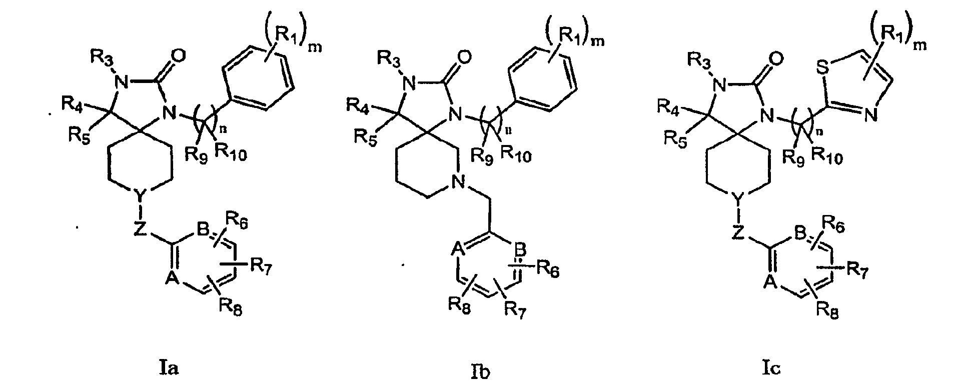 worin:
n aus 1 und 2 ausgewählt ist;
m aus 1, 2, 3, 4 und 5 ausgewählt ist;
die R₁ jeweils unabhängig voneinander aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt sind;
R₃ aus C₁₋₈-Alkyl, C₂₋₈-Alkenyl, halogensubstituiertem C₁₋₆-Alkyl, halogensubstituiertem C₂₋₆-Alkenyl, C₅₋₁₀-Heteroaryl-C₀₋₄-alkyl und C₃₋₁₂-Cycloalkyl-C₀₋₄-alkyl ausgewählt ist;
R₂ aus Wasserstoff und C₁₋₆-Alkyl ausgewählt ist;
R₄ aus Wasserstoff und C₁₋₆-Alkyl ausgewählt ist;
R₅ aus Wasserstoff und C₁₋₆-Alkyl ausgewählt ist; oder
R₄ und R₅ gemeinsam mit dem Kohlenstoffatom, an das sie beide gebunden sind, Carbonyl bilden;
Y N ist;
Z aus einer Bindung, -S(O)₀₋₂- und -CR₁₁R₁₂- ausgewählt ist, worin R₁₁ und R₁₂ unabhängig voneinander aus Wasserstoff und C₁₋₆-Alkyl ausgewählt sind;
A und B unabhängig voneinander aus CH und N ausgewählt sind;
R₆ und R₇ unabhängig voneinander aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt sind;
R₈ aus -X₂CO₂R₁₃, -X₂CR₁₄R₁₅X₃CO₂R₁₃, -X₂SCR₁₄R₁₅X₃CO₂R₁₃ und -X₂OCR₁₄R₁₅X₃CO₂R₁₃ ausgewählt ist, worin X₂ und X₃ unabhängig voneinander aus einer Bindung und C₁₋₄-Alkylen ausgewählt sind, R₁₄ und R₁₅ unabhängig voneinander aus Wasserstoff, C₁₋₄-Alkyl und C₁₋₄-Alkoxy ausgewählt sind oder R₁₄ und R₁₅ gemeinsam mit dem Kohlenstoffatom, an das sie beide gebunden sind, C₃₋₁₂-Cycloalkyl bilden, und R₁₃ aus Wasserstoff und C₁₋₆-Alkyl ausgewählt ist;
R₉ und R₁₀ unabhängig voneinander aus Wasserstoff, C₁₋₆-Alkyl und -OR₁₆ ausgewählt sind, worin R₁₆ aus Wasserstoff und C₁₋₆-Alkyl ausgewählt ist;
sowie pharmazeutisch annehmbare Salze, Hydrate, Solvate und Isomere davon.

2. Verbindung nach Anspruch 1, worin
n aus 1 und 2 ausgewählt ist;
m aus 1, 2 und 3 ausgewählt ist;
die R₁ jeweils unabhängig voneinander aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt sind;
R₃ aus C₁₋₈-Alkyl, C₂₋₈-Alkenyl, halogensubstituiertem C₁₋₆-Alkyl, halogensubstituiertem C₂₋₆-Alkenyl, C₅₋₁₀-Heteroaryl-C₀₋₄-alkyl und C₃₋₁₂-Cycloalkyl-C₀₋₄-alkyl ausgewählt ist;
R₂ aus Wasserstoff und C₁₋₆-Alkyl ausgewählt ist;
R₄ aus Wasserstoff und C₁₋₆-Alkyl ausgewählt ist;
R₅ aus Wasserstoff und C₁₋₆-Alkyl ausgewählt ist; oder
R₄ und R₅ gemeinsam mit dem Kohlenstoffatom, and das sie beide gebunden sind, Carbonyl bilden;
Y N ist;
Z aus einer Bindung, -S(O)₀₋₂- und -CR₁₁R₁₂- ausgewählt ist, worin R₁₁ und R₁₂ unabhängig voneinander aus Wasserstoff und C₁₋₆-Alkyl ausgewählt sind;
A und B unabhängig voneinander aus CH und N ausgewählt sind;
R₆ und R₇ unabhängig voneinander aus Wasserstoff, Halogen, C₁₋₆-Alkyl, halogensubstituiertem C₁₋₆-Alkyl und C₁₋₆-Alkoxy ausgewählt sind;
R₈ aus -X₂CO₂R₁₃, -X₂CR₁₄R₁₅X₃CO₂R₁₃ und -X₂OCR₁₄R₁₅X₃CO₂R₁₃ ausgewählt ist, worin X₂ und X₃ unabhängig voneinander aus einer Bindung und C₁₋₄-Alkylen ausgewählt sind, R₁₄ und R₁₅ unabhängig voneinander aus Wasserstoff und C₁₋₄-Alkyl ausgewählt sind, und R₁₃ aus Wasserstoff und C₁₋₆-Alkyl ausgewählt ist; und
R₉ und R₁₀ unabhängig voneinander aus Wasserstoff, C₁₋₆-Alkyl und -OR₁₆ ausgewählt sind, worin R₁₆ aus Wasserstoff und C₁₋₆-Alkyl ausgewählt ist.

3. Verbindung nach Anspruch 2, worin die R₁ unabhängig voneinander aus Wasserstoff, Halogen Methoxy, Trifluormethoxy und Trifluormethyl ausgewählt sind; R₃ aus Isobutyl, Cyclopropylmethyl, Cyclobutylmethyl, Isopentyl, Butyl, Cyclopentyl-methyl, 3-Methylbut-2-enyl, Pentyl, 2,2-Dimethylpropyl, 4-Fluorbutyl, 2-Ethylbutyl, 2-Methylpentyl, Cyclohexylmethyl, 3,3-Dimethyl-2-oxo-butyl, Pyrrolylpropyl, 3-Trifluormethylpropyl, Cyclohexylethyl, 2-Ethylhexyl, 2-Methylbutyl, 3,4,4-Trifluorbut-3-enyl und 3,3-Dimethylbutyl ausgewählt ist; R₄ und R₅ jeweils Wasserstoff sind oder gemeinsam mit dem Kohlenstoffatom, an das sie beide gebunden sind, Carbonyl bilden; und Z aus einer Bindung, -S(O)₂- und -CH₂- ausgewählt ist.

4. Verbindung nach Anspruch 3, worin R₈ aus -CH₂C(O)OH, -CH(CH₂)C(O)OH, -OC(CH₂)₂C(O)OH, -(CH₂)₂C(O)OH und -OCH₂C(O)OH ausgewählt ist und R₉ und R₁₀ unabhängig voneinander aus Wasserstoff, Halogen, Methyl, Methoxy und Trifluormethyl ausgewählt sind.

5. Verbindung nach Anspruch 1, die aus folgenden ausgewählt ist:
(3-{3-Isobutyl-1-[2-(4-methoxyphenyl)ethyl]-2,4-dioxo-1,3,8-triaza-spiro[4.5]dec-8-yl-methyl}phenyl)essigsäure;
(3-{3-Isobutyl-1-[2-(4-methoxyphenyl)ethyl]-2,4-dioxo-1,3,8-triaza-spiro[4.5]decan-8-sulfonyl}-4-methylphenyl)essigsäure;
(3-{3-Isobutyl-1-[2-(4-methoxyphenyl)ethyl]-2,4-dioxo-1,3,8-triaza-spiro[4.5]dec-8-yl}-phenyl)essigsäure;
2-(4-{3-Isobutyl-1-[2-(4-methoxyphenyl)ethyl]-2,4-dioxo-1,3-diaza-spiro[4.5]dec-7-yl-methyl}phenyl)propionsäure;
(3-{3-Cyclopropylmethyl-1-[2-(4-methoxyphenyl)ethyl]-2,4-dioxo-1,3,8-triaza-spiro-[4.5]dec-8-yl}phenyl)essigsäure;
{3-[3-Isobutyl-2,4-dioxo-1-(4-trifluormethoxybenzyl)-1,3,8-triaza-spiro[4.5]dec-8-yl]-phenyl}essigsäure;
2-(2-{3-Isobutyl-1-[2-(4-methoxyphenyl)ethyl]-2,4-dioxo-1,3,8-triaza-spiro[4.5]dec-8-yl}pyrimidin-4-yloxy)-2-methylpropionsäure;
2-(3-{3-Cyclobutylmethyl-1-[2-(2,4-dichlorphenyl)ethyl]-2,4-dioxo-1,3-diaza-spiro[4.5]-dec-8-yl}phenoxy)-2-methylpropionsäure;
{3-[3-Cyclopropylmethyl-2,4-dioxo-1-(4-trifluormethoxybenzyl)-1,3,8-triaza-spiro[4.5]-dec-8-yl]phenyl}essigsäure;
(3-{3-Cyclobutylmethyl-2,4-dioxo-1-[2-(4-trifluormethylphenyl)ethyl]-1,3,8-triaza-spiro-[4.5]dec-8-yl}phenyl)essigsäure;
(3-{3-Cyclobutylmethyl-1-[4-(4-methoxyphenyl)butyl]-2,4-dioxo-1,3,8-triaza-spiro[4.5]-dec-8-yl}phenyl)essigsäure;
3-{3-Isobutyl-1-[2-(4-methoxyphenyl)ethyl]-2,4-dioxo-1,3,8-triaza-spiro[4.5]dec-8-yl-methyl}benzoesäure;
(2-{3-Isobutyl-1-[2-(4-methoxyphenyl)ethyl]-2,4-dioxo-1,3,8-triaza-spiro[4.5]dec-8-yl-methyl}phenyl)essigsäure;
(3-{3-Isobutyl-1-[2-(4-methoxyphenyl)ethyl]-2,4-dioxo-1,3,8-triaza-spiro[4.5]decan-8-sulfonyl}-4-methoxyphenyl)essigsäure;
3-(3-{3-Isobutyl-1-[2-(4-methoxyphenyl)ethyl]-2,4-dioxo-1,3,8-triaza-spiro[4.5]dec-8-yl}phenyl)propionsäure;
(3-{3-Isobutyl-1-[2-(4-methoxyphenyl)ethyl]-2,4-dioxo-1,3,8-triaza-spiro[4.5]dec-8-yl}-phenoxy)essigsäure;
2-(3-{3-Isobutyl-1-[2-(4-methoxyphenyl)ethyl]-2,4-dioxo-1,3,8-triaza-spiro[4.5]dec-8-yl}phenoxy)-2-methylpropionsäure;
(5-{3-Isobutyl-1-[2-(4-methoxyphenyl)ethyl]-2,4-dioxo-1,3,8-triaza-spiro[4.5]dec-8-yl}-2-methylphenyl)essigsäure;
(3-{3-Isobutyl-1-[2-(4-methoxyphenyl)ethyl]-2,4-dioxo-1,3,8-triaza-spiro[4.5]dec-8-yl}-5-methylphenyl)essigsäure;
(2-Fluor-5-{3-isobutyl-1-[2-(4-methoxyphenyl)ethyl]-2,4-dioxo-1,3,8-triaza-spiro[4.5]-dec-8-yl}phenyl)essigsäure;
(5-{3-Isobutyl-1-[2-(4-methoxyphenyl)ethyl]-2,4-dioxo-1,3,8-triaza-spiro[4.5]dec-8-yl}-2-trifluormethylphenyl)essigsäure;
(5-{3-Isobutyl-1-[2-(4-methoxyphenyl)ethyl]-2,4-dioxo-1,3,8-triaza-spiro[4.5]dec-8-yl}-2-methoxyphenyl)essigsäure;
2-(3-{3-Isobutyl-1-[2-(4-methoxyphenyl)ethyl]-2,4-dioxo-1,3,8-triaza-spiro[4.5]dec-8-yl}phenyl)-2-methylpropionsäure;
(5-{3-Isobutyl-1-[2-(4-methoxyphenyl)ethyl]-2,4-dioxo-1,3,8-triaza-spiro[4.5]dec-8-yl}-2-methylphenoxy)essigsäure;
(2-Chlor-5-{3-isobutyl-1-[2-(4-methoxyphenyl)ethyl]-2,4-dioxo-1,3,8-triaza-spiro[4.5]-dec-8-yl}phenoxy)essigsäure;
2-(5-{3-Isobutyl-1-[2-(4-methoxyphenyl)ethyl]-2,4-dioxo-1,3,8-triaza-spiro[4.5]dec-8-yl}-2-methylphenoxy)-2-methylpropionsäure;
2-(2-Chlor-5-{3-isobutyl-1-[2-(4-methoxyphenyl)ethyl]-2,4-dioxo-1,3,8-triaza-spiro-[4.5]dec-8-yl}phenoxy)-2-methylpropionsäure;
2-(2,3-Difluor-5-{3-isobutyl-1-[2-(4-methoxyphenyl)ethyl]-2,4-dioxo-1,3,8-triaza-spiro-[4.5]dec-8-yl}phenoxy)-2-methylpropionsäure;
(3-{3-Isobutyl-1-[2-(4-methoxyphenyl)ethyl]-2-oxo-1,3,8-triaza-spiro[4.5]dec-8-yl}-phenyl)essigsäure;
(6-{3-Isobutyl-1-[2-(4-methoxyphenyl)ethyl]-2,4-dioxo-1,3,8-triaza-spiro[4.5]dec-8-yl}-pyridin-2-yl)essigsäure;
(2-{3-Isobutyl-1-[2-(4-methoxyphenyl)ethyl]-2,4-dioxo-1,3,8-triaza-spiro[4.5]dec-8-yl}-pyridin-4-yl)essigsäure;
(5-{3-Cyclobutylmethyl-1-[2-(2,4-dichlorphenyl)ethyl]-2,4-dioxo-1,3,8-triaza-spiro[4.5]-dec-8-yl}-2-methylphenyl)essigsäure;
2-(5-(3-Cyclobutylmethyl-1-[2-(2,4-dichlorphenyl)ethyl]-2,4-dioxo-1,3,8-triaza-spiro-[4.5]dec-8-yl}-2-methylphenoxy)-2-methylpropionsäure;
(2-Chlor-5-{3-cyclobutylmethyl-1-[2-(2,4-dichlorphenyl)ethyl]-2,4-dioxo-1,3,8-triaza-spiro[4.5]dec-8-yl}phenoxy)essigsäure;
2-(2-Chlor-5-{3-cyclobutylmethyl-1-[2-(2,4-dichlorphenyl)ethyl]-2,4-dioxo-1,3,8-triaza-spiro[4.5]dec-8-yl}phenoxy)-2-methylpropionsäure;
(6-{3-Cyclobutylmethyl-1-[2-(2,4-dichlorphenyl)ethyl]-2,4-dioxo-1,3,8-triaza-spiro[4.5]-dec-8-yl}pyridin-2-yl)essigsäure;
(4-{3-Isobutyl-1-[2-(4-methoxyphenyl)ethyl]-2,4-dioxo-1,3,7-triaza-spiro[4.5]dec-7-yl-methyl}phenoxy)essigsäure;
(3-{3-Cyclobutylmethyl-1-[2-(4-methoxyphenyl)ethyl]-2,4-dioxo-1,3,8-triaza-spiro[4.5]-dec-8-yl}phenyl)essigsäure;
{3-[1-[2-(4-Methoxyphenyl)ethyl]-3-(3-methylbutyl)-2,4-dioxo-1,3,8-triaza-spiro[4.5]-dec-8-yl]phenyl}essigsäure;
(3-{3-Butyl-1-[2-(4-methoxyphenyl)ethyl]-2,4-dioxo-1,3,8-triaza-spiro[4.5]dec-8-yl}-phenyl)essigsäure;
2-(4-{3-Isobutyl-1-[2-(4-methoxyphenyl)ethyl]-2,4-dioxo-1,3,8-triaza-spiro[4.5]dec-8-yl}pyrimidin-2-yloxy)-2-methylpropionsäure;
2-(6-{3-Isobutyl-1-[2-(4-methoxyphenyl)ethyl]-2,4-dioxo-1,3,8-triaza-spiro[4.5]dec-8-yl}pyrimidin-4-yloxy)-2-methylpropionsäure;
2-(4-{3-Cyclobutylmethyl-1-[2-(2,4-dichlorphenyl)ethyl]-2,4-dioxo-1,3,8-triaza-spiro-[4.5]dec-8-yl}pyrimidin-2-yloxy)-2-methylpropionsäure;
2-(2-{3-Cyclobutylmethyl-1-[2-(2,4-dichlorphenyl)ethyl]-2,4-dioxo-1,3,8-triaza-spiro-[4.5]dec-8-yl}pyrimidin-4-yloxy)-2-methylpropionsäure;
2-(6-{3-Cyclobutylmethyl-1-[2-(2,4-dichlorphenyl)ethyl]-2,4-dioxo-1,3,8-triaza-spiro-[4.5]dec-8-yl}pyrimidin-4-yloxy)-2-methylpropionsäure;
{3-[3-Cyclobutylmethyl-2,4-dioxo-1-(4-trifluormethoxybenzyl)-1,3,8-triaza-spiro[4.5]-dec-8-yl]phenyl}essigsäure;
{3-[3-Cyclobutylmethyl-2,4-dioxo-1-(4-trifluormethylbenzyl)-1,3,8-triaza-spiro[4.5]dec-8-yl]phenyl}essigsäure;
{3-[3-Cyclopentylmethyl-2,4-dioxo-1-(4-trifluormethylbenzyl)-1,3,8-triaza-spiro[4.5]-dec-8-yl]phenyl}essigsäure;
{3-[3-Cyclopentylmethyl-2,4-dioxo-1-(4-trifluormethoxybenzyl)-1,3,8-triaza-spiro[4.5]-dec-8-yl]phenyl}essigsäure;
{3-[1-(2,4-Bistrifluormethylbenzyl)-3-cyclopentylmethyl-2,4-dioxo-1,3,8-triaza-spiro-[4.5]dec-8-yl]phenyl}essigsäure;
{3-[3-Cyclobutylmethyl-2,4-dioxo-1-(3-trifluormethoxybenzyl)-1,3,8-triaza-spiro[4.5]-dec-8-yl]phenyl}essigsäure;
(3-{3-Cyclobutylmethyl-2,4-dioxo-1-[4-(4-trifluormethylphenyl)thiazol-2-ylmethyl]-1,3,8-triaza-spiro[4.5]dec-8-yl}phenyl)essigsäure;
(3-{3-Cyclopropylmethyl-2,4-dioxo-1-[4-(4-trifluormethylphenyl)thiazol-2-ylmethyl]-1,3,8-triaza-spiro[4.5]dec-8-yl}phenyl)essigsäure;
{3-[3-(3-Methylbut-2-enyl)-2,4-dioxo-1-(4-trifluormethoxybenzyl)-1,3,8-triaza-spiro-[4.5]dec-8-yl]phenyl}essigsäure;
{3-[1-[2-(4-Bromphenyl)-2-hydroxyethyl]-3-(3-methylbutyl)-2,4-dioxo-1,3,8-triaza-spiro[4.5]dec-8-yl]phenyl}essigsäure;
{3-[1-[2-(4-Chlorphenyl)-2-hydroxyethyl]-3-(3-methylbutyl)-2,4-dioxo-1,3,8-triaza-spiro[4.5]dec-8-yl]phenyl}essigsäure;
{3-[2,4-Dioxo-3-pentyl-1-(4-trifluormethoxybenzyl)-1,3,8-triaza-spiro[4.5]dec-8-yl]-phenyl}essigsäure;
{3-[3-(2,2-Dimethylpropyl)-2,4-dioxo-1-(4-trifluormethoxybenzyl)-1,3,8-triaza-spiro-[4.5]dec-8-yl]phenyl}essigsäure;
{3-[3-(2-Ethylbutyl)-2,4-dioxo-1-(4-trifluormethoxybenzyl)-1,3,8-triaza-spiro[4.5]dec-8-yl]phenyl}essigsäure;
{3-[3-(4-Fluorbutyl)-2,4-dioxo-1-(4-trifluormethoxybenzyl)-1,3,8-triaza-spiro[4.5]dec-8-yl]phenyl}essigsäure;
{3-[3-(4-Methylpentyl)-2,4-dioxo-1-(4-trifluormethoxybenzyl)-1,3,8-triaza-spiro[4.5]-dec-8-yl]phenyl}essigsäure;
{3-[3-Cyclohexylmethyl-2,4-dioxo-1-(4-trifluormethoxybenzyl)-1,3,8-triaza-spiro[4.5]-dec-8-yl]phenyl}essigsäure;
{3-[2,4-Dioxo-3-(3-pyrrol-1-yl-propyl)-1-(4-trifluormethoxybenzyl)-1,3,8-triaza-spiro-[4.5]dec-8-yl]phenyl}essigsäure;
{3-[3-(3,3-Dimethyl-2-oxo-butyl)-2,4-dioxo-1-(4-trifluormethoxybenzyl)-1,3,8-triaza-spiro[4.5]dec-8-yl]phenyl}essigsäure;
{3-[2,4-Dioxo-3-(4,4,4-trifluorbutyl)-1-(4-trifluormethoxybenzyl)-1,3,8-triaza-spiro[4.5]-dec-8-yl]phenyl}essigsäure;
{3-[3-(2-Cyclohexylethyl)-2,4-dioxo-1-(4-trifluormethoxybenzyl)-1,3,8-triaza-spiro[4.5]-dec-8-yl]phenyl}essigsäure;
{3-[3-(2-Ethylhexyl)-2,4-dioxo-1-(4-trifluormethoxybenzyl)-1,3,8-triaza-spiro[4.5]dec-8-yl]phenyl}essigsäure;
{3-[3-(2-Methylbutyl)-2,4-dioxo-1-(4-trifluormethoxybenzyl)-1,3,8-triaza-spiro[4.5]dec-8-yl]phenyl}essigsäure;
{3-[2,4-Dioxo-3-(3,4,4-trifluorbut-3-enyl)-1-(4-trifluormethoxybenzyl)-1,3,8-triaza-spiro[4.5]dec-8-yl]phenyl}essigsäure;
{3-[3-(3,3-Dimethylbutyl)-2,4-dioxo-1-(4-trifluormethoxybenzyl)-1,3,8-triaza-spiro[4.5]-dec-8-yl]phenyl}essigsäure;
{3-[1-(2,4-Dichlor-5-fluorbenzyl)-3-(3,3-dimethylbutyl)-2,4-dioxo-1,3,8-triaza-spiro-[4.5]dec-8-yl]phenyl}essigsäure;
{3-[1-(2,4-Dichlor-5-fluorbenzyl)-3-(4-fluorbutyl)-2,4-dioxo-1,3,8-triaza-spiro[4.5]dec-8-yl]phenyl}essigsäure;
(3-{3-Cyclobutylmethyl-1-[2-(2,4-dichlorphenyl)ethyl]-2,4-dioxo-1,3,8-triaza-spiro[4.5]-dec-8-yl}phenyl)essigsäure;
(3-{3-Cyclopentylmethyl-2,4-dioxo-1-[2-(4-trifluormethylphenyl)ethyl]-1,3,8-triaza-spiro[4.5]dec-8-yl}phenyl)essigsäure;
(3-{3-Cyclopentylmethyl-1-[2-(2,4-dichlorphenyl)ethyl]-2,4-dioxo-1,3,8-triaza-spiro-[4.5]dec-8-yl}phenyl)essigsäure;
(3-{3-Cyclohexylmethyl-1-[2-(2,4-dichlorphenyl)ethyl]-2,4-dioxo-1,3,8-triaza-spiro-[4.5]dec-8-yl}phenyl)essigsäure und
(3-{1-[2-(4-Chlorphenyl)ethyl]-3-cyclopentylmethyl-2,4-dioxo-1,3,8-triaza-spiro[4.5]-dec-8-yl}phenyl)essigsäure.

6. Verbindung nach einem der Ansprüche 1 bis 5 zur Verwendung in einem Verfahren zur Behandlung oder Prävention von Dyslipidämie, Hyperlipidämie, Hypercholesterinämie, Atherosklerose, Atherogenese, Hypertriglyceridämie, Herzversargen, Herzinfarkt, Gefäßerkrankungen, Herzkreislauferkrankungen, Hypertonie, Adipositas, Kachexie, Entzündungen, Arthritis, Krebs, Anorexie, Anorexia nervosa, Bulimie, Alzheimer-Krankheit, Hauterkrankungen, Atemwegserkrankungen, Augenerkrankungen, Reizdarmsyndrom, Colitis ulcerosa, Morbus Crohn, Typ-1-Diabetes, Typ-2-Diabetes oder Syndrom X.

7. Verbindung nach einem der Ansprüche 1 bis 5 zur Verwendung in einem Verfahren zur Behandlung oder Prävention von HIV-Kachexiesyndrom, kritischen Langzeiterkrankungen, reduzierter Muskelmasse und/oder Muskelkraft, reduzierter fettfreier Körpermasse, zur Aufrechterhaltung der Muskelkraft und -funktion bei älteren Patienten, zur Behandlung und Prävention reduzierter Muskelleistung und Muskelfunktion oder von Gebrechlichkeit bei älteren Patienten.

8. Verwendung einer Verbindung nach einem der Ansprüche 1 bis 5 zur Verwendung in einem Verfahren zur Behandlung oder Prävention von Dyslipidämie, Hyperlipidämie, Hypercholesterinämie, Atherosklerose, Atherogenese, Hypertriglyceridämie, Herzversargen, Herzinfarkt, Gefäßerkrankungen, Herzkreislauferkrankungen, Hypertonie, Adipositas, Kachexie, Entzündungen, Arthritis, Krebs, Anorexie, Anorexia nervosa, Bulimie, Alzheimer-Krankheit, Hauterkrankungen, Atemwegserkrankungen, Augenerkrankungen, Reizdarmsyndrom, Colitis ulcerosa, Morbus Crohn, Typ-1-Diabetes, Typ-2-Diabetes oder Syndrom X.

9. Verwendung einer Verbindung nach einem der Ansprüche 1 bis 5 zur Verwendung in einem Verfahren zur Behandlung oder Prävention von HIV-Kachexiesyndrom, kritischen Langzeiterkrankungen, reduzierter Muskelmasse und/oder Muskelkraft, reduzierter fettfreier Körpermasse, zur Aufrechterhaltung der Muskelkraft und -funktion bei älteren Patienten, zur Behandlung und Prävention reduzierter Muskelleistung und Muskelfunktion oder von Gebrechlichkeit bei älteren Patienten.

10. Pharmazeutische Zusammensetzung, die eine therapeutisch wirksame Menge einer Verbindung nach einem der Ansprüche 1 bis 5 in Kombination mit einem oder mehreren pharmazeutisch annehmbaren Exzipienten umfasst.

11. Pharmazeutische Kombination, insbesondere eine pharmazeutische Zusammensetzung, die Folgendes umfasst:
1) eine Verbindung nach einem der Ansprüche 1 bis 5 oder ein pharmazeutisch annehmbares Salz davon und
2) zumindest einen Wirkstoff, der aus folgenden ausgewählt ist:
a) Anti-Diabetes-Mittel, wie z.B. Insulin, Insulinderivate und -mimetika; Insulinsekretagoga, wie z.B. Sulfonylharnstoffe, z.B. Glipizid, Glyburid und Amaryl; insulinotrope Sulfonylharnstoffrezeptorliganden, wie z.B. Meglitinide, z.B., Nateglinid und Repaglinid; Insulinsensibilisatoren, wie z.B. Proteintyrosinphosphatase-1B- (PTP-1B-) Hemmer, wie z.B. PTP-112; GSK3- (Glykogensynthasekinase-3-) Hemmer, wie z.B. SB-517955, SB-4195052, SB-216763, NN-57-05441 und NN-57-05445; RXR-Liganden, wie z.B. GW-0791 und AGN-194204; natriumabhängige Glucose-Co-Transporter-Hemmer, wie z.B. T-1095; Glykogenphosphorylase-A-Hemmer, wie z.B. BAY R3401; Biguanide, wie z.B. Metformin; α-Glucosidase-Hemmer, wie z.B. Acarbos; GLP-1 (glucagonähnliches Peptid-1), GLP-1-Analoga, wie z.B. Exendin-4 und GLP-1-Mimetika; Dipeptidylpeptidase-IV-Hemmer, wie z.B. DPP728, Vildagliptin, MK-0431, Saxagliptin, GSK23A; einen AGE-Spalter (???); ein Thiazolidonderivat (Glitazon), wie z.B. Pioglitazon, Rosiglitazon oder (R)-1-{4-[5-Methyl-2-(4-trifluormethylphenyl)oxazol-4-ylmethoxy]benzolsulfonyl}-2,3-dihydro-1H-indol-2-carbonsäure, ein PPARγ-Agonist vom Nicht-Glitazon-Typ, wie z.B. GI-262570;
b) Hypolipidämiemittel, wie z.B. 3-Hydroxy-3-methylglutaryl-Coenzy-A- (HMG-CoA-) Reductasehemmer, wie z.B., Lovastatin, Pitavastatin, Simvastatin, Pravastatin, Cerivastatin, Mevastatin, Velostatin, Fluvastatin, Dalvastatin, Atorvastatin, Rosuvastatin und Rivastatin; Squalensynthase-Hemmers; FXR- (Farnesoid-X-Rezeptor-) und LXR-(Leber-X-Rezeptor-) Liganden; Cholestyramin; Fibrate; Nikotinsäure und Aspirin;
c) ein Anti-Adipositas-Mittel oder Appetitzügler, wie z.B. Phentermine, Leptin, Bromocriptin, Dexamphetamin, Amphetamin, Fenfluramin, Dexfenfluramin, Sibutramin, Orlistat, Dexfenfluramin, Mazindol, Phentermin, Phendimetrazin, Diethylpropion, Fluoxetin, Bupropion, Topiramat,Diethylpropion, Benzphetamin, Phenylpropanolamin oder Ecopipam, Ephedrin, Pseudoephedrin oder Cannabinoidrezeptorantagonisten;
d) Anti-Hypertoniemittel, z.B. Schleifendiuretika, wie z.B. Etacrynsäure, Furosemid und Torsemid; Diuretika, wie z.B. Thiazidderivae, Chlorothiazid, Hydrochlorothiazid, Amilorid; Hemmer des Angiotensin-konvertierenden Enzyms (ACE-Hemmer), wie z.B. Benazepril, Captopril, Enalapril, Fosinopril, Lisinopril, Moexipril, Perinodopril, Quinapril, Ramipril und Trandolapril; Hemmer der Na-K-ATPase-Membranpumpe, wie z.B. Digoxin; Neutralendopeptidase- (NEP-) Hemmer, wie z.B. Thiorphan, Terteothiorphan, SQ29072; ECE-Hemmer, wie z.B. SLV306; ACE/NEP-Hemmer, wie z.B. Omapatrilat, Sampatrilat und Fasidotril; Angiotensin-II-Antagonisten, wie z.B. Candesartan, Eprosartan, Irbesartan, Losartan, Telmisartan und Valsartan, insbesondere Valsartan; Renin-Hemmer, wie z.B. Aliskiren, Terlakiren, Ditekiren, RO 66-1132, RO-66-1168; Blocker β-adrenerger Rezeptoren, wie z.B. Acebutolol, Atenolol, Betaxolol, Bisoprolol, Metoprolol, Nadolol, Propranolol, Sotalol und Timolol; inotrope Mittel, wie z.B. Digoxin, Dobutamin und Milrinon; Calciumkanalblocker, wie z.B. Amlodipin, Bepridil, Diltiazem, Felodipin, Nicardipin, Nimodipin, Nifedipin, Nisoldipin und Verapamil; Aldosteronrezeptorantagonisten und Aldosteronsynthase-Hemmer;
e) eine HDL-steigernde Verbindung;
f) einen Cholesterinabsorptionsmodulator, wie z.B. Zetia^{®} und KT6-971;
g) Apo-A1-Analoga und -Mimetika;
h) Thrombinhemmer, wie z.B. Ximelagatran;
i) Aldosteronhemmer, wie z.B. Anastrazol, Fadrazol, Eplerenon;
j) Plättchenaggregationshemmer, wie z.B. Aspirin, Clopidogrelbisulfat;
k) Östrogen, Testosteron, einen selektiven Östrogenrezeptormodulator, einen selektiven Androgenrezeptormodulator;
l) ein chemotherapeutisches Mittel, wie z.B. Aromatase-Hemmer, wie z.B. Femara, Anti-Östrogene, Topoisomerase-Hemmer, Topoisomerase-I-Hemmer, Microtubuliaktive Mittel, Alkylierungsmittel, antineoplastische Antimetaboliten, Platinverbindungen, Verbindungen, die die Proteinkinaseaktivität senken, wie z.B. ein PDGF-Rezeptortyrosinkinasehemmer, vorzugsweise Imatinib oder 4-Methyl-N-[3-(4-methylimidazol-1-yl)-5-trifluormethylphenyl]-3-(4-pyridin-3-yl-pyrimidin-2-ylamino)benzamid; und
m) ein Mittel, das mit einem 5-HT₃-Rezeptor wechselwirkt, und/oder ein Mittel, das mit einem 5-HT₄-Rezeptor wechselwirkt, wie z.B. Tegaserod, Tegaserodhydrogenmaleat, Cisaprid, Cilansetron;
oder jeweils ein pharmazeutisch annehmbares Salz davon;
und gegebenenfalls einen pharmazeutisch annehmbaren Träger.

12. Pharmazeutische Zusammensetzung nach Anspruch 10 oder Kombination nach Anspruch 11 zur Behandlung oder Prävention von Dyslipidämie, Hyperlipidämie, Hypercholesterinämie, Atherosklerose, Hypertriglyceridämie, Herzversargen, Herzinfarkt, Gefäßerkrankungen, Herzkreislauferkrankungen, Hypertonie, Adipositas, Entzündungen, Arthritis, Krebs, Alzheimer-Krankheit, Hauterkrankungen, Atemwegserkrankungen, Augenerkrankungen, Reizdarmsyndrom, Colitis ulcerosa, Morbus Crohn, Erkrankungen mit beeinträchtigter Glucosetoleranz, einschließlich Hyperglykämie und Insulinresistenz, wie z.B. Typ-1-Diabetes, Typ-2-Diabetes, gestörtem Glucosestoffwechsel (IGM), gestörter Glucosetoleranz (IFG), abnormer Nüchternglucose (IFG) und Syndrom X.

13. Verbindung nach einem der Ansprüche 1 bis 5 oder pharmazeutische Zusammensetzung nach Anspruch 10 oder Kombination nach Anspruch 11 zur Verwendung als Medikament.

14. Verwendung einer Verbindung nach Anspruch 1 bis 5 oder einer pharmazeutischen Zusammensetzung nach Anspruch 10 oder einer Kombination nach Anspruch 11 zur Herstellung einer Medikaments zur Behandlung oder Prävention von Dyslipidämie, Hyperlipidämie, Hypercholesterinämie, Atherosklerose, Hypertriglyceridämie, Herzversargen, Herzinfarkt, Gefäßerkrankungen, Herzkreislauferkrankungen, Hypertonie, Adipositas, Entzündungen, Arthritis, Krebs, Alzheimer-Krankheit, Hauterkrankungen, Atemwegserkrankungen, Augenerkrankungen, Reizdarmsyndrom, Colitis ulcerosa, Morbus Crohn, Erkrankungen mit beeinträchtigter Glucosetoleranz, einschließlich Hyperglykämie und Insulinresistenz, wie z.B. Typ-1-Diabetes, Typ-2-Diabetes, gestörtem Glucosestoffwechsel (IGM), gestörter Glucosetoleranz (IFG), abnormer Nüchternglucose (IFG) und Syndrom X.

## Revendications

1. Composé choisi parmi Ia, Ib et Ic : 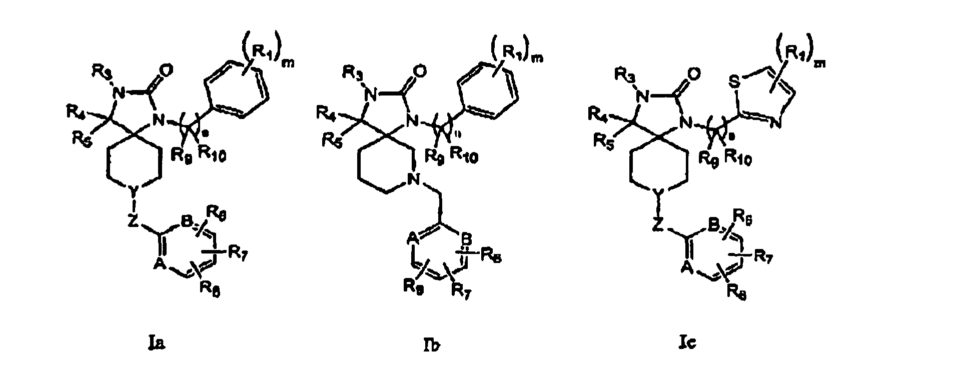 dans lesquelles :
n est choisi parmi 1 et 2 ;
m est choisi parmi 1, 2, 3, 4 et 5 ; chaque
R₁ est indépendamment choisi parmi un atome d'hydrogène, un groupe halogéno, alkyle en C_{1 à 6}, alkyle en C_{1 à 6} substitué par halogéno, alcoxy en C_{1 à 6} et alcoxy en C_{1 à 6} substitué par halogéno ;
R₃ est choisi parmi un groupe alkyle en C_{1 à 8}, alcényle en C_{2 à 8}, alkyle en C_{1 à 6} substitué par halogéno, alcényle en C_{2 à 6} substitué par halogéno, hétéroaryl en C_{5 à 10}-alkyle en C_{0 à 4} et cycloalkyl en C_{3 à 12}-alkyle en C_{0 à 4} ;
où R₂ est choisi parmi un atome d'hydrogène et un groupe alkyle en C_{1 à 6} ;
R₄ est choisi parmi un atome d'hydrogène et un groupe alkyle en C_{1 à 6} ;
R₅ est choisi parmi un atome d'hydrogène et un groupe alkyle en C_{1 à 6} ; ou R₄ et R₅ ensemble avec l'atome de carbone auquel R₄ et R₅ sont tous deux attachés forment un groupe carbonyle ;
Y est N ;
Z est choisi parmi une liaison, -S(O)_{0 à 2}- et -CR₁₁R₁₂-; où R₁₁ et R₁₂ sont indépendamment choisis parmi un atome d'hydrogène et un groupe alkyle en C_{1 à 6} ;
A et B sont indépendamment choisis parmi CH et N ;
R₆ et R₇ sont indépendamment choisis parmi un atome d'hydrogène, un groupe halogéno, alkyle en C_{1 à 6}, alkyle en C_{1 à 6} substitué par halogéno, alcoxy en C_{1 à 6} et alcoxy en C_{1 à 6} substitué par halogéno ;
R₈ est choisi parmi -X₂CO₂R₁₃, -X₂CR₁₄R₁₅X₃CO₂R₁₃-, -X₂SCR₁₄R₁₅X₃C₂R₁₃- et -X₂OCR₁₄R₁₅X₃CO₂R₁₃- ; où X₂ et X₃ sont indépendamment choisis parmi une liaison et un groupe alkylène en C_{1 à 4} ; et R₁₄ et R₁₅ sont indépendamment choisis parmi un atome d'hydrogène, un groupe alkyle en C_{1 à 4} et alcoxy en C_{1 à 4} ; où R₁₄ et R₁₅ ensemble avec l'atome de carbone auquel R₁₄ et R₁₅ sont attachés forment un groupe cycloalkyle en C_{3 à 12} ; et R₁₃ est choisi parmi un atome d'hydrogène et un groupe alkyle en C_{1 à 6} ;
R₉ et R₁₀ sont indépendamment choisis parmi un atome d'hydrogène, un groupe alkyle en C_{1 à 6} et -OR₁₆ ; où R₁₆ est choisi parmi un atome d'hydrogène et un groupe alkyle en C_{1 à 6} ; et les sels, hydrates, solvates et isomères pharmaceutiquement acceptables de celui-ci.

2. Composé selon la revendication 1, dans lequel :
n est choisi parmi 1 et 2 ;
m est choisi parmi 1, 2 et 3 ; chaque
R₁ est indépendamment choisi parmi un atome d'hydrogène, un groupe halogéno, alkyle en C_{1 à 6}, alkyle en C_{1 à 6} substitué par halogéno, alcoxy en C_{1 à 6} est alcoxy en C_{1 à 6} substitué par halogéno ;
R₃ est choisi parmi un groupe alkyle en C_{1 à 8}, alcényle en C_{2 à 8}, alkyle en C_{1 à 6} substitué par halogéno, alcényle en C_{2 à 6} substitué par halogéno, hétéroaryl en C_{5 à 10}-alkyle en C_{0 à 4} et cycloalkyl en C_{3 à 12}-alkyle en C_{0 à 4} ;
où R₂ est choisi parmi un atome d'hydrogène et un groupe alkyle en C_{1 à 6} ;
R₄ est choisi parmi un atome d'hydrogène et un groupe alkyle en C_{1 à 6} ;
R₅ est choisi parmi un atome d'hydrogène et un groupe alkyle en C_{1 à 6} ; ou R₄ et R₅ ensemble avec l'atome de carbone auquel R₄ et R₅ sont tous deux attachés forment un groupe carbonyle ;
Y est N ;
Z est choisi parmi une liaison, -S(O)_{0 à 2}- et -CR₁₁R₁₂-; où R₁₁ et R₁₂ sont indépendamment choisis parmi un atome d'hydrogène et un groupe alkyle en C_{1 à 6} ;
A et B sont indépendamment choisis parmi CH et N ;
R₆ et R₇ sont indépendamment choisis parmi un atome d'hydrogène, un groupe halogéno, alkyle en C_{1 à 6}, alkyle en C_{1 à 6} substitué par halogéno et alcoxy en C_{1 à 6} ;
R₈ est choisi parmi -X₂CO₂R₁₃, -X₂CR₁₄R₁₅X₃CO₂R₁₃- et -X₂OCR₁₄R₁₅X₃CO₂R₁₃- ; où X₂ et X₃ sont indépendamment choisis parmi une liaison et un groupe alkylène en C_{1 à 4} ; et R₁₄ et R₁₅ sont indépendamment choisis parmi un atome d'hydrogène et un groupe alkyle en C_{1 à 4} ; i R₁₃ est choisi parmi un atome d'hydrogène un groupe alkyle en C_{1 à 6} ; et
R₉ et R₁₀ sont indépendamment choisis parmi un atome d'hydrogène, un groupe alkyle en C_{1 à 6} et -OR₁₆ ; où R₁₆ est choisi parmi un atome d'hydrogène et un groupe alkyle en C_{1 à 6}.

3. Composé selon la revendication 2, dans lequel : R₁ est indépendamment choisi parmi un atome d'hydrogène, un groupe halogéno, méthoxy, trifluorométhoxy et trifluorométhyle ; R₃ est choisi parmi un groupe isobutyle, cyclopropyl-méthyle, cyclobutyl-méthyle, isopentyle, butyle, cyclopentyl-méthyle, 3-méthyl-but-2-ényle, pentyle, 2,2-diméthyl-propyle, 4-fluoro-butyle, 2-éthyl-butyle, 2-méthyl-pentyle, cyclohexyl-méthyle, 3,3-diméthyl-2-oxo-butyle, pyrrolyl-propyle, 3-trifluorométhyl-propyle, cyclohexyl-éthyle, 2-éthyl-hexyle, 2-méthyl-butyle, 3,4,4-trifluoro-but-3-ényle et 3,3-diméthyl-butyle ; R₄ et R₅ sont chacun un atome d'hydrogène ou R₄ et R₅ ensemble avec l'atome de carbone auquel R₄ et R₅ sont tous deux attachés forment un groupe carbonyle ; et Z est choisi parmi une liaison, -S(O)₂- et -CH₂-.

4. Composé selon la revendication 3, dans lequel : R₈ est choisi parmi -CH₂C(O)OH, -CH(CH₂)C(O)OH, -OC(CH₂)₂C(O)OH, - (CH₂)₂C(O)OH et -OCH₂C(O)OH ; et R₉ et R₁₀ sont indépendamment choisis parmi un atome d'hydrogène, un groupe halogéno, méthyle, méthoxy et trifluorométhyle.

5. Composé selon la revendication 1, choisi parmi :
l'acide (3-{3-isobutyl-1-[2-(4-méthoxy-phényl)-éthyl]-2,4-dioxo-1,3,8-triaza-spiro[4.5]déc-8-ylméthyl}-phényl)-acétique ; l'acide (3-{3-isobutyl-1-[2-(4-méthoxy-phényl)-éthyl]-2,4-dioxo-1,3,8-triaza-spiro[4.5]décane-8-sulfonyl}-4-méthyl-phényl)-acétique ; l'acide (3-{3-isobutyl-1-[2-(4-méthoxy-phényl)-éthyl]-2,4-dioxo-1,3,8-triaza-spiro[4.5]déc-8-yl}-phényl)-acétique ; l'acide 2-(4-{3-isobutyl-1-[2-(4-méthoxy-phényl)-éthyl]-2,4-dioxo-1,3-diaza-spiro[4.5]déc-7-ylméthyl}-phényl)-propionique ; l'acide (3-{3-cyclopropylméthyl-1-[2-(4-méthoxy-phényl)-éthyl]-2,4-dioxo-1,3,8-triaza-spiro[4.5]déc-8-yl}-phényl)-acétique ; l'acide {3-[3-isobutyl-2,4-dioxo-1-(4-trifluorométhoxy-benzyl)-1,3,8-triaza-spiro[4.5]déc-8-yl]-phényl}-acétique ; l'acide 2-(2-{3-isobutyl-1-[2-(4-méthoxy-phényl)-éthyl]-2,4-dioxo-1,3,8-triaza-spiro[4.5]déc-8-yl}-pyrimidin-4-yloxy)-2-méthyl-propionique ; l'acide 2-(3-{3-cyclobutylméthyl-1-[2-(2,4-dichloro-phényl)-éthyl]-2,4-dioxo-1,3-diaza-spiro[4.5]déc-8-yl}-phénoxy)-2-méthyl-propionique ; l'acide {3-[3-cyclopropylméthyl-2,4-dioxo-1-(4-trifluorométhoxy-benzyl)-1,3,8-triaza-spiro[4.5]déc-8-yl]-phényl}-acétique ; l'acide (3-{3-cyclobutylméthyl-2,4-dioxo-1-[2-(4-trifluorométhyl-phényl)-éthyl]-1,3,8-triaza-spiro[4.5]déc-8-yl}-phényl)-acétique ; l'acide (3-{3-cyclobutylméthyl-1-[4-(4-méthoxy-phényl)-butyl]-2,4-dioxo-1,3,8-triaza-spiro[4.5]déc-8-yl}-phényl)-acétique ; l'acide 3-{3-isobutyl-1-[2-(4-méthoxy-phényl)-éthyl]-2,4-dioxo-1,3,8-triaza-spiro[4.5]déc-8-ylméthyl}-benzoïque ; l'acide (2-{3-isobutyl-1-[2-(4-méthoxy-phényl)-éthyl]-2,4-dioxo-1,3,8-triaza-spiro[4.5]déc-8-ylméthyl}-phényl)-acétique ; l'acide (3-{3-isobutyl-1-[2-(4-méthoxy-phényl)-éthyl]-2,4-dioxo-1,3,8-triaza-spiro[4.5]décane-8-sulfonyl}-4-méthoxy-phényl)-acétique ; l'acide 3-(3-{3-isobutyl-1-[2-(4-méthoxy-phényl)-éthyl]-2,4-dioxo-1,3,8-triaza-spiro[4.5]déc-8-yl}-phényl)-propionique ; l'acide (3-{3-isobutyl-1-[2-(4-méthoxy-phényl)-éthyl]-2,4-dioxo-1,3,8-triaza-spiro[4.5]déc-8-yl}-phénoxy)-acétique ; l'acide 2-(3-{3-isobutyl-1-[2-(4-méthoxy-phényl)-éthyl]-2,4-dioxo-1,3,8-triaza-spiro[4.5]déc-8-yl}-phénoxy)-2-méthyl-propionique ; l'acide (5-{3-isobutyl-1-[2-(4-méthoxy-phényl)-éthyl]-2,4-dioxo-1,3,8-triaza-spiro[4.5]déc-8-yl}-2-méthyl-phényl)-acétique ; l'acide (3-{3-isobutyl-1-[2-(4-méthoxy-phényl)-éthyl]-2,4-dioxo-1,3,8-triaza-spiro[4.5]déc-8-yl}-5-méthyl-phényl)-acétique ; l'acide (2-fluoro-5-{3-isobutyl-1-[2-(4-méthoxy-phényl)-éthyl]-2,4-dioxo-1,3,8-triaza-spiro[4.5]déc-8-yl}-phényl)-acétique ; l'acide (5-{3-isobutyl-1-[2-(4-méthoxy-phényl)-éthyl]-2,4-dioxo-1,3,8-triaza-spiro[4.5]déc-8-yl}-2-trifluorométhyl-phényl)-acétique ; l'acide (5-{3-isobutyl-1-[2-(4-méthoxy-phényl)-éthyl]-2,4-dioxo-1,3,8-triaza-spiro[4.5]déc-8-yl}-méthoxy-phényl)-acétique ; l'acide 2-(3-{3-isobutyl-1-[2-(4-méthoxy-phényl)-éthyl]-2,4-dioxo-1,3,8-triaza-spiro[4.5]déc-8-yl}-phényl)-2-méthyl-propionique ; l'acide (5-{3-isobutyl-1-[2-(4-méthoxy-phényl)-éthyl]-2,4-dioxo-1,3,8-triaza-spiro[4.5]déc-8-yl}-2-méthyl-phénoxy)-acétique ; l'acide (2-chloro-5-{3-isobutyl-1-[2-(4-méthoxy-phényl)-éthyl]-2,4-dioxo-1,3,8-triaza-spiro[4.5]déc-8-yl}-phénoxy)-acétique ; l'acide 2-(5-{3-isobutyl-1-[2-(4-méthoxy-phényl)-éthyl]-2,4-dioxo-1,3,8-triaza-spiro[4.5]déc-8-yl}-2-méthyl-phénoxy)-2-méthyl-propionique ; l'acide 2-(2-chloro-5-{3-isobutyl-1-[2-(4-méthoxy-phényl)-éthyl]-2,4-dioxo-1,3,8-triaza-spiro[4.5]déc-8-yl}-phénoxy)-2-méthyl-propionique ; l'acide 2-(2,3-difluoro-5-{3-isobutyl-1-[2-(4-méthoxy-phényl)-éthyl]-2,4-dioxo-1,3,8-triaza-spiro[4.5]déc-8-yl}-phénoxy)-2-méthyl-propionique ; l'acide (3-{3-isobutyl-1-[2-(4-méthoxy-phényl)-éthyl]-2-oxo-1,3,8-triaza-spiro[4.5]déc-8-yl}-phényl)-acétique ; l'acide (6-{3-isobutyl-1-[2-(4-méthoxy-phényl)-éthyl]-2,4-dioxo-1,3,8-triaza-spiro[4.5]déc-8-yl}-pyridin-2-yl)-acétique ; l'acide (2-{3-isobutyl-1-[2-(4-méthoxy-phényl)-éthyl]-2,4-dioxo-1,3,8-triaza-spiro[4.5]déc-8-yl}-pyridin-4-yl)-acétique ; l'acide (5-{3-cyclobutylméthyl-1-[2-(2,4-dichloro-phényl-éthyl]-2,4-dioxo-1,3,8-triaza-spiro[4.5]déc-8-yl}-2-méthyl-phényl)-acétique ; l'acide 2-(5-{3-cyclobutylméthyl-1-[2-(2,4-dichloro-phényl)-éthyl]-2,4-dioxo-1,3,8-triaza-spiro[4.5]déc-8-yl}-2-méthyl-phénoxy)-2-méthyl-propionique ; l'acide (2-chloro-5-{3-cyclobutylméthyl-1-[2-(2,4-dichloro-phényl)-éthyl]-2,4-dioxo-1,3,8-triaza-spiro[4.5]déc-8-yl}-phénoxy)-acétique ; l'acide 2-(2-chloro-5-{3-cyclobutylméthyl-1-[2-(2,4-dichloro-phényl)-éthyl]-2,4-dioxo-1,3,8-triaza-spiro[4.5]déc-8-yl}-phénoxy)-2-méthyl-propionique ; l'acide (6-{3-cyclobutylméthyl-1-[2-(2,4-dichloro-phényl)-éthyl]-2,4-dioxo-1,3,8-triaza-spiro[4.5]déc-8-yl}-pyridin-2-yl)-acétique ; l'acide (4-{3-isobutyl-1-[2-(4-méthoxy-phényl)-éthyl]-2,4-dioxo-1,3,7-triaza-spiro[4.5]déc-7-ylméthyl}-phénoxy)-acétique ; l'acide (3-{3-cyclobutylméthyl-1-[2-(4-méthoxy-phényl)-éthyl]-2,4-dioxo-1,3,8-triaza-spiro[4.5]déc-8-yl}-phényl)-acétique ; l'acide {3-[1-[2-(4-méthoxy-phényl)-éthyl]-3-(3-méthyl-butyl)-2,4-dioxo-1,3,8-triaza-spiro[4.5]déc-8-yl]-phényl}-acétique ; l'acide (3-{3-butyl-1-[2-(4-méthoxy-phényl)-éthyl]-2,4-dioxo-1,3,8-triaza-spiro[4.5]déc-8-yl}-phényl)-acétique ; l'acide 2-(4-{3-isobutyl-1-[2-(4-méthoxy-phényl)-éthyl]-2,4-dioxo-1,3,8-triaza-spiro[4.5]déc-8-yl}-pyrimidin-2-yloxy)-2-méthyl-propionique ; l'acide 2-(6-{3-isobutyl-1-[2-(4-méthoxy-phényl)-éthyl]-2,4-dioxo-1,3,8-triaza-spiro[4.5]déc-8-yl}-pyrimidin-4-yloxy)-2-méthyl-propionique ; l'acide 2-(4-{3-cyclobutylméthyl-1-[2-(2,4-dichloro-phényl)-éthyl]-2,4-dioxo-1,3,8-triaza-spiro[4.5]déc-8-yl}-pyrimidin-2-yloxy)-2-méthyl-propionique ; l'acide 2-(2-{3-cyclobutylméthyl-1-[2-(2,4-dichloro-phényl)-éthyl]-2,4-dioxo-1,3,8-triaza-spiro[4.5]déc-8-yl}-pyrimidin-4-yloxy)-2-méthyl-propionique ; l'acide 2-(6-{3-cyclobutylméthyl-1-[2-(2,4-dichloro-phényl)-éthyl]-2,4-dioxo-1,3,8-triaza-spiro[4.5]déc-8-yl}-pyrimidin-4-yloxy)-2-méthyl-propionique ; l'acide {3-[3-cyclobutylméthyl-2,4-dioxo-1-(4-trifluorométhoxy-benzyl)-1,3,8-triaza-spiro[4.5]déc-8-yl]-phényl}-acétique ; l'acide {3-[3-cyclobutylméthyl-2,4-dioxo-1-(4-trifluorométhyl-benzyl)-1,3,8-triaza-spiro[4.5]déc-8-yl]-phényl}-acétique ; l'acide {3-[3-cyclopentylméthyl-2,4-dioxo-1-(4-trifluorométhyl-benzyl)-1,3,8-triaza-spiro[4.5]déc-8-yl]-phényl}-acétique ; l'acide {3-[3-cyclopentylméthyl-2,4-dioxo-1-(4-trifluorométhoxy-benzyl)-1,3,8-triaza-spiro[4.5]déc-8-yl]-phényl}-acétique ; l'acide {3-[1-(2,4-bis-trifluorométhyl-benzyl)-8-cyclopentylméthyl-2,4-dioxo-1,3,8-triaza-spiro[4.5]déc-8-yl]-phényl}-acétique ; l'acide {3-[3-cyclobutylméthyl-2,4-dioxo-1-(3-trifluorométhoxy-benzyl)-1,3,8-triaza-spiro[4.5]déc-8-yl]-phényl}-acétique ; l'acide (3-{3-cyclobutylméthyl-2,4-dioxo-1-[4-(4-trifluorométhyl-phényl)-thiazol-2-ylméthyl]-1,3,8-triaza-spiro[4.5]déc-8-yl]-phényl-acétique ; l'acide (3-{3-cyclopropylméthyl-2,4-dioxo-1-[4-(4-trifluorométhyl-phényl)-thiazol-2-ylméthyl]-1,3,8-triaza-spiro[4.5]déc-8-yl}-phényl)-acétique ; l'acide {3-[3-(3-méthyl-but-2-ényl)-2,4-dioxo-1-(4-trifluorométhoxy-benzyl)-1,3,8-triaza-spiro[4.5]déc-8-yl]-phényl}-acétique ; l'acide {3-[1-[2-(4-bromo-phényl)-2-hydroxy-éthyl]-3-(3-méthyl-butyl)-2,4-dioxo-1,3,8-triaza-spiro[4.5]déc-8-yl]-phényl}-acétique ; l'acide {3-[1-[2-(4-chloro-phényl)-2-hydroxy-éthyl]-3-(3-méthyl-butyl)-2,4-dioxo-1,3,8-triaza-spiro[4.5]déc-8-yl]-phényl}-acétique ; l'acide {3-[2,4-dioxo-3-pentyl-1-(4-trifluorométhoxy-benzyl)-1,3,8-triaza-spiro[4.5]déc-8-yl]-phényl}-acétique ; l'acide {3-[3-(2,2-diméthyl-propyl)-2,4-dioxo-1-(4-trifluorométhoxy-benzyl)-1,3,8-triaza-spiro[4.5]déc-8-yl]-phényl}-acétique ; l'acide {3-[3-(2-éthyl-butyl)-2,4-dioxo-1-(4-trifluorométhoxy-benzyl)-1,3,8-triaza-spiro[4.5]déc-8-yl]-phényl}-acétique ; l'acide {3-[3-(4-fluoro-butyl)-2,4-dioxo-1-(4-trifluorométhoxy-benzyl)-1,3,8-triaza-spiro[4.5]déc-8-yl]-phényl}-acétique ; l'acide {3-[3-(4-méthyl-pentyl)-2,4-dioxo-1-(4-trifluorométhoxy-benzyl)-1,3,8-triaza-spiro[4.5]déc-8-yl]-phényl}-acétique ; l'acide {3-[3-cyclohexylméthyl-2,4-dioxo-1-(4-trifluorométhoxy-benzyl)-1,3,8-triaza-spiro[4.5]déc-8-yl]-phényl}-acétique ; l'acide (3-[2,4-dioxo-3-(3-pyrrol-1-yl-propyl)-1-(4-trifluorométhoxy-benzyl)-1,3,8-triaza-spiro[4.5]déc-8-yl]-phényl}-acétique ; l'acide {3-[3-(3,3-diméthyl-2-oxo-butyl)-2,4-dioxo-1-(4-trifluorométhoxy-benzyl)-1,3,8-triaza-spiro[4.5]déc-8-yl]-phényl}-acétique ; l'acide {3-[2,4-dioxo-3-(4,4,4-trifluoro-butyl)-1-(4-trifluorométhoxy-benzyl)-1,3,8-triaza-spiro[4.5]déc-8-yl]-phényl}-acétique ; l'acide {3-[3-(2-cyclohexyl-éthyl)-2,4-dioxo-1-(4-trifluorométhoxy-benzyl)-1,3,8-triaza-spiro[4.5]déc-8-yl]-phényl}-acétique ; l'acide {3-[3-(2-éthyl-hexyl)-2,4-dioxo-1-(4-trifluorométhoxy-benzyl)-1,3,8-triaza-spiro[4.5]déc-8-yl]-phényl}-acétique ; l'acide {3-[3-(2-méthyl-butyl)-2,4-dioxo-1-(4-trifluorométhoxy-benzyl)-1,3,8-triaza-spiro[4.5]déc-8-yl]-phényl}-acétique ; l'acide {3-[2,4-dioxo-3-(3,4,4-trifluoro-but-3-ényl)-1-(4-trifluorométhoxy-benzyl)-1,3,8-triaza-spiro[4.5]déc-8-yl]-phényl}-acétique ; l'acide {3-[3-(3,3-diméthyl-butyl)-2,4-dioxo-1-(4-trifluorométhoxy-benzyl)-1,3,8-triaza-spiro[4.5]déc-8-yl)-phényl}-acétique ; l'acide {3-[1-(2,4-dichloro-5-fluoro-benzyl)-3-(3,3-diméthyl-butyl)-2,4-dioxo-1,3,8-triaza-spiro[4.5]déc-8-yll-phényll-acétique ; l'acide {3-[1-(2,4-dichloro-5-fluoro-benzyl)-3-(4-fluoro-butyl)-2,4-dioxo-1,3,8-triaza-spiro[4.5]déc-8-yl]-phényl}-acétique ; l'acide (3-{3-cyclobutylméthyl-1-[2-(2,4-dichloro-phényl)-éthyl]-2,4-dioxo-1,3,8-triaza-spiro[4.5]déc-8-yl}-phényl)-acétique ; l'acide (3-{3-cyclopentylméthyl-2,4-dioxo-1-[2-(4-trifluorométhyl-phényl)-éthyl]-1,3,8-triaza-spiro[4.5]déc-8-yl}-phényl)-acétique ; l'acide (3-{3-cyclopentylméthyl-1-[2-(2,4-dichloro-phényl)-éthyl]-2,4-dioxo-1,3,8-triaza-spiro[4.5]déc-8-yl}-phényl)-acétique ; l'acide (3-{3-cyclohexylméthyl-1-[2-(2,4-dichloro-phényl)-éthyl]-2,4-dioxo-1,3,8-triaza-spiro[4.5]déc-8-yl}-phényl)-acétique ; et l'acide (3-{1-[2-(4-chloro-phényl)-éthyl]-3-cyclopentylméthyl-2,4-dioxo-1,3,8-triaza-spiro[4.5]déc-8-yl}-phényl)-acétique.

6. Composé selon l'une quelconque des revendications 1 à 5 à utiliser dans un procédé de traitement ou de prévention de la dyslipidémie, de l'hyperlipidémie, de l'hypercholestérolémie, de l'athérosclérose, de l'athérogenèse, de l'hypertriglycéridémie, de l'insuffisance cardiaque, de l'infarctus du myocarde, des maladies vasculaires, des maladies cardiovasculaires, de l'hypertension, de l'obésité, du marasme, de l'inflammation, de l'arthrite, du cancer, de l_{'}anorexie, de l'anorexie mentale, de la boulimie, de la maladie d'Alzheimer, des troubles cutanés, des maladies respiratoires, des troubles ophtalmiques, des troubles du côlon irritable, de la colite ulcéreuse, de la maladie de Crohn, du diabète de type 1, du diabète du type 2 ou du syndrome X.

7. Composé selon l'une quelconque des revendications 1 à 5 à utiliser dans un procédé de traitement ou de prévention du syndrome cachectique lié au VIH, de la maladie grave à long terme, de la masse musculaire et/ou force musculaire diminuée, de la masse maigre diminuée, de la maintenance de la force musculaire et du fonctionnement musculaire chez les personnes âgées, de l'endurance musculaire diminuée et du fonctionnement musculaire diminué, ou de la fragilité chez les personnes âgées.

8. Utilisation d'un composé selon l'une quelconque des revendications 1 à 5 dans la fabrication d'un médicament destiné au traitement ou à la prévention de la dyslipidémie, de l'hyperlipidémie, de l'hypercholestérolémie, de l'athérosclérose, de l'athérogenèse, de l'hypertriglycéridémie, de l'insuffisance cardiaque, de l'infarctus du myocarde, des maladies vasculaires, des maladies cardiovasculaires, de l'hypertension, de l'obésité, du marasme, de l'inflammation, de l'arthrite, du cancer, de l'anorexie, de l'anorexie mentale, de la boulimie, de la maladie d'Alzheimer, des troubles cutanés, des maladies respiratoires, des troubles ophtalmiques, des troubles du côlon irritable, de la colite ulcéreuse, de la maladie de Crohn, du diabète de type 1, du diabète du type 2 ou du syndrome X.

9. Utilisation d'un composé selon l'une quelconque des revendications 1 à 5 dans la fabrication d'un médicament destiné au traitement ou à la prévention du syndrome cachectique lié au VIH, de la maladie grave à long terme, de la masse musculaire et/ou force musculaire diminuée, de la masse maigre diminuée, de la maintenance de la force musculaire et du fonctionnement musculaire chez les personnes âgées, de l'endurance musculaire diminuée et du fonctionnement musculaire diminué, ou de la fragilité chez les personnes âgées.

10. Composition pharmaceutique comprenant une quantité thérapeutiquement efficace d'un composé selon l'une quelconque des revendications 1 à 5 en combinaison avec un ou plusieurs excipients pharmaceutiquement acceptables.

11. Combinaison pharmaceutique, en particulier composition pharmaceutique, comprenant : 1) un composé selon l'une quelconque des revendications 1 à 5 ou un sel pharmaceutiquement acceptable de celui-ci ; et 2) au moins un ingrédient actif choisi parmi :
a) les agents antidiabétiques tels que l'insuline, les dérivés d'insuline et les mimétiques ; les sécrétagogues d'insuline tels que les sulfonylurées, par exemple, le glipizide, le glyburide et l'amaryle ; les ligands du récepteur de sulfonylurée insulinotrope tels que les méglitinides, par exemple, le natéglinide et le répaglinide ; un médicament insulinosensibilisant tel que les inhibiteurs de la protéine tyrosine phosphatase-IB (PTP-IB) tels que PTP-112 ; les inhibiteurs de GSK3 (glycogène synthase kinase-3) tels que SB-517955, SB-4195052, SB-216763, NN-57-05441 et NN-57-05445 ; les ligands de RXR tels que GW-0791 et AGN-194204 ; les inhibiteurs de cotransporteur de glucose dépendant du sodium tels que T-1095 ; les inhibiteurs de glycogène phosphorylase A tels que BAY R3401 ; les biguanides tels que la metformine ; les inhibiteurs d'alpha-glucosidase tels que l'acarbose ; GLP-I (peptide-1 de type glucagon), les analogues de GLP-I tels que les mimétiques de l'exendine-4 et GLP-I ; les inhibiteurs de dipeptidyl peptidase IV tels que DPP728, la vildagliptine, MK-0431, la saxagliptine, GSK23A ; un briseur AGE ; un dérivé de thiazolidone (glitazone) tels que la pioglitazone, la rosiglitazone, ou l'acide (R)-1-{4-[5-méthyl-2-(4-trifluorométhyl-phényl)-oxazol-4-ylméthoxy]-benzènesulfonyl}-2,3-dihydro-1H-indole-2-carboxylique, un agoniste de PPARγ de type non glitazone par exemple, GI-262570 ;
b) les agents hypolipidémiques tels que les inhibiteurs de réductase de coenzyme A de 3-hydroxy-3-méthyl-glutaryle (HMG-CoA), par exemple, la lovastatine, la pitavastatine, la simvastatine, la pravastatine, la cérivastatine, la mévastatine, la vélostatine, la fluvastatine, la dalvastatine, l'atorvastatine, la rosuvastatine et la rivastatine ; les inhibiteurs de squalène synthase ; les ligands de FXR (récepteur de farnésoïde X) et LXR (récepteur de foie X) ; la cholestyramine ; les fibrates ; l'acide nicotinique et l'aspirine ;
c) un agent anti-obésité ou un agent régulateur de l'appétit tel que la phentermine, la leptine, la bromocriptine, la dexamphétamine, l'amphétamine, la fenfluramine, la dexfenfluramine, la sibutramine, l'orlistat, la dexfenfluramine, le mazindol, la phentermine, la phendimétrazine, le diéthylpropion, la fluoxétine, le bupropion, le topiramate, le diéthylpropion, la benzphétamine, la phénylpropanolamine ou l'écopipam, l'éphédrine, la pseudoéphédrine ou les antagonistes du récepteur de cannabinoïde ;
d) les agents anti-hypertenseurs, par exemple, les diurétiques de l'anse de Henle tels que l'acide éthacrynique, le furosémide et le torsémide ; les diurétiques tels que les dérivés de thiazide, le chlorithiazide, l'hydrochlorothiazide, l'amiloride ; les inhibiteurs de l'enzyme de conversion de l'angiotensine (ACE) tels que le bénazépril, le captopril, l'énalapril, le fosinopril, le lisinopril, le moexipril, le périnodopril, le quinapril, le ramipril et le trandolapril ; les inhibiteurs de la pompe de membrane de Na-K-ATPase tels que la digoxine ; les inhibiteurs de neutralendopeptidase (NEP) par exemple le thiorphan, le tertéo-thiorphan, SQ29072 ; les inhibiteurs de ECE par exemple SLV306 ; les inhibiteurs de ACE/NEP tels que l'omapatrilat, le sampatrilat et le fasidotril ; les antagonistes de l'angiotensine II tels que le candésartan, l'éprosartan, l'irbésartan, le losartan, le telmisartan et le valsartan, en particulier le valsartan ; les inhibiteurs de rénine tels que l'aliskiren, le terlakiren, le ditékiren, RO-66-1132, RO-66-1168 ; les bloqueurs du récepteur β-adrénergique tels que l'acébutolol, l'aténolol, le bétaxolol, le bisoprolol, le métoprolol, le nadolol, le propranolol, le sotalol et le timolol ; les agents inotropes tels que la digoxine, la dobutamine et la milrinone ; les bloqueurs des canaux calciques tels que l'amlodipine, le bépridil, le diltiazem, la félodipine, la nicardipine, la nimodipine, la nifédipine, la nisoldipine et le vérapamil ; les antagonistes du récepteur d'aldostérone ; et les inhibiteurs d'aldostérone synthase ;
e) un composé d'augmentation de HDL ;
f) un modulateur de l'absorption du cholestérol tels que Zetia^{®} et KT6-971 ;
g) les analogues et mimétiques de Apo-A1 ;
h) les inhibiteurs de thrombine tels que le ximélagatran ;
i) les inhibiteurs d'aldostérone tels que l'anastrazole, le fadrazole, l'éplérénone ;
j) les inhibiteurs d'agrégation plaquettaire tels que l'aspirine, le clopidogrel bisulfate ;
k) l'oestrogène, la testostérone, un modulateur du récepteur de l'oestrogène sélectif, un modulateur du récepteur de l'androgène sélectif ;
l) un agent chimiothérapeutique tel que les inhibiteurs d'aromatase, par exemple le fémara, les anti-oestrogènes, les inhibiteurs de topoisomérase I, les inhibiteurs de topoisomérase II, les agents actifs de microtubule, les agents d'alkylation, les antimétabolites antinéoplasiques, les composés platine, les composés diminuant l'activité de la protéine kinase tels qu'un inhibiteur de tyrosine kinase du récepteur de PDGF de préférence l'imatinib ou le 4-méthyl-N-[3-(4-(méthyl-imidazol-1-yl)-5-trifluorométhyl-phényl]-3-(4-pyridin-3-yl-pyrimidin-2-ylamino)-benzamide ; et
m) un agent interagissant avec un récepteur de 5-HT₃ et/ou un agent interagissant avec un récepteur de 5-HT₄ tel que le tégasérod, l'hydrogénomaléate de tégasérod, le cisapride, le cilansétron ;
ou, dans chaque cas un sel pharmaceutiquement acceptable de celui-ci ; et facultativement un support pharmaceutiquement acceptable.

12. Composition pharmaceutique selon la revendication 10 ou combinaison selon la revendication 11, pour le traitement ou la prévention de la dyslipidémie, de l'hyperlipidémie, de l'hypercholestérolémie, de l'athérosclérose, de l'hypertriglycéridémie, de l'insuffisance cardiaque, de l'infarctus du myocarde, des maladies vasculaires, des maladies cardiovasculaires, de l'hypertension, de l'obésité, de l'inflammation, de l'arthrite, du cancer, de la maladie d'Alzheimer, des troubles cutanés, des maladies respiratoires, des troubles ophtalmiques, des affections abdominales inflammatoires, des IBD (maladie du côlon irritable), de la colite ulcéreuse, de la maladie de Crohn, des affections dans lesquelles une tolérance au glucose altérée, une hyperglycémie et une résistance à l'insuline sont impliquées, telles que les diabètes de type 1 et de type 2, un métabolisme de glucose altéré (IGM), une tolérance au glucose altérée (IGT), un glucose à jeun altéré (IFG), et le syndrome X.

13. Composé selon l'une quelconque des revendications 1 à 5, ou composition pharmaceutique selon la revendication 10 ou combinaison selon la revendication 11, à utiliser comme médicament.

14. Utilisation d'un composé selon l'une quelconque des revendications 1 à 5, ou composition pharmaceutique selon la revendication 10 ou combinaison selon la revendication 11, pour la fabrication d'un médicament destiné au traitement ou à la prévention de la dyslipidémie, de l'hyperlipidémie, de l'hypercholestérolémie, de l'athérosclérose, de l'hypertriglycéridémie, de l'insuffisance cardiaque, de l'infarctus du myocarde, des maladies vasculaires, des maladies cardiovasculaires, de l'hypertension, de l'obésité, de l'inflammation, de l'arthrite, du cancer, de la maladie d'Alzheimer, des troubles cutanés, des maladies respiratoires, des troubles ophtalmiques, des affections abdominales inflammatoires, des IBD (maladie du côlon irritable), de la colite ulcéreuse, de la maladie de Crohn, des affections dans lesquelles une tolérance au glucose altérée, une hyperglycémie et une résistance à l'insuline sont impliquées, telles que les diabètes de type 1 et de type 2, un métabolisme de glucose altéré (IGM), une tolérance au glucose altérée (IGT), un glucose à jeun altéré (IFG), et du syndrome X.
